(19) Europäisches Patentamt
European Patent Office
Office européen des brevets

(11) EP 4 600 358 A1

(12) EUROPEAN PATENT APPLICATION
published in accordance with Art. 153(4) EPC

(43) Date of publication:
**13.08.2025 Bulletin 2025/33**

(21) Application number: **24788021.4**

(22) Date of filing: **08.04.2024**

(51) International Patent Classification (IPC):
***C12N 15/113*** *(2010.01)* ***A61K 31/7088*** *(2006.01)*
***A61P 37/00*** *(2006.01)*

(52) Cooperative Patent Classification (CPC):
**C12N 15/113; A61K 31/7088; A61P 37/00; C07H 21/00; C12N 15/111;** C12N 2310/11; C12N 2310/14; C12N 2310/315; C12N 2310/317; C12N 2310/321; C12N 2310/322; C12N 2310/351; C12N 2310/3521; C12N 2310/3533

(86) International application number:
**PCT/CN2024/086555**

(87) International publication number:
**WO 2024/212918 (17.10.2024 Gazette 2024/42)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **14.04.2023 PCT/CN2023/088483**
**01.09.2023 PCT/CN2023/116571**

(71) Applicant: **Rigerna Therapeutics (Suzhou) Co., Ltd.**
**Suzhou, Jiangsu 215128 (CN)**

(72) Inventors:
- **HUANG, Yuanyu**
  **Suzhou, Jiangsu 215128 (CN)**
- **LI, Haitao**
  **Suzhou, Jiangsu 215128 (CN)**
- **KONG, Lina**
  **Suzhou, Jiangsu 215128 (CN)**
- **FAN, Zhibin**
  **Suzhou, Jiangsu 215128 (CN)**
- **GAO, Yongxin**
  **Suzhou, Jiangsu 215128 (CN)**
- **HAN, Xiaofeng**
  **Suzhou, Jiangsu 215128 (CN)**

(74) Representative: **Hoffmann Eitle**
**Patent- und Rechtsanwälte PartmbB**
**Arabellastraße 30**
**81925 München (DE)**

Remarks:
The complete document including Reference Table(s) and the Sequence Listing(s) can be downloaded from the EPO website

# (54) COMPOUND FOR INHIBITING C3 GENE EXPRESSION, PHARMACEUTICAL COMPOSITION AND USE THEREOF

(57) A compound for inhibiting C3 gene expression, a pharmaceutical composition and a use thereof, belonging to the technical field of small nucleic acid drug delivery. A compound conjugated with an oligonucleotide and a pharmaceutical composition thereof can significantly inhibit the expression of C3 mRNA in animal liver tissue and C3 protein in serum and can reduce C3 deposition in renal tissue and improve the course of disease featuring C3 deposition in renal tissue. The no-adverse-effect level (NOAEL) in SD rats and cynomolgus monkeys is 300 mg/kg. The compound conjugated with a oligonucleotide and the pharmaceutical composition thereof are helpful in alleviating, preventing and/or treating diseases or conditions mediated by dysregulated C3 gene expression.

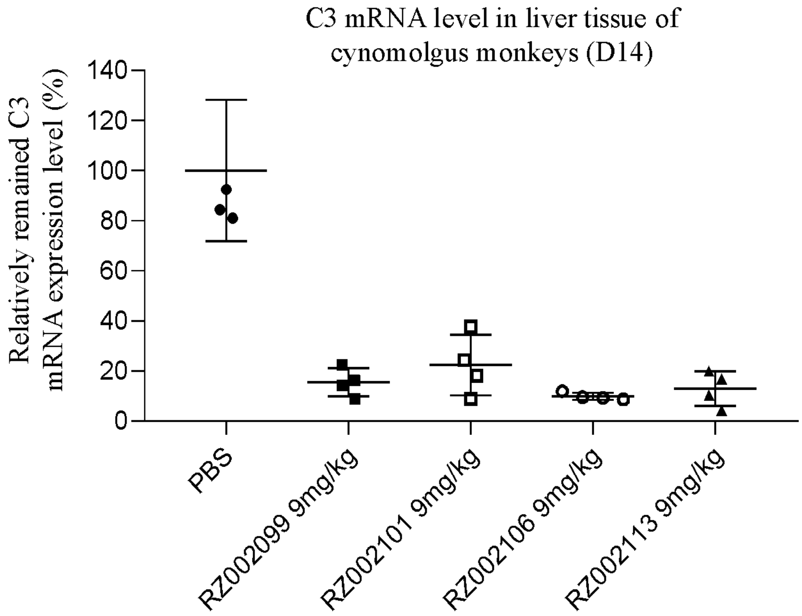


FIG. 1

AA C3 mRNA levels in liver tissue of cynomolgus monkeys (D14)
BB C3 mRNA relative remaining expression level (%)

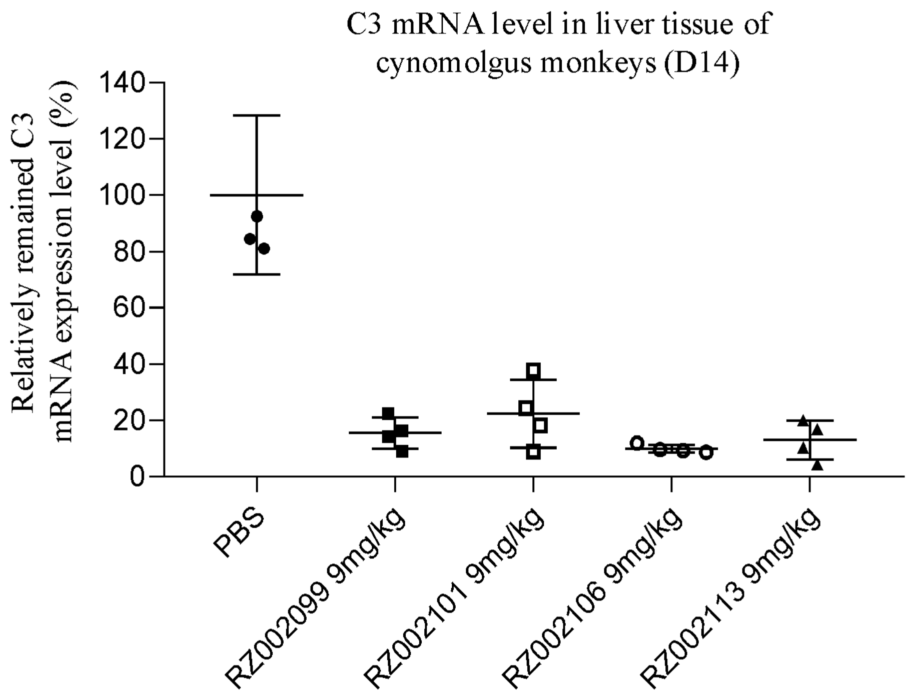


**FIG. 1**

## Description

### FIELD

The present disclosure relates to the technical field of delivery of small nucleic acid drugs, and particularly relates to a compound conjugated with an oligonucleotide for use in inhibiting expression of C3 gene, a pharmaceutical composition, and use thereof.

### BACKGROUND

The complement system is an important component of the innate immune system, comprises more than 50 soluble or membrane-bound proteins, and plays an important role in various physiological processes in the body, including defense against exogenous substances, cell lysis, inflammatory response, clearance of immune complexes and apoptotic cells, and enhancement of humoral immune response.

The complement system is activated through three main pathways, namely the classical, alternative and mannan-binding lectin (MBL) pathways.

Since the complement system plays an important role in the body's defense against exogenous substances, clearance of immune complexes from the body, and acquired immunity, abnormalities of the complement system are closely associated with a variety of diseases, including renal diseases and autoimmune disorders. For example, atypical haemolytic uremic syndrome (aHUS) characteristically presents with microangiopathic haemolytic anaemia, thrombocytopenia and acute kidney failure. More than 50% of aHUS incidents are associated with mutations or polymorphisms in complement regulators, such as Factor H, Factor I, MCP, C4BP, Factor B, and C3, and the production of anti-Factor H autoantibodies. These mutations or polymorphisms usually present as heterozygotes and affect the secretion and function of these proteins. In addition, membranoproliferative glomerulonephritis (MPGN) type II is a serious renal disease, characterized by electron-dense deposits visible under electron microscopy, accompanied by hyperplasia of glomerular basement membranes and mesangial cells. Populations with deficiency of Factor H or C3 are susceptible to such diseases. Moreover, the production of autoantibodies against C3 convertase (also known as the C3 nephritic Factor, C3NeF) in the alternative pathway is also associated with MPGN. Glomerulonephritis is a disease caused by kidney damage resulted from hyperactivation of complement induced by immune complexes in the renal vasculature. C3 and its regulatory proteins Factor I and Factor H are closely associated with the occurrence and development of glomerulonephritis. Deficiency of Factor I and Factor H can lead to dysregulation of C3. Moreover, C1q knockout mice produce autoantibodies, and apoptotic cells cannot be cleared effectively, which causes the C1q knockout mice to be susceptible to glomerulonephritis induced by immune complex.

In terms of the three pathways of the complement system, namely the classical pathway, the MBL pathway, and the alternative pathway, current developments are focusing on targets such as C1 (C1q, C1r and C1s), mannose-binding lectin-associated serine protease (MASP), C2, C3, C5, and C6. Several companies are developing polypeptide-like drugs (such as AMY-101 and APL-1) or recombinant enzymes (like CB2782) targeting the critical complement protein C3 to treat diseases including paroxysmal nocturnal hemoglobinuria (PNH), chronic obstructive pulmonary disease (COPD), and age-related macular degeneration (AMD). However, there are currently few drugs targeting the critical complement protein C3 for treating kidney diseases.

Therefore, it is important to provide an oligonucleotide-conjugated compound inhibiting the expression of C3 gene to attenuate the course of disease in patients with the deposition of C3 in renal tissue, ameliorate, prevent and/or treat diseases or conditions mediated by the C3 gene.

### SUMMARY

The present disclosure provides a compound conjugated with an oligonucleotide for use in inhibiting expression of C3 gene, a pharmaceutical composition, and use thereof. The compound conjugated with an oligonucleotide and the pharmaceutical composition thereof provided by the present disclosure can significantly inhibit expression of C3 mRNA in HepG2 cells, can significantly inhibit expression of C3 mRNA in animal liver tissue and C3 protein level in serum, can significantly reduce the C3 protein level in serum of cynomolgus monkeys, can significantly inhibit the activity of complement alternative pathway in serum of cynomolgus monkeys but have no effect on the complement classical pathway in serum, can significantly reduce the urine uTP and UPCR levels and increase urine eGFR level in cynomolgus monkeys with nephropathy, can reduce C3 mRNA and protein levels in CFA-IgA mice, can reduce C3 deposition in renal tissues and improve the C3 deposition course in renal tissues, and have a no-observed-adverse-effect level (NOAEL) in SD rats and cynomolgus monkeys of 300 mg/kg. The compounds conjugated with oligonucleotides and the pharmaceutical compositions thereof provided by the present disclosure are useful for alleviating, preventing and/or treating a disease or condition mediated by dysregulation of C3 gene expression.

In a first aspect of the present disclosure, provided is a compound conjugated with an oligonucleotide having a structure represented by formula (I), or a pharmaceutically acceptable salt thereof:

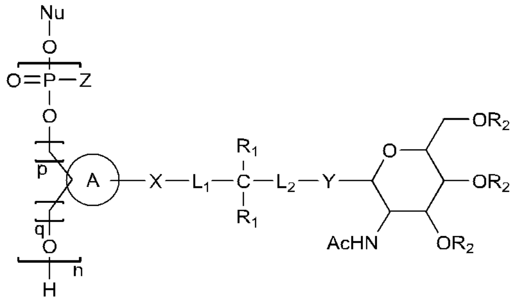

formula (I)

wherein, in the structure,

each A is independently an unsubstituted or substituted 4- to 10-membered aliphatic ring,

n is selected from the group consisting of 1, 2, 3 and 4,

each Z is independently selected from the group consisting of hydroxyl and mercapto,

each p is independently selected from the group consisting of 1, 2 and 3,

each q is independently selected from the group consisting of 1, 2 and 3,

each X is independently selected from the group consisting of NH, O and S,

each $L_1$ is independently selected from the group consisting of

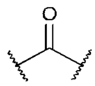, 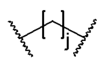

and

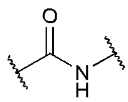,

wherein j is selected from the group consisting of 1, 2, 3, 4, 5, 6, 7, 8, 9 and 10,

each $R_1$ is independently selected from the group consisting of H, $C_1$-$C_6$ alkyl, $C_1$-$C_6$ haloalkyl and $C_1$-$C_6$ alkoxy,

each $L_2$ is independently selected from the group consisting of $C_1$-$C_{30}$ alkylidene and

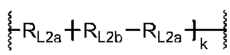,

wherein each $R_{L2a}$, is independently $C_1$-$C_{10}$ alkylidene, each $R_{L2b}$ is independently selected from the group consisting of O, S, NH and -NH-C(O)-, and k is selected from the group consisting of 1, 2, 3, 4, 5, 6, 7, 8, 9 and 10,

each Y is independently selected from the group consisting of NH, O and S, and

each $R_2$ is independently selected from the group consisting of: H,

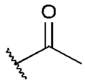, 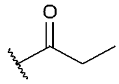,

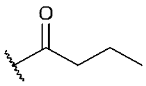, 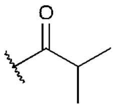, 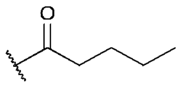, 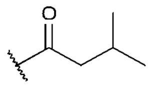, 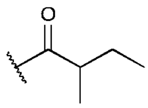, 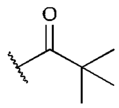, 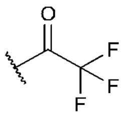

and

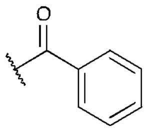,

wherein Nu represents a double-stranded oligonucleotide or a pharmaceutically acceptable salt thereof for reducing the expression of complement 3 (C3), the double-stranded oligonucleotide comprises a sense strand and an antisense strand, wherein the sense strand and the antisense strand form a double-stranded region, the antisense strand comprises a complementary region that has complementarity to a target sequence of C3 mRNA, and the complementary region is 17 to 35 contiguous nucleotides in length.

In some specific embodiments of the present disclosure, the antisense strand of the double-stranded oligonucleotide represented by Nu comprises a nucleotide sequence of or differing by 1 or 2 nucleotides from any one of the sequences set forth in SEQ ID NOs: 2, 4, 6, 8 and 10, and/or, the sense strand of the double-stranded oligonucleotide comprises a nucleotide sequence of or differing by 1 or 2 nucleotides from any one of the sequences set forth in SEQ ID NOs: 1, 3, 5, 7, and 9.

In some specific embodiments of the present disclosure, each nucleotide in the double-stranded oligonucleotide is independently selected from the group consisting of:

2'-fluoro modified nucleotide, 2'-deoxy modified nucleotide, 2'-O-methyl modified nucleotide, 2'-O-$(CH_2)_n$-O-R modified nucleotide, 2'-amino-modified nucleotide, abasic nucleotide, and a nucleotide analogue, wherein the nucleotide analogue is one or more selected from the group consisting of PNA, MNA, BNA, LNA, GNA, TNA and UNA, wherein , n is selected from the group consisting of 1 and 2, R is selected from the group consisting of optionally substituted $C_{1-6}$ alkyl and optionally substituted $C_{1-6}$ alkoxy, when R comprises a substituent, the substituent is selected from the group consisting of halogen, $C_{1-6}$ alkoxy, hydroxyl and amino.

In some specific embodiments of the present disclosure, in the direction from 5' end to 3' end, nucleotides at positions 7 to 10 of the nucleotide sequence of the sense strand of the double-stranded oligonucleotide are 2'-fluoro modified nucleotides, and nucleotides at the other positions in the sense strand are 2'-O-methyl modified nucleotides; nucleotides at positions 2, 6, 14, and 16 of the nucleotide sequence of the antisense strand are 2'-fluoro modified nucleotides, any one of nucleotides at positions 9 to 12 is a 2'-fluoro modified nucleotide, at least one of nucleotides at positions 8 and 15 is a 2'-O-methoxyethyl modified nucleotide, and nucleotides at the other positions in the antisense strand are 2'-O-methyl modified nucleotides.

In some specific embodiments of the present disclosure, in the direction from the 5' end to 3' end, at least one of linkages between the following nucleotides of the sense strand is a phosphorothioate linkage: a linkage between the first nucleotide and the second nucleotide at 5' end of the sense strand and a linkage between the second nucleotide and the third nucleotide at 5' end of the sense strand.

In some specific embodiments of the present disclosure, in the direction from the 5' end to 3' end, at least one of linkages between the following nucleotides of the antisense strand is a phosphorothioate linkage: a linkage between the first nucleotide and the second nucleotide at 5' end of the antisense strand, a linkage between the second nucleotide and the third nucleotide at 5' end of the antisense strand, a linkage between the first nucleotide and the second nucleotide at 3' end of the antisense strand, and a linkage between the second nucleotide and the third nucleotide at 3' end of the antisense strand.

In some specific embodiments of the present disclosure, each nucleotide in the double-stranded oligonucleotide is a modified nucleotide,

the sense strand comprises or is selected from any one of the modified nucleotide sequences set forth in Z1) to Z21), and

the antisense strand comprises or is selected from any one of the modified nucleotide sequences set forth in F1) to F36).

In a second aspect of the present disclosure, provided is a pharmaceutical composition, the pharmaceutical composition comprises the compound conjugated with an oligonucleotide according to the first aspect of the present disclosure.
In a third aspect of the present disclosure, provided is use of the compound conjugated with an oligonucleotide according to the first aspect of the present disclosure or the pharmaceutical composition according to the second aspect of the present disclosure in the manufacture of a medicament for alleviating, preventing and/or treating a C3 gene-mediated disease or condition.
In a fourth aspect of the present disclosure, provided is a kit comprising the compound conjugated with an oligonucleotide according to the first aspect of the present disclosure or the pharmaceutical composition according to the second aspect of the present disclosure.
In a fifth aspect of the present disclosure, provided is a method for inhibiting C3 gene expression in a subject in need thereof, wherein the method comprises administering to the subject the compound conjugated with an oligonucleotide according to the first aspect of the present disclosure or the pharmaceutical composition according to the second aspect of the present disclosure.
In a sixth aspect of the present disclosure, provided is a method for alleviating, treating and/or preventing a C3-mediated disease or condition in a subject in need thereof, wherein the method comprises administering to the subject the compound conjugated with an oligonucleotide according to the first aspect of the present disclosure or the pharmaceutical composition according to the second aspect of the present disclosure.
In some specific embodiments of the present disclosure, the C3 gene-mediated disease or condition includes a disease related to mRNA expression level of C3 gene.
In some specific embodiments of the present disclosure, the C3 gene-mediated disease or condition includes IgA nephropathy, atypical haemolytic uremic syndrome, paroxysmal nocturnal haemoglobinuria (PNH), C3 glomerulopathy, lupus nephitis and membranous nephritis.
The compound conjugated with an oligonucleotide and the pharmaceutical composition thereof provided by the present disclosure can significantly inhibit expression of C3 mRNA in HepG2 cells, can significantly inhibit expression of C3 mRNA in animal liver tissue and C3 protein level in serum, can significantly reduce the C3 protein level in serum of cynomolgus monkeys, can significantly inhibit the activity of complement alternative pathway in serum of cynomolgus monkeys but have no effect on the complement classical pathway in serum, can significantly reduce the urine uTP and UPCR levels and increase urine eGFR level in cynomolgus monkeys with nephropathy, can reduce C3 mRNA and protein levels in CFA-IgA mice, can reduce C3 deposition in renal tissues and improve the C3 deposition course in renal tissues, and have a no-observed-adverse-effect level (NOAEL) in SD rats and cynomolgus monkeys of 300 mg/kg. The compounds conjugated with oligonucleotides and the pharmaceutical compositions thereof provided by the present disclosure are useful for alleviating, preventing and/or treating a disease or condition mediated by dysregulation of C3 gene expression.

## BRIEF DESCRIPTION OF DRAWINGS

FIG. 1 shows the inhibition on C3 mRNA in the liver of cynomolgus monkeys after administration of compounds conjugated with oligonucleotides in Example 2.

FIG. 2 shows the change of C3 protein levels in serum of cynomolgus monkeys after administration of compounds conjugated with oligonucleotides in Example 3.

FIG. 3 shows the change of activity of complement alternative pathway in serum of cynomolgus monkeys after administration of compounds conjugated with oligonucleotides in Example 4.

FIG. 4 shows the change of activity of complement classical pathway in serum of cynomolgus monkeys after administration of compounds conjugated with oligonucleotides in Example 4.

FIG. 5 shows the relative expression levels of SEAP in mouse serum after a single administration of compounds

conjugated with oligonucleotides in Example 5.

FIG. 6 shows the relative expression levels of SEAP in mouse serum at different administration frequencies in Example 5.

FIG. 7 shows the relative expression levels of C3 mRNA in the liver of B-hC3 mice in Example 6.

FIG. 8 shows the relative expression levels of C3 protein in B-hC3 mice serum after a single administration of compounds conjugated with oligonucleotides in Example 6.

FIG. 9 shows the relative expression levels of C3 protein in B-hC3 serum at different administration frequencies in Example 6.

FIG. 10 shows the uTP levels in the cynomolgus monkey nephropathy model after administration of varying doses of compounds conjugated with oligonucleotides in Example 7.

FIG. 11 shows the UPCR levels in the cynomolgus monkey nephropathy model after administration of varying doses of compounds conjugated with oligonucleotides in Example 7.

FIG. 12 shows the eGFR levels in the cynomolgus monkey nephropathy model after administration of varying doses of compounds conjugated with oligonucleotides in Example 7.

FIG. 13 shows the Crea levels in the cynomolgus monkey nephropathy model after administration of varying doses of compounds conjugated with oligonucleotides in Example 7.

FIG. 14a shows the HE staining results (200x) of negative control group, indicating that no abnormalities were observed in the liver; FIG. 14b shows the HE staining results (200x) of RZ002106 high dose group, indicating that mild vacuolar degeneration was observed in cells surrounding the hepatic central vein; FIG. 14c shows the HE staining results (400x) of negative control group, indicating that no abnormalities were observed in the kidney, and FIG. 14d shows the HE staining results (400x) of RZ002106 high dose group, indicating that mild basophilic granules were observed in the tubular epithelial cells of renal cortex.

FIG. 15a shows the HE staining results (400x) of RZ002106 high dose group, indicating that mild basophilic granules were observed in Kupffer cells within the liver sinusoids; FIG. 15b shows the HE staining results (100x) of RZ002106 high dose group, indicating that no abnormal pathological changes in relation to the test sample were observed in the kidneys, FIG. 15c shows the HE staining results (100x) of RZ002106 high dose group, indicating that the increase in mild foamy macrophages was observed in the medulla of the mesenteric lymph nodes, FIG. 15d shows the HE staining results (100x) of RZ002106 high dose group, indicating that the increase in moderate foamy macrophages was observed in the medulla of the inguinal lymph nodes, and FIG. 15e shows the HE staining results (100x) of RZ002106 high dose group, indicating that the increase in mild foamy macrophages was observed in the medulla of the submandibular lymph nodes.

FIG. 16 shows the inhibition on C3 mRNA in the liver of CFA-hIgA mice after administration of compounds conjugated with oligonucleotides in Example 10.

FIG. 17 shows the expression levels of C3 protein in the liver of CFA-hIgA mice after administration of compounds conjugated with oligonucleotides in Example 10.

FIG. 18 shows the CAP activity levels in serum of CFA-hIgA mice after administration of compounds conjugated with oligonucleotides in Example 10.

## DETAILED DESCRIPTION

The present disclosure provides a compound conjugated with an oligonucleotide, a pharmaceutical composition and uses thereof. Those skilled in the art can learn from the content herein and appropriately improve the process parameters for realization. It should be particularly noted that all similar substitutions and modifications are obvious to those skilled in the art, and they are deemed to be included in the present disclosure. The method and the application of the present disclosure have been described through the preferred embodiments, and it is obvious that the method and

application described herein may be changed or appropriately modified and combined to realize and apply the technology of the present disclosure by those skilled in the art without departing from the content, spirit and scope of the present disclosure.

Term explanation

As used herein, the term "including" or "comprising" is open-ended, which includes the contents specified in the present disclosure, and does not exclude other contents.

As used herein, "optional" or "optionally" means that the subsequently described event or condition may or may not occur, and that the description includes instances wherein the event or condition may or may not occur.

As used herein, the term "small interfering RNA (siRNA)" refers to a double-stranded RNA that comprises a sense and an antisense strands, and each strand is 17 to 30 nucleotides in length. The siRNA mediates the targeted cleavage of RNA transcripts via RNA-induced silencing complex (RISC) pathway through the formation of a RISC. Specifically, the siRNA directs the specific degradation of mRNA sequences through the known process of RNA interference (RNAi), which inhibits the translation of mRNA into amino acids and thereby transformation into proteins. For example, siRNA can regulate (e.g., inhibit) the expression of C3 in cells.

As used herein, the term "antisense strand (or called as guide strand)" includes a region that is substantially complementary to a target sequence (e.g., C3 mRNA). The term "sense strand (or called as passenger strand)" refers to an iRNA strand that comprises a region that is substantially complementary to the antisense strand. The term "substantially complementary" means fully complementary or at least partially complementary. For example, the antisense strand is fully complementary or at least partially complementary to a target sequence. In the case of partial complementarity, a mismatch may be present within the internal or end region of the molecule, wherein the most tolerated mismatch is present within the end region, e.g., within the 5, 4, 3 or 2 nucleotides at the 5'- and/or 3'-end of the iRNA.

It is noted that the antisense strand being "at least partially substantially complementary" to the mRNA means that the antisense strand has a polynucleotide that is substantially complementary to a contiguous portion of the mRNA of interest. Alternatively, when a polynucleotide is substantially non-intermittently complementary to a portion of the mRNA encoding C3, the antisense strand is complementary to at least a portion of the C3 mRNA.

As used herein, the term "target sequence" refers to a contiguous portion of the nucleotide sequence of the mRNA molecule formed during transcription of the C3 gene, including mRNA that is the processed product of the primary transcription product RNA. C3 can be found in a cell, e.g., a cell in a subject.

As used herein, the term "complementary" refers to the ability of an oligonucleotide of a first sequence to hybridize with an oligonucleotide of a second sequence under certain conditions to form a double-stranded structure.

As used herein, the term "substantially complementary" means that there are not more than 3 nucleotide mispairings, not more than 2 nucleotide mispairings, or not more than 1 nucleotide mispairing between the sense and antisense strands in the double-stranded region, such as 3 nucleotide mispairings, 2 nucleotide mispairings, 1 nucleotide mispairing, and 0 nucleotide mispairing, while the ability to hybridize under the relevant conditions is retained. Additionally, in cases where one or more single-stranded protruding terminus formed when two oligonucleotides are designed for hybridization, such protruding terminus should not be considered as mispairings in terms of determining complementarity. In the present disclosure, the "complementary" sequence may also include, or be formed exclusively from non-Watson-Crick base pairs and/or from non-natural as well as modified nucleotides in terms of meeting the above hybridization capability requirements. Such non-Watson-Crick base pairs include, but are not limited to, G:·U wobble base pair or Hoogstein base pair. Correspondingly, in the present disclosure, unless otherwise specified, "mispairing" means that in the siRNA duplex molecule, the bases at corresponding sites are not presented in a manner of being complementarily paired.

As used herein, the term "nucleotide difference", the term "nucleotide base difference" and the term "nucleotide sequence difference" can be used interchangeably, which refers to a change in the base type of a nucleotide at the same or a corresponding position compared to the original nucleotide sequence. For example, if a nucleotide base in the original nucleotide sequence is A, and a nucleotide base at the same or corresponding position is changed to U, C, G, dT, dC, dG, or the like, it is considered that there is a difference in the nucleotide sequence at that position. It should be noted that in the case where the nucleotides at the same or corresponding positions differ only in the presence or absence of a modification or the type of modification as compared to the original nucleotide sequence, a difference in the nucleotide sequence at the position is not considered to exist.

As used herein, the term "protruding terminus" refers to at least one unpaired nucleotide protruding from the double helix structure of a double-stranded oligonucleotide, which is also a nucleotide sequence other than the double-stranded region in siRNA structure. For example, a nucleotide protruding terminus is present when the 3' end of one strand of the sense and/or antisense strand extends beyond the 5' end of the other strand, or when the 5' end of one strand of the sense and/or antisense strand extends beyond the 3' end of the other strand. The protruding terminus may comprise at least one nucleotide, at least two nucleotides, at least three nucleotides, at least four nucleotides, at least five nucleotides, or more nucleotides. The nucleotide protruding terminus may comprise, or consist of, a nucleotide/nucleoside analogue,

including a deoxyribonucleotide/nucleoside. The protruding terminus may be located on the sense strand, the antisense strand, or any combination thereof. In addition, the nucleotide at the protruding terminus may present at the 5' end, the 3' end, or both ends of the antisense or the sense strand.

As used herein, the term "DEPC $H_2O$" is ultrapure water (Type I water) treated with diethyl pyrocarbonate (DEPC) and sterilized under high temperature and high pressure.

As used herein, the term "subject" refers to any animal that is examined, studied, or treated, and it is not intended to limit the present disclosure to any particular type of subject. In some embodiments of the present disclosure, human is a preferred subject, while in some other embodiments, a non-human animal is a preferred subject, including, but not limited to, a mouse, monkey, ferret, cow, sheep, goat, pig, chicken, turkey, dog, cat, horse, and reptile.

As used herein, the term "inhibiting expression of C3 gene" includes any level of inhibition on C3 gene, e.g., at least partial inhibition on expression of C3 gene, such as inhibition by at least about 20%, at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 91%, at least about 92%, at least about 93%, at least about 94%, at least about 95%, at least about 96%, at least about 97%, at least about 98%, or at least about 99%.Wherein, the expression of C3 gene can be evaluated based on the level of any variable associated with the expression of C3 gene, e.g., mRNA levels or protein levels of C3. Inhibition can be evaluated by a reduction in the absolute or relative level of one or more of these variables compared to control levels. The control level may be any type of control level utilized in the art, e.g., a baseline level before administration, or a measured level of a similar subject, cell, or sample that has not been treated or treated with a control (e.g., a control containing only buffer or control without containing an active ingredient).

As used herein, "conjugating" refers to two or more chemical moieties being linked to each other via a covalent linkage. A "conjugate" refers to a compound formed by covalent linkage of individual chemical moieties. A "conjugating molecule" can be understood as a specific compound capable of being conjugated with a siRNA via reactions, thus finally forming the oligonucleotide conjugate of the present disclosure

As used herein, a "pharmaceutical composition" may be useful in the treatment of a disease or the *in vitro* cell culture. When used in the treatment of a disease, the term "pharmaceutical composition" generally is in a unit dose form and can be prepared by any of the methods known in the pharmaceutical field. All methods comprise a step of combining an active ingredient with one or more excipients as accessory ingredients. Typically, the composition is prepared by uniformly and sufficiently mixing the active siRNA with a liquid excipient, a finely divided solid excipient, or both.

As used herein, the term "pharmaceutically acceptable" means that the substance or composition must be chemically and/or toxicologically compatible with the other ingredients of the formulation and/or with the mammal being treated therewith. Preferably, "pharmaceutically acceptable" in the present disclosure means approved by a regulatory agency of the Federal or a state government or listed in the U.S. Pharmacopeia or other generally recognized pharmacopeia for use in animals, and particularly in humans.

As used herein, the term "pharmaceutically acceptable excipient" may include any solvent, solid excipient, diluent, other liquid excipient, etc., which is suitable for the specific target dosage form. Except to the extent that any conventional excipient is incompatible with the siRNA of the present disclosure, for example, producing any adverse biological effects or harmful interactions with any other components of a pharmaceutically acceptable composition, the uses thereof are also contemplated by the present disclosure.

As used herein, except to the extent that any conventional excipient is incompatible with the siRNA of the present disclosure, for example, producing any adverse biological effects or harmful interactions with any other components of a pharmaceutically acceptable composition, the uses thereof are also contemplated by the present disclosure.

<u>Compound conjugated with an oligonucleotide</u>

In a first aspect of the present disclosure, provided is a compound conjugated with an oligonucleotide or a pharmaceutically acceptable salt thereof, wherein the compound has a structure represented by formula (I):

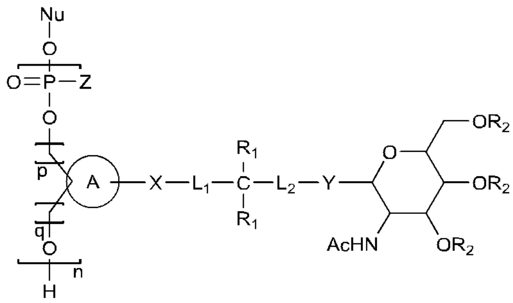

formula (I)

wherein, in the structure,

each A is independently an unsubstituted or substituted 4- to 10-membered aliphatic ring,

n is selected from the group consisting of 1, 2, 3 and 4,

each Z is independently selected from the group consisting of hydroxyl and mercapto,

each p is independently selected from the group consisting of 1, 2 and 3,

each q is independently selected from the group consisting of 1, 2 and 3,

each X is independently selected from the group consisting of NH, O and S,

each $L_1$ is independently selected from the group consisting of

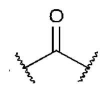, 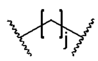

and

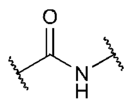,

wherein j is selected from the group consisting of 1, 2, 3, 4, 5, 6, 7, 8, 9 and 10,

each $R_1$ is independently selected from the group consisting of H, $C_1$-$C_6$ alkyl, $C_1$-$C_6$ haloalkyl and $C_1$-$C_6$ alkoxy,

each $L_2$ is independently selected from the group consisting of $C_1$-$C_{30}$ alkylidene and

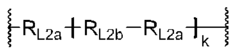,

wherein each $R_{L2a}$ is independently $C_1$-$C_{10}$ alkylidene, each $R_{L2b}$ is independently selected from the group consisting of O, S, NH and -NH-C(O)-, and k is selected from the group consisting of 1, 2, 3, 4, 5, 6, 7, 8, 9 and 10,

each Y is independently selected from the group consisting of NH, O and S, and

each $R_2$ is independently selected from the group consisting of: H,

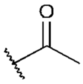, 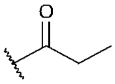,

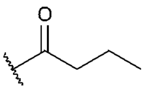, 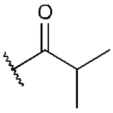, 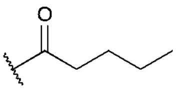, 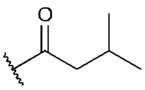, 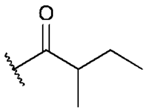, 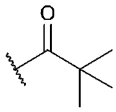, 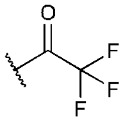

and

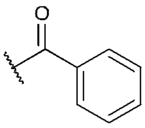.

In some embodiments of the present disclosure, each A is independently selected from the group consisting of an unsubstituted or substituted 4- to 10-membered cycloalkane group and an unsubstituted or substituted 4- to 10-membered cycloolefine group.

In some embodiments of the present disclosure, each A is independently an unsubstituted or substituted 4- to 10-membered cycloalkane group.

In some embodiments of the present disclosure, each A is independently a 4- to 10-membered cycloalkane group, such as a monocyclic ring, a spirocyclic ring, or a bridged ring.

In some embodiments of the present disclosure, each A is independently selected from the group consisting of

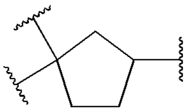, 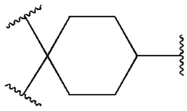, 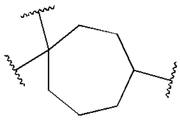, 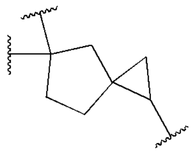

and

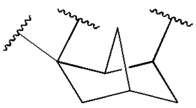.

In some specific embodiments of the present disclosure, each A is

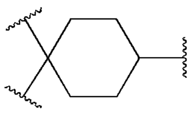.

In some specific embodiments of the present disclosure, the compound conjugated with an oligonucleotide has a structure represented by formula (II), or a pharmaceutically acceptable salt thereof:

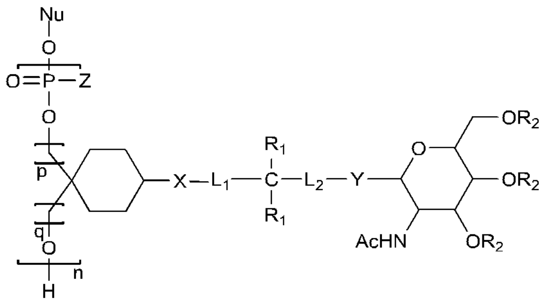

formula (II)

in formula (II), p, q, n, Z, X, Y, $L_1$, $L_2$ and $R_1$ are as defined above, and $R_2$ is H.

In some specific embodiments of the present disclosure, each X is NH.

In some specific embodiments of the present disclosure, each $L_1$ is

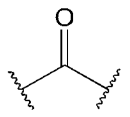

In some specific embodiments of the present disclosure, the compound conjugated with an oligonucleotide has a structure represented by formula (III), or a pharmaceutically acceptable salt thereof:

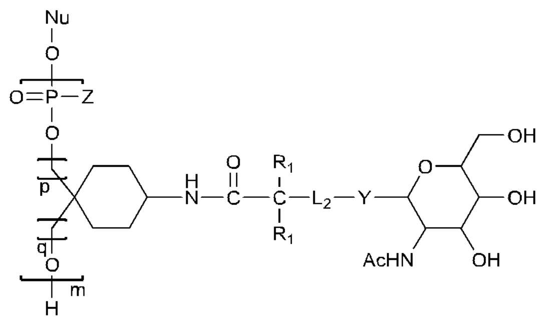

formula (III)

in formula (III), m is selected from the group consisting of 1, 2, 3 and 4, and the other substituents are as defined above.

In some specific embodiments of the present disclosure, each Z is hydroxyl.

In some embodiments of the present disclosure, each p is independently selected from the group consisting of 1 and 2.

In some specific embodiments of the present disclosure, each p is 1.

In some embodiments of the present disclosure, each q is independently selected from the group consisting of 1 and 2.

In some specific embodiments of the present disclosure, each q is 1.

In some specific embodiments of the present disclosure, each p is 1 and each q is 1.

In some specific embodiments of the present disclosure, each $R_1$ is H.

In some specific embodiments of the present disclosure, each Y is O.

Further, in some specific embodiments of the present disclosure, the compound conjugated with an oligonucleotide has a structure represented by formula (IV), or a pharmaceutically acceptable salt thereof,

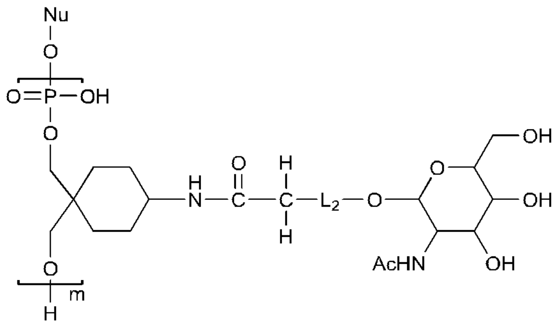

formula (IV)

in formula (IV), m is selected from the group consisting of 1, 2, 3 and 4, and $L_2$ is independently selected from the group consisting of

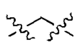, 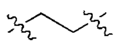, 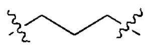,

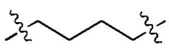, 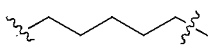, 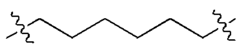, 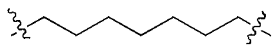,

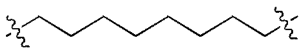, 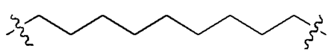, 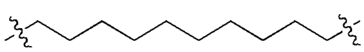,

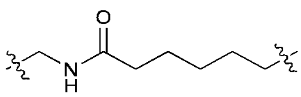, 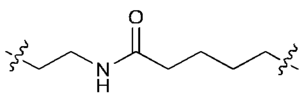, 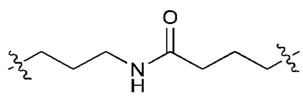,

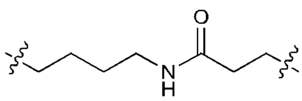 and 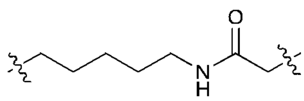.

In some alternative embodiments of the present disclosure, each $L_2$ is independently selected from the group consisting of

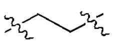 and 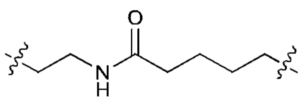.

In some specific embodiments of the present disclosure, $L_2$ is

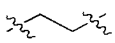.

In some specific embodiments of the present disclosure, $L_2$ is

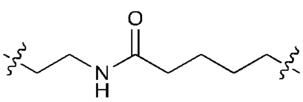.

In some specific embodiments of the present disclosure, the compound conjugated with an oligonucleotide has a structure selected from the group consisting of:

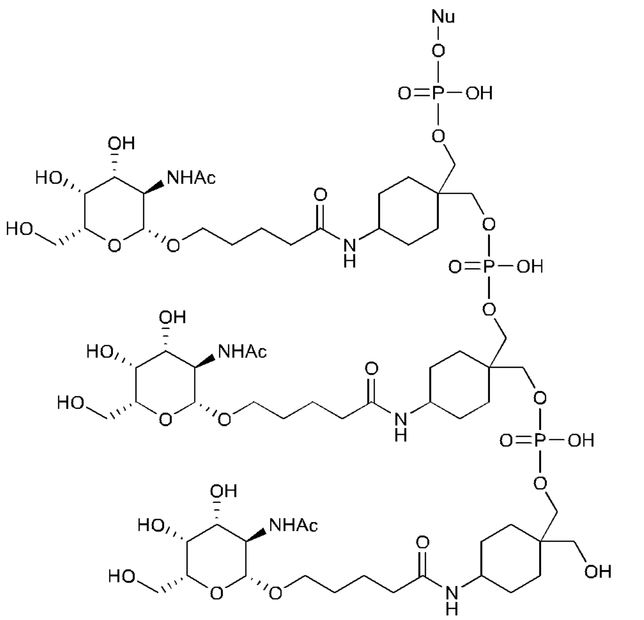

CR01008

and

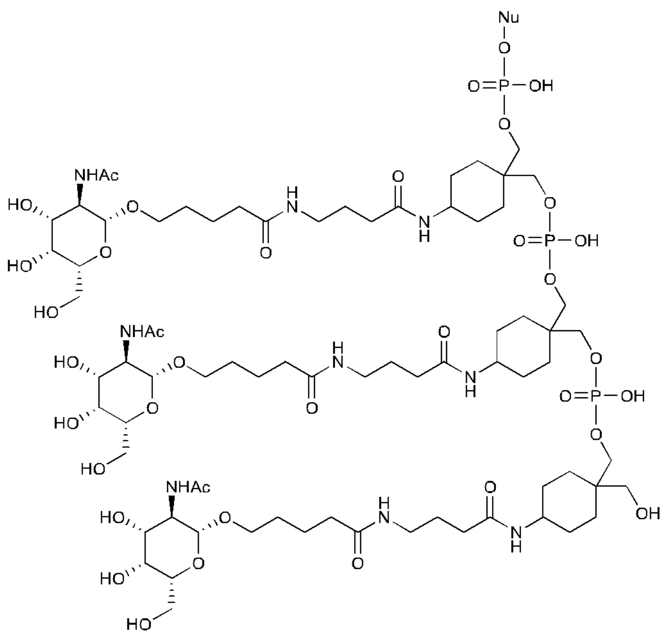

CR01013,

or a pharmaceutically acceptable salt thereof.

Wherein, Nu represents a double-stranded oligonucleotide or a pharmaceutically acceptable salt thereof for reducing the expression of complement 3 (C3), the double-stranded oligonucleotide comprises a sense strand and an antisense strand, wherein the sense strand and the antisense strand are complementary or substantially complementary to form a double-stranded region, and the "substantially complementary" means that there are not more than 3 nucleotide mispairings between the sense and antisense strands in the double-stranded region.

The antisense strand comprises a complementary region that has complementarity to a target sequence of C3 mRNA, and the complementary region is 17 to 35 contiguous nucleotides in length. Further, in the direction from 5' end to 3'

end, nucleotides at positions 2 to 19 of the antisense strand comprise a complementary region that has complementarity to a target sequence of C3 mRNA.

In some specific embodiments of the present disclosure, the sense strand of Nu in the compound conjugated with an oligonucleotide is linked to a phosphate group at 3' end.

In some specific embodiments of the present disclosure, the antisense strand of the double-stranded oligonucleotide represented by Nu comprises a nucleotide sequence of or differing by 1 or 2 nucleotides from any one of the sequences set forth in SEQ ID NOs: 2, 4, 6, 8 and 10.

In some specific embodiments of the present disclosure, the sense strand of the double-stranded oligonucleotide represented by Nu comprises a nucleotide sequence of or differing by 1 or 2 nucleotides from any one of the sequences set forth in SEQ ID NOs: 1, 3, 5, 7, and 9.

In some specific embodiments of the present disclosure, the antisense strand of the double-stranded oligonucleotide represented by Nu comprises a nucleotide sequence selected from the group consisting of the sequences set forth in SEQ ID NOs: 2, 4, 6, 8 and 10, and the sense strand of the double-stranded oligonucleotide represented by Nu comprises a nucleotide sequence selected from the group consisting of the sequences set forth in SEQ ID NOs: 1, 3, 5, 7, and 9.

In some specific embodiments of the present disclosure, the double-stranded oligonucleotide is one or more selected from the group consisting of:

1) an antisense strand having a nucleotide sequence of or differing by 1 or 2 nucleotides from the nucleotide sequence set forth in SEQ ID NO: 2 and a sense strand having a nucleotide sequence of or differing by 1 or 2 nucleotides from the nucleotide sequence set forth in SEQ ID NO: 1,

2) an antisense strand having a nucleotide sequence of or differing by 1 or 2 nucleotides from the nucleotide sequence set forth in SEQ ID NO: 4 and a sense strand having a nucleotide sequence of or differing by 1 or 2 nucleotides from the nucleotide sequence set forth in SEQ ID NO: 3,

3) an antisense strand having a nucleotide sequence of or differing by 1 or 2 nucleotides from the nucleotide sequence set forth in SEQ ID NO: 6 and a sense strand having a nucleotide sequence of or differing by 1 or 2 nucleotides from the nucleotide sequence set forth in SEQ ID NO: 5,

4) an antisense strand having a nucleotide sequence of or differing by 1 or 2 nucleotides from the nucleotide sequence set forth in SEQ ID NO: 8 and a sense strand having a nucleotide sequence of or differing by 1 or 2 nucleotides from the nucleotide sequence set forth in SEQ ID NO: 7, and

5) an antisense strand having a nucleotide sequence of or differing by 1 or 2 nucleotides from the nucleotide sequence set forth in SEQ ID NO: 10 and a sense strand having a nucleotide sequence of or differing by 1 or 2 nucleotides from the nucleotide sequence set forth in SEQ ID NO: 9.

In some specific embodiments of the present disclosure, the double-stranded oligonucleotide is one or more selected from the group consisting of:

1) an antisense strand having a nucleotide sequence set forth in SEQ ID NO: 2 and a sense strand having a nucleotide sequence set forth in SEQ ID NO: 1,

2) an antisense strand having a nucleotide sequence set forth in SEQ ID NO: 4 and a sense strand having a nucleotide sequence set forth in SEQ ID NO: 3,

3) an antisense strand having a nucleotide sequence set forth in SEQ ID NO: 6 and a sense strand having a nucleotide sequence set forth in SEQ ID NO: 5,

4) an antisense strand having a nucleotide sequence set forth in SEQ ID NO: 8 and a sense strand having a nucleotide sequence set forth in SEQ ID NO: 7, and

5) an antisense strand having a nucleotide sequence set forth in SEQ ID NO: 10 and a sense strand having a nucleotide sequence set forth in SEQ ID NO: 9.

In some specific embodiments of the present disclosure, the double-stranded oligonucleotide comprises:
an antisense strand having a nucleotide sequence of or differing by 1 or 2 nucleotides from the nucleotide sequence set

forth in SEQ ID NO: 2 and a sense strand having a nucleotide sequence of or differing by 1 or 2 nucleotides from the nucleotide sequence set forth in SEQ ID NO: 1.

In some specific embodiments of the present disclosure, the double-stranded oligonucleotide comprises: an antisense strand having a nucleotide sequence set forth in SEQ ID NO: 2 and a sense strand having a nucleotide sequence set forth in SEQ ID NO: 1.

In some embodiments of the present disclosure, the siRNA may also contain modified nucleotides as required, and the modified nucleotides have no effect of weakening or invalidating the function of the siRNA to inhibit expression of C3 gene. At present, there are many ways to modify siRNA in this field, including, for example, backbone modification (such as phosphate group modification), ribose group modification and base modification. In some embodiments of the present disclosure, at least one nucleotide in the sense strand or antisense strand of the siRNA is a modified nucleotide, for example, the modified nucleotide is a nucleotide group in which a ribose group and an optional phosphate group are modified, but not limited thereto. In some specific embodiments of the present disclosure, each nucleotide in the double-stranded oligonucleotide is independently a modified or unmodified nucleotide.

In some specific embodiments of the present disclosure, substantially all nucleotides in the double-stranded oligonucleotide are independently selected from modified nucleotides. Wherein, "substantially all nucleotides are selected from modified nucleotides" means that most but not all of the nucleotides in the double-stranded oligonucleotide are modified, and the double-stranded oligonucleotide may contain no more than 5, 4, 3, 2 or 1 unmodified nucleotide.

**In** some specific embodiments of the present disclosure, all nucleotides in the double-stranded oligonucleotide are independently selected from modified nucleotides.

Wherein the nucleotide has a structure of

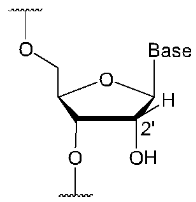

,

Base represents a nucleoside base, and the nucleoside base on each nucleotide is independently selected from the group consisting of uracil U, thymine T, cytosine C, adenine A, and guanine G.

In some specific embodiments of the present disclosure, each nucleotide in the double-stranded oligonucleotide is independently one or more selected from the group consisting of:

2'-fluoro modified nucleotide, 2'-deoxy modified nucleotide, 2'-O-methyl modified nucleotide, 2'-O-$(CH_2)_n$-O-R modified nucleotide, 2'-amino-modified nucleotide, abasic nucleotide, and a nucleotide analogue; wherien the nucleotide analogue is selected from the group consisting of peptide nucleic acid (PNA), morpholino (MNA), bridged nucleic acid (BNA), locked nucleic acid (LNA), glycol nucleic acid (GNA), threose nucleic acid (TNA) and unlocked nucleic acid (UNA).

Wherein, n is selected from the group consisting of 1 and 2, R is selected from the group consisting of optionally substituted $C_{1-6}$ alkyl and optionally substituted $C_{1-6}$ alkoxy, when R comprises a substituent, the substituent is selected from the group consisting of halogen, $C_{1-6}$ alkoxy, hydroxyl and amino.

In the present disclosure, a 2'-O-$(CH_2)_n$-R modified nucleotide means that a hydrogen atom on the hydroxyl group at the 2'position of the ribosyl group of the nucleotide is substituted by -$(CH_2)_n$-R. Wherein, when n is 1, the 2'-O-$(CH_2)_n$-R-modified nucleotide is selected from the group consisting of a 2'-O-ethoxymethyl-modified nucleotide and a 2'-O-2,2,2-trifluoroethoxymethyl-modified nucleotide. When n is 2, the 2'-O-$(CH_2)_n$-R modified nucleotide is a 2'-O-methoxyethyl modified nucleotide (also known as a 2'-O-moe modified nucleotide).

In some specific embodiments of the present disclosure, the 2'-O-$(CH_2)_n$-O-R-modified nucleotide is selected from the group consisting of a 2'-O-methoxyethyl-modified nucleotide and a 2'-O-ethoxymethyl-modified nucleotide.

In some specific embodiments of the present disclosure, the double-stranded oligonucleotide comprises at least one 2'-O-methoxyethyl modified nucleotide.

In some specific embodiments of the present disclosure, the sense strand is 18 to 21 nucleotides in length and the antisense strand is 19 to 23 nucleotides in length.

In some specific embodiments of the present disclosure, the sense strand is 17 to 21 nucleotides in length and the antisense strand is 19 to 23 nucleotides in length.

In some specific embodiments of the present disclosure, the sense strand is 19 nucleotides in length and the antisense strand is 21 nucleotides in length.

In some specific embodiments of the present disclosure, the antisense strand comprises at least one 2'-O-methoxyethyl modified nucleotide.

In some specific embodiments of the present disclosure, in the direction from 5' end to 3' end, nucleotides at

positions 7 to 10 of the nucleotide sequence of the sense strand of the double-stranded oligonucleotide are 2'-fluoro modified nucleotides, and nucleotides at the other positions in the sense strand are 2'-O-methyl modified nucleotides; nucleotides at positions 2, 6, 14, and 16 of the nucleotide sequence of the antisense strand are 2'-fluoro modified nucleotides, any one of nucleotides at positions 9 to 12 is a 2'-fluoro modified nucleotide, at least one of nucleotides at positions 8 and 15 is a 2'-O-methoxyethyl modified nucleotide, and nucleotides at the other positions in the antisense strand are 2'-O-methyl modified nucleotides.

In some specific embodiments of the present disclosure, in the direction from 5' end to 3' end, nucleotides at positions 7 to 10 of the nucleotide sequence of the sense strand of the double-stranded oligonucleotide are 2'-fluoro modified nucleotides, and nucleotides at the other positions in the sense strand are 2'-O-methyl modified nucleotides; nucleotides at positions 2, 6, 14, and 16 of the nucleotide sequence of the antisense strand are 2'-fluoro modified nucleotides, any one of nucleotides at positions 9 to 12 is a 2'-fluoro modified nucleotide, a nucleotide at positions 8 or 15 is a 2'-O-methoxyethyl modified nucleotide, and nucleotides at the other positions in the antisense strand are 2'-O-methyl modified nucleotides.

In some specific embodiments of the present disclosure, the sense strand and the antisense strand in the double-stranded oligonucleotide have a modification selected from the group consisting of (ds-1) to (ds-8):

(ds-1) in the direction from 5' end to 3' end, in the sense strand, nucleotides at positions 7 to 10 of the nucleotide sequence are 2'-fluoro modified nucleotides, and nucleotides at the other positions are 2'-O-methyl modified nucleotides; in the antisense strand, nucleotides at positions 2, 6, 9, 14, and 16 of the nucleotide sequence are 2'-fluoro modified nucleotides, a nucleotide at position 8 is a 2'-O-methoxyethyl-modified nucleotide, and nucleotides at the other positions are 2'-O-methyl-modified nucleotides,

(ds-2) in the direction from 5' end to 3' end, in the sense strand, nucleotides at positions 7 to 10 of the nucleotide sequence are 2'-fluoro modified nucleotides, and nucleotides at the other positions are 2'-O-methyl modified nucleotides; in the antisense strand, nucleotides at positions 2, 6, 10, 14, and 16 of the nucleotide sequence are 2'-fluoro modified nucleotides, a nucleotide at position 8 is a 2'-O-methoxyethyl-modified nucleotide, and nucleotides at the other positions are 2'-O-methyl-modified nucleotides,

(ds-3) in the direction from 5' end to 3' end, in the sense strand, nucleotides at positions 7 to 10 of the nucleotide sequence are 2'-fluoro modified nucleotides, and nucleotides at the other positions are 2'-O-methyl modified nucleotides; in the antisense strand, nucleotides at positions 2, 6, 11, 14, and 16 of the nucleotide sequence are 2'-fluoro modified nucleotides, a nucleotide at position 8 is a 2'-O-methoxyethyl-modified nucleotide, and nucleotides at the other positions are 2'-O-methyl-modified nucleotides,

(ds-4) in the direction from 5' end to 3' end, in the sense strand, nucleotides at positions 7 to 10 of the nucleotide sequence are 2'-fluoro modified nucleotides, and nucleotides at the other positions are 2'-O-methyl modified nucleotides; in the antisense strand, nucleotides at positions 2, 6, 12, 14, and 16 of the nucleotide sequence are 2'-fluoro modified nucleotides, a nucleotide at position 8 is a 2'-O-methoxyethyl-modified nucleotide, and nucleotides at the other positions are 2'-O-methyl-modified nucleotides,

(ds-5) in the direction from 5' end to 3' end, in the sense strand, nucleotides at positions 7 to 10 of the nucleotide sequence are 2'-fluoro modified nucleotides, and nucleotides at the other positions are 2'-O-methyl modified nucleotides; in the antisense strand, nucleotides at positions 2, 6, 9, 14, and 16 of the nucleotide sequence are 2'-fluoro modified nucleotides, a nucleotide at position 15 is a 2'-O-methoxyethyl-modified nucleotide, and nucleotides at the other positions are 2'-O-methyl-modified nucleotides,

(ds-6) in the direction from 5' end to 3' end, in the sense strand, nucleotides at positions 7 to 10 of the nucleotide sequence are 2'-fluoro modified nucleotides, and nucleotides at the other positions are 2'-O-methyl modified nucleotides; in the antisense strand, nucleotides at positions 2, 6, 10, 14, and 16 of the nucleotide sequence are 2'-fluoro modified nucleotides, a nucleotide at position 15 is a 2'-O-methoxyethyl-modified nucleotide, and nucleotides at the other positions are 2'-O-methyl-modified nucleotides,

(ds-7) in the direction from 5' end to 3' end, in the sense strand, nucleotides at positions 7 to 10 of the nucleotide sequence are 2'-fluoro modified nucleotides, and nucleotides at the other positions are 2'-O-methyl modified nucleotides; in the antisense strand, nucleotides at positions 2, 6, 11, 14, and 16 of the nucleotide sequence are 2'-fluoro modified nucleotides, a nucleotide at position 15 is a 2'-O-methoxyethyl-modified nucleotide, and nucleotides at the other positions are 2'-O-methyl-modified nucleotides, and

(ds-8) in the direction from 5' end to 3' end, in the sense strand, nucleotides at positions 7 to 10 of the nucleotide sequence are 2'-fluoro modified nucleotides, and nucleotides at the other positions are 2'-O-methyl modified nucleotides; in the antisense strand, nucleotides at positions 2, 6, 12, 14, and 16 of the nucleotide sequence are 2'-fluoro modified nucleotides, a nucleotide at position 15 is a 2'-O-methoxyethyl-modified nucleotide, and nucleotides at the other positions are 2'-O-methyl-modified nucleotides.

In some specific embodiments of the present disclosure, the sense strand and/or the antisense strand independently comprise one or more phosphorothioate linkages between nucleotides.

In some specific embodiments of the present disclosure, in the direction from the 5' end to 3' end, at least one of the following linkages is a phosphorothioate linkage:

(1) a linkage between the first nucleotide and the second nucleotide at 5' end of the sense strand, and

(2) a linkage between the second nucleotide and the third nucleotide at 5' end of the sense strand.

In some specific embodiments of the present disclosure, in the direction from the 5' end to 3' end, at least one of the following linkages is a phosphorothioate linkage:

(1) a linkage between the first nucleotide and the second nucleotide at 5' end of the antisense strand,

(2) a linkage between the second nucleotide and the third nucleotide at 5' end of the antisense strand,

(3) a linkage between the first nucleotide and the second nucleotide at 3' end of the antisense strand, and

(4) a linkage between the second nucleotide and the third nucleotide at 3' end of the antisense strand.

In some optional embodiments of the present disclosure, the sense strand or the antisense strand comprises a 3' protruding terminus having at least 1 nucleotide.

In some optional embodiments of the present disclosure, the antisense strand comprises a 3' protruding terminus having at least 1 nucleotide.

In some optional embodiments of the present disclosure, the antisense strand comprises a 3' protruding terminus having 2 nucleotides.

In some specific embodiments of the present disclosure, each nucleotide in the double-stranded oligonucleotide is a modified nucleotide.

In some specific embodiments of the present disclosure, the sense strand comprises or is selected from any one nucleotide sequence selected from the group consisting of modified nucleotide sequences set forth in Z1) to Z21):
in the direction from the 5' end to 3' end of the sense strand:

Z1) CmsGmsAmAmGmCmUfCfAfUfGmAmAmUmAmUmAmUmUm

Z2) CmsGmsAmAmG(moe)CmUfCfAfUfGmAmAmUmAmUmAmUmUm

Z3) CmsGmsAmAmGmCmUfCfAfUfGmA(moe)AmUmAmUmAmUmUm

Z4) CmsGmsAmAmGmCmUfCfAfUfGmAmA(moe)UmAmUmAmUmUm

Z5) CmsGmsAmAmGmCmUfCfAfUfGmAmAmUmAmUmAmT(moe)Um

Z6) CmsAmsGmAmGmAmAfAfUfUfCmUmAmCmUmAmCmAmUm,

Z7) CmsAmsGmAmG(moe)AmAfAfUfUfCmUmAmCmUmAmCmAmUm,

Z8) CmsAmsGmAmGmAmAfAfUfCTfCmT(moe)AmCmUmAmCmAmUm,

Z9) CmsAmsGmAmGmAmAfAfUfCTfCmUmA(moe)CmUmAmCmAmUm

Z10) CmsAmsGmAmGmAmAfAfUfCTfCmUmAmCmUmAmCmA(moe)Um

Z11) GmsAmsGmAmAmUmUfGfCfUfUmCmAmUmAmCmAmAmAm

Z12) GmsAmsGmAmA(moe)UmUfGfCfCTfUmCmAmUmAmCmAmAmAm

Z13) GmsAmsGmAmAmUmUfGfCfCTfUmC(moe)AmUmAmCmAmAmAm

Z14) GmsAmsGmAmAmUmUfGfCfCTfUmCmA(moe)UmAmCmAmAmAm

Z15) GmsAmsGmAmAmUmUfGfCfCTfUmCmAmUmAmCmAmA(moe)Am

Z16) CmsAmsAmCmUmCmAfCfCfUfGmUmAmAmUmAmAmAmUm

Z17) CmsAmsAmCmT(moe)CmAfCfCfCTfGmUmAmAmUmAmAmAmUm

Z18) CmsAmsAmCmUmCmAfCfCfUfGmT(moe)AmAmUmAmAmAmUm

Z19) CmsAmsAmCmUmCmAfCfCfUFGmUmMA(moe)AmUmAmAmAmUm

Z20) CmsAmsAmCmUmCmAfCfCfUfGmUmAmAmUmAmAmA(moe)Um

Z21) AmsAmsAmUmUmCmUfAfCfUfAmCmAmUmCmUmAmUmAm.

In some specific embodiments of the present disclosure, the antisense strand comprises or is selected from any one nucleotide sequence selected from the group consisting of modified nucleotide sequences set forth in F1) to F36): in the direction from the 5' end to 3' end of the antisense strand:

F1) AmsAfsUmAmUmAfUmUmCfAmUmGmAmGfC(moe)UfUmCmGmsUmsAm
F2) AmsAfsUmAmUmAfUmUmCmAfUmGmAmGfC(moe)UfUmCmGmsUmsAm
F3) AmsAfsUmAmUmAfUmUmCmAmUfGmAmGfC(moe)UfUmCmGmsUmsAm
F4) AmsAfsUmAmUmAfUmT(moe)CmAfUmGmAmGfCmUfUmCmGmsUmsAm
F5) AmsUfsGmUmAmGfUmAmGfAmAmUmUmUfC(moe)UfCmUmGmsUmsAm
F6) AmsUfsGmMUmMAmGfUnmAmMGmAfAmUmUmUfC(moe)UfCmUmGmsUmsAm
F7) AmsUfsGmUmAmGfUmAmGmAmAfUmUmUfC(moe)UfCmUmGmsUmsAm
F8) AmsUfsGmUmAmGfUmA(moe)GmAfAmUmUmUfCmUfCmUmGmsUmsAm
F9) UmsUfsUmGmUmAfUmGmAfAmGmCmAmAfT(moe)UfCmUmCmsCmsUm
F10) UmsUfsUmGmUmAfUmGmAmAfGmCmAmAfT(moe)UfCmUmCmsCmsUm
F11) UmsUfsUmGmUmAfUmGmAmAmGfCmAmAfT(moe)UfCmUmCmsCmsUm
F12) UmsUfsUmGmUmAfUmG(moe)AmAfGmCmAmAfUmUfCmUmCmsCmsUm
F13) AmsUfsUmUmAmUfUmAmCfAmGmGmUmGfA(moe)GfUmUmGmsAmsUm
F14) AmsUfsUmUmAmUfUmAmCmAfGmGmUmGfA(moe)GfUmUmGmsAmsUm
F15) AmsUfsUmUmAmUfUmAmCmAmGfGmUmGfA(moe)GfUmUmGmsAmsUm
F16) AmsUfsUmUmAmUfUmA(moe)CmAfGmGmUmGfAmGfUmUmGmsAmsUm
F17) AmsAfsUmAmUmAfUmT(moe)CmAmUfGmAmGfCmUfUmCmGmsUmsAm
F18) AmsUfsGmUmAmGfUmA(moe)GmAmAfUmUmUfCmUfCmUmGmsUmsAm
F19) AmsUfsUmUmAmUfUmA(moe)CmAmGfGmUmGfAmGfUmUmGmsAmsUm
F20) AmsUfsGmUmAmGfUmAmGmAmAmUfUmUfC(moe)UfCmUmGmsUmsAm
F21) UmsAfsUmAmGmAfUmGmUmAmGmUfAmGfAmAfUmUmUmsCmsUm
F22) UmsAfsUmAmGmAfUmGmUfAmGmUmAmGfA(moe)AfUmUmUmsCmsUm
F23) UmsAfsUmAmGmAfUmGmUmAmGmUfAmGfA(moe)AfUmUmUmsCmsUm
F24) AmsAfsUmAmUmAfUmUmCfAmUmGmAmGfCmUfUmCmGmsUmsAm
F25) AmsAfsUmAmUmAfUmUmCmAmUmGfAmGfCmUfUmCmGmsUmsAm
F26) AmsAfsUmAmUmAfUmUmCmAmUmGfAmGfC(moe)UfUmCmGmsUmsAm
F27) AmsUfsUmUmAmUfUmAmCfAmGmGmUmGfAmGfUmUmGmsAmsUm
F28) AmsUfsUmUmAmUfUmAmCmAmGmGfUmGfAmGfUmUmGmsAmsUm
F29) AmsUfsUmUmAmUfUmAmCmAmGmGfUmGfA(moe)GfUmUmGmsAmsUm
F30) AmsUfsGmUmAmGfUmAmGfAmAmUmUmUfCmUfCmUmGmsUmsAm
F31) AmsUfsGmUmAmGfUmAmGmAmAmUfUmUfCmUfCmUmGmsUmsAm
F32) UmsAfsUmAmGmAfUmGmUfAmGmUmAmGfAmAfUmUmUmsCmsUm

F33) UmsUfsUmGmUmAfUmGmAfAmGmCmAmAfUmUfCmUmCmsCmsUm
F34) UmsUfsUmGmUmAfUmGmAmAmGmCfAmAfUmUfCmUmCmsCmsUm
F35) UmsUfsUmGmUmAfUmGmAfAmGmCmAmAfU(moe)UfCmUmCmsCmsUm
F36) UmsUfsUmGmUmAfUmGmAmAmGmCfAmAfU(moe)UfCmUmCmsCmsUm.

Wherein C represents cytidine-3'-phosphate, G represents guanosine-3'-phosphate, U represents uridine-3'-phosphate, A represents adenosine-3'-phosphate, and T represents thymidine-3'-phosphate, m represents that the nucleotide adjacent to the left side of the letter m is a 2'-O-methyl-modified nucleotide, f represents that the nucleotide adjacent to the left side of the letter f is a 2'-fluoro-modified nucleotide, (moe) represents that the nucleotide adjacent to the left side of the combination sign (moe) is a 2'-O-methoxyethyl-modified nucleotide, and s represents that the two nucleotides adjacent to both sides of the letter s are linked by a phosphorothioate linkage.

In some specific embodiments of the present disclosure, the double-stranded oligonucleotide is one or more sets selected from the group consisting of: set 1, set 2, set 3 and set 4.

| | sense strand (5'-3') | antisense strand(5'-3') |
|---|---|---|
| set 1 | CmsGmsAmAmGmCmUfCfAfUfGmAmAmUmAmUmAmUmUm | AmsAfsUmAmUmAfUmUmCmAmUfGmAmGfC(moe)UfUmCmGmsUmsAm |
| set 2 | CmsGmsAmAmGmCmUfCfAfUfGmAmA(moe)UmAmUmAmUmUm | AmsAfsUmAmUmAfUmUmCmAmUfGmAmGfC(moe)UfUmCmGmsUmsAm |
| set 3 | CmsAmsGmAmGmAmAfAfUfUfCmUmAmCmUmAmCmAmUm | AmsUfsGmUmAmGfUmAmGmAmAfUmUmUfC(moe)UfCmUmGmsUmsAm |
| set 4 | CmsAmsAmCmUmCmAfCfCfUfGmUmAmAmUmAmAmA(moe)Um | AmsUfsUmUmAmUfUmA(moe)CmAmGfGmUmGfAmGfUmUmGmsAmsUm |

In some specific embodiments of the present disclosure, the double-stranded oligonucleotide comprises a set 3.

| set | sense strand (5'-3') | antisense strand (5'-3') |
|---|---|---|
| set 3 | CmsAmsGmAmGmAmAfAfUfUfCmUmAmCmUmAmCmAmUm | AmsUfsGmUmAmGfUmAmGmAmAfUmUmUfC(moe)UfCmUmGmsUmsAm |

In some specific embodiments of the present disclosure, the compound conjugated with an oligonucleotide is selected from one or more of the groups shown in Table 6.

In some specific embodiments of the present disclosure, the compound conjugated with an oligonucleotide is selected from one or more of the following groups.

| | sense strand (5'-3') | antisense strand (5'-3') |
|---|---|---|
| RZ0020 99 | CmsGmsAmAmGmCmUfCfAfUfGmAmAmUmAmUmAmUmUm_(CR01008×3) | AmsAfsUmAmUmAfUmUmCmAmUfGmAmGfC(moe)UfUmCmGmsUmsAm |
| RZ0021 01 | CmsGmsAmAmGmCmUfCfAfUfGmAmA(moe)UmAmUmAmUmUm_(CR01008×3) | AmsAfsUmAmUmAfUmUmCmAmUfGmAmGfC(moe)UfUmCmGmsUmsAm |
| RZ0021 06 | CmsAmsGmAmGmAmAfAfUfUfCmUmAmCmUmAmCmAmUm_(CR01008×3) | AmsUfsGmUmAmGfUmAmGmAmAfUmUmUfC(moe)UfCmUmGmsUmsAm |
| RZ0021 13 | CmsAmsAmCmUmCmAfCfCfUfGmUmAmAmUmAmAmA(moe)Um_(CR01008×3) | AmsUfsUmUmAmUfUmA(moe)CmAmGfGmUmGfAmGfUmUmGmsAmsUm |

Exemplarily, "_(CR01008×3)" indicates that the ligand represented by (CR01008×3) is conjugated to the 3' end of the sense strand.

In some specific embodiments of the present disclosure, the compound conjugated with an oligonucleotide comprises the following group:

| group | sense strand(5'-3') | antisense strand(5'-3') |
|---|---|---|
| RZ002106 | CmsAmsGmAmGmAmAfAfUfUfCmUmAmCmUmAmCmAmUm_(CR01008×3) | AmsUfsGmUmAmGfUmAmGmAmAfUmUmUfC(moe)UfCmUmGmsUmsAm |

Pharmaceutical composition

In a second aspect of the present disclosure, provided is a pharmaceutical composition comprising the compound conjugated with an oligonucleotide according to the first aspect of the present disclosure.

In some optional embodiments of the present disclosure, the pharmaceutical composition can further comprises one or more pharmaceutically acceptable excipients.

The pharmaceutical composition of the present disclosure includes formulations suitable for parenteral administration. The formulations may be conveniently presented in unit dose forms and may be prepared by any method known in the field of pharmacy. The amount of active ingredient that can be combined with an excipient substance to prepare a single dose form is generally the amount of siRNA that produces a therapeutic effect.

Use

In a third aspect of the present disclosure, provided is use of the compound conjugated with an oligonucleotide according to the first aspect of the present disclosure or the pharmaceutical composition according to the second aspect of the present disclosure in the manufacture of a medicament for alleviating, preventing and/or treating a C3 gene-mediated disease or condition.

In some optional embodiments of the present disclosure, the C3 gene-mediated disease or condition includes IgA nephropathy, atypical haemolytic uremic syndrome (aHUS), including paroxysmal nocturnal haemoglobinuria (PNH),

C3 glomerulopathy, lupus nephitis and/or membranous nephritis.

Kit

In a fourth aspect of the present disclosure, provided is a kit comprising the compound conjugated with an oligonucleotide according to the first aspect of the present disclosure or the pharmaceutical composition according to the second aspect of the present disclosure.

Method for inhibiting C3 gene expression

In a fifth aspect of the present disclosure, provided is a method for inhibiting C3 gene expression in a subject in need thereof, wherein the method comprises administering to the subject the compound conjugated with an oligonucleotide according to the first aspect of the present disclosure or the pharmaceutical composition according to the second aspect of the present disclosure.

In some specific embodiments of the present disclosure, a method for inhibiting C3 expression in a cell *in vitro* comprises contacting the cell with the compound conjugated with an oligonucleotide according to the first aspect of the present disclosure or the pharmaceutical composition according to the second aspect of the present disclosure.

In some optional embodiments of the present disclosure, the inhibiting the expression of C3 gene in a cell is performed for a period of time that is sufficient to degrade the mRNA transcript of the C3 gene.

Method for treating a disease

In a sixth aspect of the present disclosure, provided is a method for alleviating, treating and/or preventing a C3-mediated disease or condition in a subject in need thereof, wherein the method comprises administering to the subject the compound conjugated with an oligonucleotide according to the first aspect of the present disclosure or the pharmaceutical composition according to the second aspect of the present disclosure.

In some specific embodiments of the present disclosure, the subject is a human.

In some specific embodiments of the present disclosure, the administration includes subcutaneous administration or intravenous administration.

In some specific embodiments of the present disclosure, the C3 gene-mediated disease or condition includes a disease related to mRNA expression level of C3 gene.

In some specific embodiments of the present disclosure, the C3 gene-mediated disease or condition includes IgA nephropathy, atypical haemolytic uremic syndrome, paroxysmal nocturnal haemoglobinuria (PNH), C3 glomerulopathy, lupus nephitis and membranous nephritis.

The compounds conjugated with oligonucleotides and the pharmaceutical compositions thereof provided by the present disclosure can significantly inhibit expression of C3 mRNA in HepG2 cells, can significantly inhibit C3 mRNA expression in animal liver tissue and C3 protein level in serum, can significantly reduce the C3 protein level in serum of cynomolgus monkeys, can significantly inhibit the activity of complement alternative pathway in serum of cynomolgus monkeys but have no effect on the complement classical pathway in serum, can significantly reduce the urine uTP and UPCR levels and increase urine eGFR level in cynomolgus monkeys with nephropathy, can reduce C3 mRNA and protein levels in CFA-IgA mice, and can reduce C3 deposition in renal tissues and ameliorate the C3 deposition course in renal tissues. The no-observed-adverse-effect level (NOAEL) in SD rats and cynomolgus monkeys is 300 mg/kg. The compounds conjugated with oligonucleotides and the pharmaceutical compositions thereof provided by the present disclosure are useful for alleviating, preventing and/or treating a disease or condition mediated by C3 gene expression dysregulation.

To make the purposes, technical solutions, and advantages of the present disclosure clearer, embodiments of the present disclosure are described in detail below in conjunction with examples.

Unless otherwise indicated, the reagents used in the production of the compounds of the present disclosure were purchased from Beijing Ouhe Technology Co., LTD., wherein the information of the main reagents is shown in Table 1.

Table 1 Main reagents

| Reagent | Abbreviation | CAS number |
|---|---|---|
| Lithium aluminum hydride (LiAlH4) | - | 16853-85-3 |
| Wet palladium carbon (10 mass% loading) | Pd/C | - |
| Palladium carbon hydroxide (10 mass% loading) | Pd(OH)2/C | - |
| Benzotriazole-N,N,N',N'- tetramethylurea hexafluorophosphate | HBTU | 94790-37-1 |

(continued)

| Reagent | Abbreviation | CAS number |
|---|---|---|
| 2-(7-azobenzotriazole)-N,N,N',N' - | HATU | 148893-10-1 |
| tetramethylurea hexafluorophosphate | | |
| 4,4'-Bismethoxy triphenylmethyl chloride/4,4'-dimethoxy triphenyl chloromethane | DMTrCl | 40615-36-9 |
| Bis(diisopropylamino) (2-cyanoethoxy) phosphine | - | 102691-36-1 |
| 4,5-Dicyanoimidazole | DCI | 1122-28-7 |
| 4-Dimethylaminopyridine | DMAP | 1122-58-3 |
| Aminoalkyl-CPG, Model: C3006-1000 | 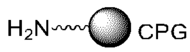 | - |
| 4M hydrochloric acid in 1,4-dioxane solution | - | - |
| Trans-4-(Boc-amino)cyclohexyl formaldehyde | - | 181308-57-6 |
| N-benzyloxycarbonyl-4-aminobutyric acid | - | 5105-78-2 |
| 5-[[(2R,3R,4R,5R,6R)-3-acetamido -4,5-diacetoxy -6-(acetoxymethyl)-2-tetrahydropyranyl]oxy] valeric acid | Compound 4 | 1159408-54-4 |

Wherein, CPG represents controlled pore glass (CPG) support.

Unless otherwise indicated, the reagents, consumables and instruments used in the biological assay of the present disclosure are commercially available. Among them, the main reagents and consumables are detailed in Table 2, and the main instruments are detailed in Table 3.

Table 2 Main reagents and consumables

| Reagent | Manufacturer |
|---|---|
| Opti-MEM | GENOM Bio |
| MEM Medium | Gibco |
| FBS | Gibco |
| Penicilin-streptomycin | HyClone |
| Trypsin | Gibco |
| Power SYBR Green RNA-to-CT™ 1-Step | Thermo Fisher |
| 1×PBS | M&C GENE TECHNOLOGY(BEIJING) LTD. |
| DMEM Medium | M&C GENE TECHNOLOGY(BEIJING) LTD. |
| Opti-MEM™ Medium | Gibco |
| Serum | Sigma |
| Trypsin | M&C GENE TECHNOLOGY(BEIJING) LTD. |
| Double antibiotics | BBI |
| Lipofectamine RNAiMax | Invitrogen |
| Nucleic acid extraction or purification kit | Zhejiang Hanwei Technology Co., Ltd. |
| RevertAid First Strand cDNA Synthesis Kit | Thermo Fisher Scientific |
| TaqMan Fast Advanced Master Mix | Thermo Fisher Scientific |
| SYBR Select Master Mix | Thermo Fisher Scientific |
| RNA Extraction Kit (Hanwei) | Zhejiang Hanwei Technology Co., Ltd. |
| RNALater | Thermo Fisher Scientific |
| Ambion® RNAlater® | Invitrogen |

(continued)

| Reagent | Manufacturer |
|---|---|
| human C3a ELISA kit | Hycult Biotech |
| WIESLAB® Complement System Alternative Pathway - RUO | IBL America |
| WIESLAB® Complement System Classical Pathway- RUO | IBL America |

Table 3 Main instruments

| Instrument | Manufacturer |
|---|---|
| Fully automatic nucleic acid extraction equipment | Zhejiang Hanwei Technology Co., Ltd. |
| High-speed freezing centrifuge | Eppendorf |
| Carbon dioxide incubator | Thermo Fisher Scientific |
| Biological safety cabinet | Shanghai Lishen |
| Constant temperature water bath pot | Shanghai Boxun |
| Automatic cell counter | Shanghai Countstar |
| Inverted microscope | Olympus |
| NANODROP OneC | Thermo Fisher Scientific |
| Gradient PCR Amplifier | Eppendorf |
| CFX Opus 384 | Bio-Rad |
| LightCycler 480 | Roche |
| Gel imager | Shanghai Tanon Science & Technology Co.,Ltd. |
| Electrophoresis equipment | Beijing Liuyi Biotechnology Co., Ltd. |
| Automatic Tissue Homogenizer-Tissuelyser II | Shanghai Jingxin Industrial Development Co., Ltd |
| Real-time fluorescence quantitative PCR instrument | Roche |
| Paraffin slicer | Jinhua YIDI |
| Tissue Dehydrator | Leica |
| Fully automatic biological tissue embedding machine | Jinhua YIDI |
| Automatic hematology analyzer | SYSMEX |

The reagent ratios described in the present disclosure are calculated as volume-to-volume (v/v), unless otherwise noted.

Preparation of compounds

Preparative example 1: Synthesis of compounds CR01008 and CR01008Z

(1.1) Synthesis of compound CR01008

In this preparative example, the synthetic route of compound CR01008 is shown below.

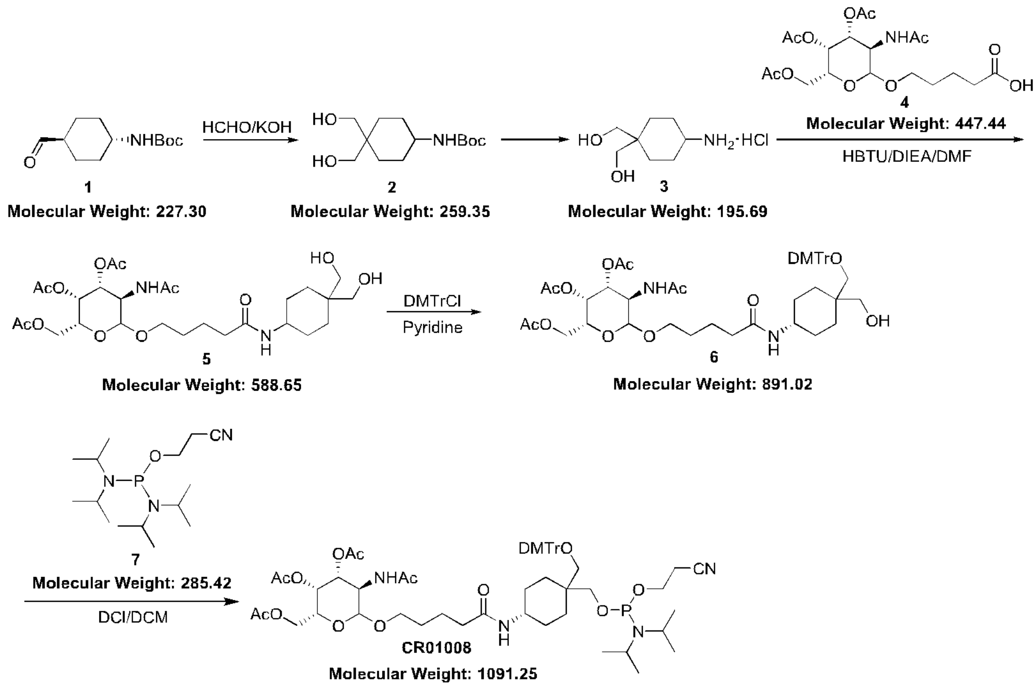


(1.1.1) Synthesis of compound 2

Compound 1 (trans-4-(Boc-amino)cyclohexylformaldehyde, 10.0 g, 1.0 eq) and aqueous formaldehyde solution (8.9 g, 37 mass%, 2.4 eq) were dissolved in 33 ml of methanol, and 13 ml of an aqueous KOH solution at a concentration of 45.3 mass% was added dropwise. After the dropwise addition was completed, the reaction system was stirred for 30 min at 25°C, heated up to 60°C, and refluxed at 60°C for 2 h of reaction. After the reaction was completed, the reaction solution was cooled to room temperature and evaporated to dryness under reduced pressure to obtain a crude product as white solid. The crude product was slurried with a small amount of water, and then filtered to obtain compound 2 as a white solid (9 g, 78.9% yield). MS-ESI *(m/z)* = 260 $[M + H]^+$.

(1.1.2) Synthesis of compound 3

The compound 2 (9 g, 1 eq) prepared according to step (1.1.1) was dissolved in 70 ml of 1,4-dioxane, added with a solution of hydrogen chloride in 1,4-dioxane (45 ml, 4 M) and stirred at 25°C for 1 h of reaction. After the reaction was completed, the reaction solution was evaporated to dryness under reduced pressure to obtain compound 3 as a white solid (6.8 g, 100% yield).

(1.1.3) Synthesis of compound 5

The compound 3 (1.8 g, 2.0 eq) prepared according to step (1.1.2), compound 4 (5-[[(2R,3R,4R,5R,6R)-3-acetamido-4,5-diacetoxy-6-(acetyloxymethyl)-2-tetrahydropyranyl]oxy ]pentanoic acid, 2.1 g, 1.0 eq), and DIEA (N,N-diisopropylethylamine, 3.5 g, 6.0 eq) were dissolved in 15 ml of DMF, added with HBTU (1.9 g, 1.1 eq), and stirred at 25°C under $N_2$ atmosphere for 3 h of reaction. After the reaction was completed, the reaction solution was evaporated to dryness under reduced pressure and purified by reverse phase chromatography (22 vol% aqueous solution of acetonitrile) to obtain compound 5 as a white solid (1.78 g, 64.4% yield). MS-ESI *(m/z)* = 589$[M + H]^+$.

(1.1.4) Synthesis of compound 6

The compound 5 (1.54 g, 1.0 eq) prepared according to step (1.1.3) was dissolved in 15 ml of pyridine, the reaction system was cooled down to 0°C in an ice-water bath and DMTrCl (4,4'-dimethoxytriphenylmethyl chloride, 1.32 g, 1.5 eq) was added at 0°C to perform 3 h of reaction at 25°C. The reaction was quenched by adding 15 ml of methanol to the reaction solution. After the reaction was completed, the reaction solution was evaporated to dryness under reduced pressure and purified by reverse phase chromatography (60 vol% aqueous solution of acetonitrile) to obtain compound 6 as a yellow solid (1 g, 42.7% yield). MS-ESI (m/z) = 891 $[M + H]^+$.

(1.1.5) Synthesis of compound CR01008

The compound 6 (1.08 g, 1.0 eq) prepared according to step (1.1.4) was dissolved in 20 ml of anhydrous dichloromethane, DCI (115 mg, 0.8 eq) and compound 7 (bis(diisopropylamino)(2-cyanoethoxy)phosphine, 732 mg, 2.1 eq) were added separately, nitrogen replacement was performed three times, and the reaction system was stirred at 25°C for 2 h of reaction. After the reaction was completed, the reaction solution was added with 20 ml of saturated sodium bicarbonate aqueous solution and extracted with 20 ml of dichloromethane 3 times (3×20 ml). The organic phases were combined, evaporated to dryness under reduced pressure, purified by reverse phase chromatography (72 vol% aqueous solution of acetonitrile) and then dried under vacuum for 12 h to obtain the compound CR01008 as a white powder (1 g, 76.0% yield). MS-ESI (m/z) = 1091 [M + Na]$^+$.

1H NMR (400 MHz, DMSO-d6) δ 1.05 (d, J = 6.7 Hz, 6H).1.14 (d, J = 6.7 Hz, 6H), 1.37 - 1.17 (m, 5H), 1.60 - 1.40 (m, 6H),1.68 - 1.62 (m, 1H),1.80 (s, 3H),1.80 (s, 3H),1.92 (s, 3H), 2.02 (s, 5H),2.13 (s, 3H),2.71 (t, J = 5.9 Hz, 2H), 2.79 (d, J = 8.4 Hz, 1H), 2.87 (d, J = 8.4 Hz, 1H),3.36 (s, 1H), 3.58 - 3.39 (m, 3H), 3.69 - 3.60 (m, 2H), 3.75 (s, 7H), 3.90 (dt, J = 11.2, 8.8 Hz, 1H), 4.05 (s, 3H),4.51 (d, J = 8.4 Hz, 1H), 4.99 (dd, J = 11.3, 3.4 Hz, 1H), 5.24 (d, J = 3.4 Hz, 1H), 5.78 (s, 1H), 6.93 - 6.87 (m, 4H),7.35 - 7.21 (m, 7H), 7.44 - 7.37 (m, 2H), 7.66 (d, J = 7.8 Hz, 1H), 7.84 (d, J = 9.2 Hz, 1H).

(1.2) Synthesis of compound CR01008Z

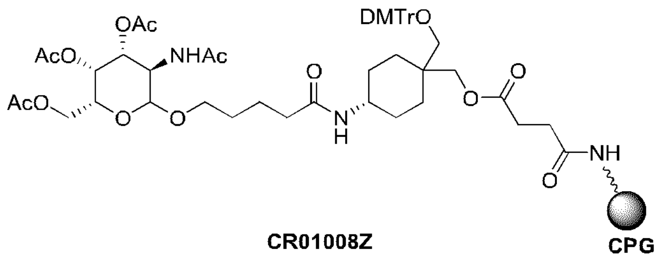

CR01008Z

In this example, the synthetic route of compound CR01008Z is shown below.

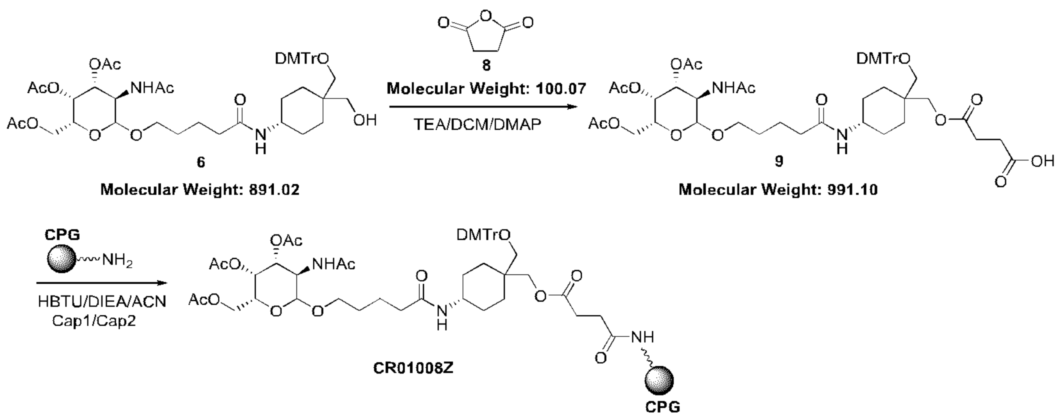


(1.2.1) Synthesis of compound 9

The compound 6 prepared according to step (1.1.4) (500 mg) was dissolved in 10 ml of dichloromethane, compound 8 (succinic anhydride, 112 mg), DMAP (6.8 mg) and TEA (226.2 mg) were added, nitrogen replacement was performed three times, and the reaction system was stirred at 25°C for 16 h of reaction. The reaction solution was purified by flash chromatography to obtain compound 9 (300 mg, 53.6% yield). MS-ESI *(m/z)* = 1013 [M + Na]$^+$.

(1.2.2) Synthesis of compound CR01008Z

The compound 9 (50 mg) prepared according to step (1.2.1), aminoalkyl CPG (1.25 g, 80 μmol/g, 0.1 mmol), HBTU (27 mg), and DIEA (12 mg) were added to a 20 ml vial, and the reaction was carried out on a shaker for 16 h. After the reaction was completed, the reaction solution was filtered to obtain a filter cake. The filter cake was washed once with 10 ml

of acetonitrile (1×10 ml) and then dried under vacuum. The dried filter cake, DMAP (3 mg), Cap1 (10 ml, 200 V) and Cap2 (1 ml, 20 V) were added to a 20 ml vial, and the reaction was carried out on a shaker for 6 h. After the reaction was completed, the reaction solution was filtered to obtain a filter cake. The filter cake was washed once with 10 ml of acetonitrile (1×10 ml) and then dried under vacuum to obtain the compound CR01008Z (1.03 g, loading amount: 20-30 μmol/g).

Wherein, Cap1 and Cap2 were capping agents, Cap1 was a solution of 20 vol% N-methylimidazole in a pyridine/acetonitrile mixture, the volume ratio of pyridine to acetonitrile was 3:5, and Cap2 was a solution of 20 vol% acetic anhydride in acetonitrile.

Preparative example 2: Preparation of compounds CR01013 and CR01013Z

(2.1) Preparation of compound CR01013

In this example, the synthetic route of compound CR01013 is shown below.

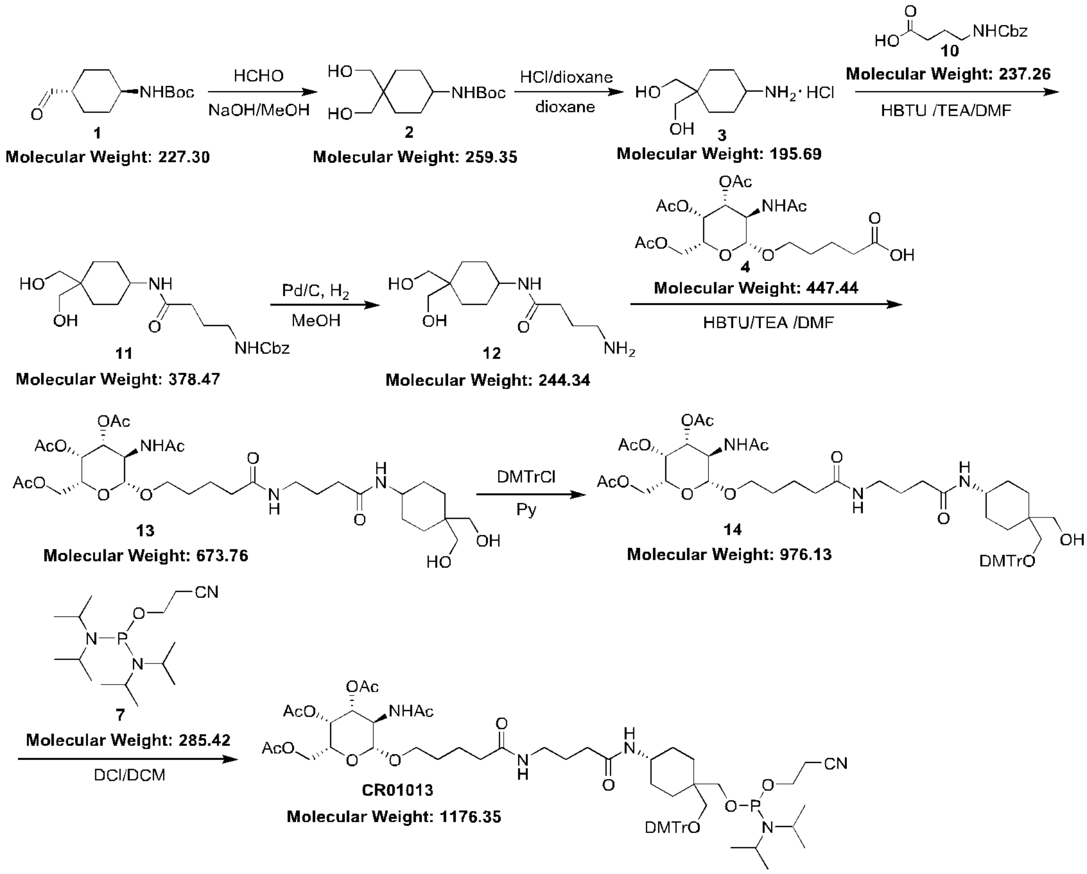


(2.1.1) Synthesis of compound 2

Compound 1 (trans-4-(Boc-amino)cyclohexylcarboxaldehyde, 4.9 g) was dissolved in 17 ml of methanol. An aqueous solution of formaldehyde (4.21 g, at a concentration of 37 mass%) and an aqueous solution of sodium hydroxide (6.5 ml, at a concentration of 45.3 mass%) were added dropwise. After the dropwise addition was completed, the reaction system was heated to 60°C, and stirred at 60°C for 2 h of reaction. After the reaction was completed, the reaction solution was cooled to 25°C and evaporated to dryness under reduced pressure to obtain a crude product as a white solid. The crude product was slurried with a small amount of water, filtered and dried to obtain compound 2 as a white solid (4.8 g, 85.9% yield). ESI-MS *(m/z)* = 260.2$[M+H]^+$.

(2.1.2) Synthesis of compound 3

The compound 2 (4.8 g) prepared according to step (2.1.1) was dissolved in 25 ml of 1,4-dioxane, a solution of hydrochloric acid in 1,4-dioxane (25 ml, 4 M) was added, and the reaction system was stirred and reacted at 25°C for 2 h. After the reaction was completed, the reaction solution was evaporated to dryness under reduced pressure to obtain

compound 3 as a white solid (3.6 g, 99.4% yield).

(2.1.3) Synthesis of compound 11

The compound 3 (3.6 g) prepared according to step (2.1.2) was dissolved in 36 ml of DMF, and then TEA (5.62 g), compound 10 (N-benzyloxycarbonyl-4-aminobutyric acid, 5.28 g), and HBTU (8.43 g) were added, and the reaction system was stirred and reacted at 25°C for 16 h. After the reaction was completed, 200 ml of saturated aqueous sodium bicarbonate solution was added. The reaction solution was extracted with 100 ml of ethyl acetate three times (3×100 ml), the organic phases were combined, washed with 50 ml of saturated aqueous solution of sodium chloride (1×50 ml), dried over anhydrous sodium sulfate, evaporated to dryness under reduced pressure, and then purified by normal-phase chromatography (eluent: dichloromethane/ methanol=10/1, v/v) to obtain compound 11 as a white solid (2.3 g, 33.0% yield). ESI MS (m/z) = 379.5$[M+H]^+$.

(2.1.4) Synthesis of compound 12

The compound 11 (2.3 g) prepared in step (2.1.3) was dissolved in 23 ml of methanol, wet palladium carbon (230 mg, 10 mass% loading) was added, hydrogen replacement was performed three times, and the reaction system was stirred and reacted at 25°C for 16 h under a hydrogen atmosphere (15 psi). After the reaction was completed, the reaction solution was filtered to obtain a filtrate, and the filtrate was evaporated to dryness under reduced pressure to obtain compound 12 as a yellow oil (1.48 g, 99.8% yield).

(2.1.5) Synthesis of compound 13

The compound 12 (1.48 g) prepared in step (2.1.4) was dissolved in 15 ml of DMF, triethylamine (TEA, 1.22 g), compound 4 (1.35 g), and HBTU (3.45 g) were added, and the reaction system was stirred and reacted at 25°C for 16 h. After the reaction was completed, 150 ml of saturated aqueous solution of sodium bicarbonate was added, the reaction solution was extracted with 50 ml of ethyl acetate for three times (3×50 ml). The organic phases were combined, washed with 30 ml of saturated aqueous solution of sodium chloride (1×30 ml), dried over anhydrous sodium sulfate, evaporated to dryness under reduced pressure, and then purified by reverse phase chromatography (C18 column, eluent: water/-acetonitrile=5/1, v/v) to obtain compound 13 as a white solid (1.3 g, 31.8% yield). ESI-MS (m/z): 674.3 $[M+H]^+$.

(2.1.6) Synthesis of compound 14

The compound 13 (1.1 g) prepared in step (2.1.5) was dissolved in 11 ml of pyridine, the reaction system was cooled down to 0°C in an ice-water bath, and DMTrCl (813 mg) was added in batches at 0°C. The reaction system was stirred and reacted at 0°C for 1 h. After the reaction was completed, the reaction solution was added with methanol to quench the reaction, evaporated to remove solvent, and purified by reverse-phase chromatography (eluent: water/-acetonitrile=1/4, v/v) to obtain compound 14 as a white solid (800 mg, 50.3% yield). ESI-MS (m/z):976.5$[M+H]^+$.

(2.1.7) Synthesis of compound CR01013

At 25°C, the compound 14 (550 mg) prepared in step (2.1.6) was dissolved in 5 ml of dichloromethane (DCM), and 4,5-dicyanoimidazole (DCI, 53.2 mg) and compound 7 (2-cyanoethyl N,N,N',N'-tetraisopropyl phosphordiamidite, 255.4 mg) were added. Nitrogen replacement was performed three times, and the reaction system was stirred at 25°C in nitrogen atmosphere for 1 h. After the reaction was completed, the reaction solution was washed twice with 5 ml of saturated aqueous sodium bicarbonate solution (2×5 ml) and then once with 30 ml of saturated aqueous sodium chloride solution (1×30 ml). The organic phase was separated, dried over anhydrous sodium sulfate, evaporated under reduced pressure to remove solvent, and purified by normal-phase chromatography (eluent: dichloromethane/methanol=20/1, v/v) to obtain compound CR01013 as a white solid (532 mg, 80.4% yield). ESI-MS (m/z): 1176. 7$[M+H]^+$.

1H NMR (400 MHz, DMSO-d6) δ 0.95 - 1.05 (d, J = 6.7 Hz, 5H), 1.06 - 1.15 (q, J = 7.6 Hz, 8H), 1.15 1.21 (t, J = 7.2 Hz, 14H), 1.72 - 1.80 (s, 3H), 1.84 - 1.92 (s, 3H), 1.94 - 2.07 (d, J = 16.0 Hz, 7H), 2.07 - 2.14 (s, 3H), 2.64 - 2.72 (q, J = 5.8 Hz, 2H), 2.74 - 2.89 (d, J = 8.5 Hz, 2H), 3.35 - 3.56 (m, 4H), 3.57 - 3.70 (m, 4H), 3.71 - 3.77 (s, 6H), 3.81 - 3.93 (m, 1H), 3.96 - 4.09 (d, J = 6.4 Hz, 3H), 6.82 - 6.97 (d, J = 8.7 Hz, 4H), 7.17 - 7.27 (t, J = 8.7 Hz, 5H), 7.27 - 7.34 (t, J = 7.6 Hz, 2H), 7.34 - 7.43 (d, J = 7.5 Hz, 2H).

(2.2) Synthesis of compound CR01013Z

In this example, the synthetic route of compound CR01013Z is shown below.

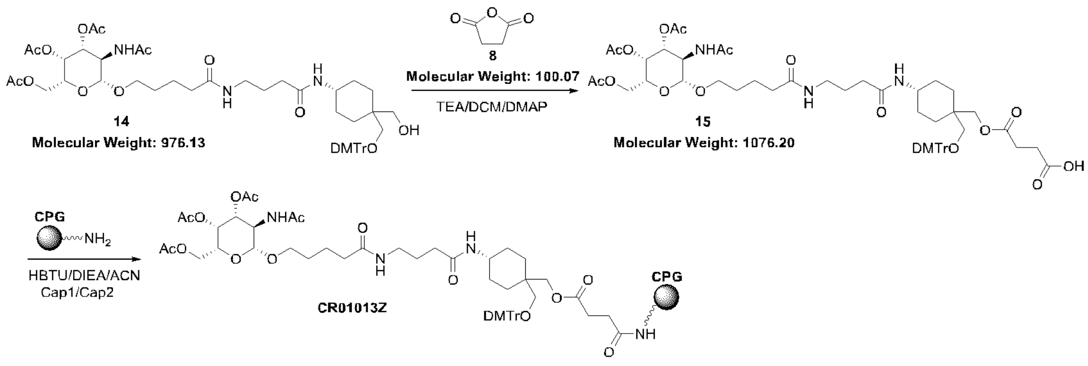


(2.2.1) Synthesis of compound 15

At 25°C, the compound 14 (100 mg, 0.10 mmol) prepared in step (2.1.6) was dissolved in 2 ml of dichloromethane, and triethylamine (25.9 mg, 0.25 mmol), DMAP (1.25 mg, 0.01 mmol) and compound 8 (succinic anhydride, 15.4 mg, 0.15 mmol) were added. The reaction system was stirred at 25°C for 16 h. After the reaction was completed, the reaction solution was evaporated to remove solvent and purified by reverse-phase chromatography (C18 column, eluent: water/acetonitrile = 2/1, v/v) to obtain compound 15 as a yellow oil (110 mg, 0.10 mmol, 100% yield). ESI-MS (m/z) = 1099.3 $[M+Na]^+$.

(2.2.2) Synthesis of compound CR01013Z

The compound 15 (50 mg, 0.04 mmol) prepared in step (2.2.1) was dissolved in 10 ml of acetonitrile, HBTU (24.2 mg, 0.06 mmol), DIEA (11.0 mg, 0.08 mmol) and aminoalkyl-CPG (1.06 g, loading amount: 80 μmol/g) were added, and the reaction system was stirred at 25°C for 16 h of reaction. After the reaction was completed, the reaction solution was filtered to obtain a filter cake, and the filter cake was washed twice with 50 ml of dichloromethane (2×50 ml), three times with 50 ml of acetonitrile (3×50 ml), and once with 50 ml of ethyl acetate (1×50 ml) in sequence, and then dried under vacuum. Cap1 (4.8 ml), Cap2 (0.54 ml) and DMAP (2.59 mg) were added to the dried filter cake, and the reaction system was stirred and reacted at 25°C for 5 h. After the reaction was completed, the reaction solution was filtered to obtain a filter cake. The filter cake was washed three times with 50 ml of acetonitrile (3×50 ml) and dried under vacuum to obtain compound CR01013Z (900 mg, loading amount: 20-30 μmol/g).

Wherein, Cap1 and Cap2 were capping agents, Cap1 was a solution of 20 vol% N-methylimidazole in a pyridine/acetonitrile mixture, the volume ratio of pyridine to acetonitrile was 3:5, and Cap2 was a solution of 20 vol% acetic anhydride in acetonitrile.

Preparative example 3: Preparation of double-stranded oligonucleotide (SiRNA)

(3.1) Synthesis of sense strand (SS)

According to the solid-phase nucleic acids synthesis using phosphoramidite method, nucleoside monomers were linked one by one in the direction from 3' to 5'. The linking of each nucleoside monomer comprised a four-step reaction of deprotection, coupling, capping, and oxidation or sulfurization. The synthesis condition was given below.

The nucleoside monomers were prepared to a solution of 0.1 M nucleoside monomer in acetonitrile.

The condition for deprotection reaction in each step was identical, including a temperature of 25°C, a reaction time of 70 seconds, a solution of dichloroacetic acid in dichloromethane (3 vol%) as a deprotection agent, and a molar ratio of the dichloroacetic acid to the protecting group 4,4'-dimethoxytrityl on the solid phase support of 5:1.

The condition for coupling reaction in each step was identical, including a temperature of 25°C, a molar ratio of the nucleic acid sequence linked to the solid phase support to the nucleoside monomers of 1:10, a molar ratio of the nucleic acid sequence linked to the solid phase support to a coupling agent of 1:65, a reaction time of 600 seconds, a solution of 0.5 M 5-ethylthio-1H-tetrazole in acetonitrile as a coupling agent, and a solution of 0.2 M xanthane hydride in a acetonitrile/pyridine mixture (volume ratio of acetonitrile: pyridine = 1:1) as a thio agent.

The condition for capping reaction in each step was identical, including a temperature of 25°C, a reaction time of 2 min, a mixed solution of Cap1 and Cap2 in a molar ratio of 1:1 as a capping agent, a solution of 20 vol% N-methylimidazole in a pyridine/acetonitrile mixture as Cap1, a volume ratio of pyridine to acetonitrile of 3:5, a solution of 20 vol% acetic anhydride in acetonitrile as Cap2, and a molar ratio of N-methylimidazole in the capping agent Cap1: acetic anhydride in the capping agent Cap2: the nucleic acid sequence connected to the solid phase support of 1:1:1.

The condition for oxidation reaction in each step was identical. The condition for oxidation reaction included a

temperature of 25°C, a reaction time of 3 seconds, 0.05 M iodine water as an oxidation agent, and a molar ratio of iodine to the nucleic acid sequence connected to the solid phase support in the coupling reaction of 30: 1. The oxidation reaction was carried out in a mixed solvent of water/pyridine (volume ratio of water: pyridine = 1:9). The condition for sulfurization reaction included a temperature of 25°C, a reaction time of 360 seconds, a solution of 0.2 M xanthane hydride in pyridine as a thio agent, and a molar ratio of the thio agent to the nucleic acid sequence connected to the solid phase support in the coupling reaction of 4:1. The sulfurization reaction was carried out in a mixed solvent of water/pyridine (volume ratio of water: pyridine = 1:9).

After the last nucleoside monomer was linked, the nucleic acid sequence connected to the solid phase support was cleaved, deprotected, purified and desalted in turn, and then freeze-dried to obtain the sense strand.

The conditions for cleavage and deprotection were as follows: the synthesized nucleotide sequence connected to the solid phase support was added into a 25 mass% aqueous ammonia solution for 16 h of reaction at 55°C, wherein the aqueous ammonia solution was used in an amount of 0.5 ml/μmol. The solvent was removed, and the residue was concentrated under vacuum to dryness. After the treatment by aqueous ammonia solution was completed, the resulting product was dissolved with 0.4 ml/μmol N-methylpyrrolidone according to the amount of single-stranded nucleic acid, and then added with 0.3 ml/μmol triethylamine and 0.6 ml/μmol triethylamine trihydrofluoride to remove 2'-O-TBDMS protection from the ribose.

The conditions for purification and desalination were as follows: the nucleic acids were purified using a preparative ion chromatography column (Source 15Q) with a gradient elution by NaCl. Specifically, eluent 1 was 20 mM sodium phosphate (pH 8.1) in a mixed solvent of water/acetonitrile (volume ratio of water: acetonitrile = 9: 1); eluent 2 was 1.5 M sodium chloride and 20 mM sodium phosphate (pH 8.1) in a mixed solvent of water/acetonitrile (volume ratio of water: acetonitrile = 9: 1); elution gradient was eluent 1: eluent 2 = (100: 0) to (50: 50). The eluate was collected, combined and desalted by using a reverse phase chromatography purification column. The conditions for desalination included a sephadex column (packing material: Sephadex-G25) for desalination, and deionized water for elution.

Detection: The purity detection was performed using ion exchange chromatography (IEX-HPLC), and the molecular weight was measured by liquid chromatography-mass spectrometry (LC-MS). The measured value of the molecular weight and the theoretical value of the molecular weight were compared. When the measured value was consistent with the theoretical value, it indicated that a sense strand of siRNA was obtained.

(3.2) Synthesis of antisense strand (AS)

Antisense strands were synthesized using a general solid phase support. The reaction conditions of deprotection, coupling, capping, oxidation or sulfurization, cleavage and deprotection, and purification and desalination in the solid-phase synthesis method of antisense strand were the same as those used for the synthesis of the sense strand in step (3.1).

Detection: The purity detection was performed using ion exchange chromatography (IEX-HPLC), and the molecular weight was measured by liquid chromatography-mass spectrometry (LC-MS). The measured value of the molecular weight and the theoretical value of the molecular weight were compared. When the measured value was consistent with the theoretical value, it indicated that an antisense strand of siRNA was obtained.

(3) Synthesis of double-stranded siRNA

The sense strand synthesized in step (3.1) and the antisense strand synthesized in step (3.2) were mixed at an equimolar ratio, dissolved in water for injection, heated to 95°C, slowly cooled to room temperature and left to stand at room temperature for 10 min to allow the sense and antisense strands to form a double-stranded structures by hydrogen bonds, thereby obtaining the siRNA shown in Table 1.

Unmodified double-stranded oligonucleotides (siRNAs) prepared according to the methods provided in the present disclosure are shown in Table 4.

Table 4 Unmodified siRNA

| Number of siRNA | Sense strand (5'-3') | SEQ ID | Antisense strand (5'-3') | SEQ ID |
|---|---|---|---|---|
| 1 | CAGAGAAAUUCUACU ACAU | No.1 | AUGUAGUAGAAUUUCUC UGUA | No.2 |
| 2 | AAAUUCUACUACAUC UAUA | No.3 | UAUAGAUGUAGUAGAA UUUCU | No.4 |

(continued)

| Number of siRNA | Sense strand (5'-3') | SEQ ID | Antisense strand (5'-3') | SEQ ID |
|---|---|---|---|---|
| 3 | CGAAGCUCAUGAAUA UAUU | No.5 | AAUAUAUUCAUGAGCUU CGUA | No.6 |
| 4 | CAACUCACCUGUAAU AAAU | No.7 | AUUUAUUACAGGUGAGU UGAU | No.8 |
| 5 | GAGAAUUGCUUCAUA CAAA | No.9 | UUUGUAUGAAGCAAUUC UCCU | No.10 |

The sequence listing software requires "U" to be represented by "T" when editing RNA sequences, so the sequence list does not correctly characterize the siRNA sequences of the present disclosure, and all sequences are subjected to the specification.

The modified double-stranded oligonucleotides (siRNAs) shown in Table 5 were prepared by methods provided by the present disclosure.

Table 5 Modified siRNA sequences

| Group | Sense strand (5'-3') | Antisense strand (5'-3') |
|---|---|---|
| 1 | CmsGmsAmAmGmCmUfCfAfUfGmA mAmUmAmUmAmUmUm | AmsAfsUmAmUmAfUmUmCfAmUmGmAm GfC(moe)UfUmCmGmsUmsAm |
| 2 | CmsGmsAmAmGmCmUfCfAfUfGmA mAmUmAmUmAmUmUm | AmsAfsUmAmUmAfUmUmCmAfUmGmAm GfC(moe)UfUmCmGmsUmsAm |
| 3 | CmsGmsAmAmG(moe)CmUfCfAfUfG mAmAmUmAmUmAmUmUm | AmsAfsUmAmUmAfUmUmCmAfUmGmAm GfC(moe)UfUmCmGmsUmsAm |
| 4 | CmsGmsAmAmGmCmUfCfAfUfGmA( moe)AmUmAmUmAmUmUm | AmsAfsUmAmUmAfUmUmCmAfUmGmAm GfC(moe)UfUmCmGmsUmsAm |
| 5 | CmsGmsAmAmGmCmUfCfAfUfGmA mA(moe)UmAmUmAmUmUm | AmsAfsUmAmUmAfUmUmCmAfUmGmAm GfC(moe)UfUmCmGmsUmsAm |
| 6 | CmsGmsAmAmGmCmUfCfAfUfGmA mAmUmAmUmAmT(moe)Um | AmsAfsUmAmUmAfUmUmCmAfUmGmAm GfC(moe)UfUmCmGmsUmsAm |
| 7 | CmsGmsAmAmG(moe)CmUfCfAfUfG mAmAmUmAmUmAmUmUm | AmsAfsUmAmUmAfUmT(moe)CmAfUmGm AmGfCmUfUmCmGmsUmsAm |
| 8 | CmsGmsAmAmGmCmUfCfAfUfGmA( moe)AmUmAmUmAmUmUm | AmsAfsUmAmUmAfUmT(moe)CmAfUmGm AmGfCmUfUmCmGmsUmsAm |
| 9 | CmsGmsAmAmGmCmUfCfAfUfGmA mA(moe)UmAmUmAmUmUm | AmsAfsUmAmUmAfUmT(moe)CmAfUmGm AmGfCmUfUmCmGmsUmsAm |
| 10 | CmsGmsAmAmGmCmUfCfAfUfGmA mAmUmAmUmAmT(moe)Um | AmsAfsUmAmUmAfUmT(moe)CmAfUmGm AmGfCmUfUmCmGmsUmsAm |
| 11 | CmsAmsGmAmGmAmAfAfUfUfCmU mAmCmUmAmCmAmUm | AmsUfsGmUmAmGfUmAmGfAmAmUmUm UfC(moe)UfCmUmGmsUmsAm |
| 12 | CmsAmsGmAmGmAmAfAfUfUfCmU mAmCmUmAmCmAmUm | AmsUfsGmUmAmGfUmAmGmAfAmUmUm UfC(moe)UfCmUmGmsUmsAm |

(continued)

| Group | Sense strand (5'-3') | Antisense strand (5'-3') |
|---|---|---|
| 13 | CmsAmsGmAmG(moe)AmAfAfUfUfCmUmAmCmUmAmCmAmUm | AmsUfsGmUmAmGfUmAmGmAfAmUmUmUfC(moe)UfCmUmGmsUmsAm |
| 14 | CmsAmsGmAmGmAmAfAfUfUfCmT(moe)AmCmUmAmCmAmUm | AmsUfsGmUmAmGfUmAmGmAfAmUmUmUfC(moe)UfCmUmGmsUmsAm |
| 15 | CmsAmsGmAmGmAmAfAfUfUfCmUmA(moe)CmUmAmCmAmUm | AmsUfsGmUmAmGfUmAmGmAfAmUmUmUfC(moe)UfCmUmGmsUmsAm |
| 16 | CmsAmsGmAmGmAmAfAfUfUfCmUmAmCmUmAmCmA(moe)Um | AmsUfsGmUmAmGfUmAmGmAfAmUmUmUfC(moe)UfCmUmGmsUmsAm |
| 17 | CmsAmsGmAmG(moe)AmAfAfUfUfCmUmAmCmUmAmCmAmUm | AmsUfsGmUmAmGfUmA(moe)GmAfAmUmUmUfCmUfCmUmGmsUmsAm |
| 18 | CmsAmsGmAmGmAmAfAfUfUfCmT(moe)AmCmUmAmCmAmUm | AmsUfsGmUmAmGfUmA(moe)GmAfAmUmUmUfCmUfCmUmGmsUmsAm |
| 19 | CmsAmsGmAmGmAmAfAfUfUfCmUmA(moe)CmUmAmCmAmUm | AmsUfsGmUmAmGfUmA(moe)GmAfAmUmUmUfCmUfCmUmGmsUmsAm |
| 20 | CmsAmsGmAmGmAmAfAfUfUfCmUmAmCmUmAmCmA(moe)Um | AmsUfsGmUmAmGfUmA(moe)GmAfAmUmUmUfCmUfCmUmGmsUmsAm |
| 21 | GmsAmsGmAmAmUmUfGfCfUfUmCmAmUmAmCmAmAmAm | UmsUfsUmGmUmAfUmGmAfAmGmCmAmAfT(moe)UfCmUmCmsCmsUm |
| 22 | GmsAmsGmAmAmUmUfGfCfUfUmCmAmUmAmCmAmAmAm | UmsUfsUmGmUmAfUmGmAmAfGmCmAmAfT(moe)UfCmUmCmsCmsUm |
| 23 | GmsAmsGmAmAmUmUfGfCfUfUmCmAmUmAmCmAmAmAm | UmsUfsUmGmUmAfUmGmAmAmGfCmAmAfT(moe)UfCmUmCmsCmsUm |
| 24 | GmsAmsGmAmA(moe)UmUfGfCfUfUmCmAmUmAmCmAmAmAm | UmsUfsUmGmUmAfUmGmAmAfGmCmAmAfT(moe)UfCmUmCmsCmsUm |
| 25 | GmsAmsGmAmAmUmUfGfCfUfUmC(moe)AmUmAmCmAmAmAm | UmsUfsUmGmUmAfUmGmAmAfGmCmAmAfT(moe)UfCmUmCmsCmsUm |
| 26 | GmsAmsGmAmAmUmUfGfCfUfUmCmA(moe)UmAmCmAmAmAm | UmsUfsUmGmUmAfUmGmAmAfGmCmAmAfT(moe)UfCmUmCmsCmsUm |
| 27 | GmsAmsGmAmAmUmUfGfCfUfUmCmAmUmAmCmAmA(moe)Am | UmsUfsUmGmUmAfUmGmAmAfGmCmAmAfT(moe)UfCmUmCmsCmsUm |
| 28 | GmsAmsGmAmA(moe)UmUfGfCfUfUmCmAmUmAmCmAmAmAm | UmsUfsUmGmUmAfUmG(moe)AmAfGmCmAmAfUmUfCmUmCmsCmsUm |
| 29 | GmsAmsGmAmAmUmUfGfCfUfUmC(moe)AmUmAmCmAmAmAm | UmsUfsUmGmUmAfUmG(moe)AmAfGmCmAmAfUmUfCmUmCmsCmsUm |
| 30 | GmsAmsGmAmAmUmUfGfCfUfUmCmA(moe)UmAmCmAmAmAm | UmsUfsUmGmUmAfUmG(moe)AmAfGmCmAmAfUmUfCmUmCmsCmsUm |

(continued)

| Group | Sense strand (5'-3') | Antisense strand (5'-3') |
|---|---|---|
| 31 | GmsAmsGmAmAmUmUfGfCfUfUmCmAmUmAmCmAmA(moe)Am | UmsUfsUmGmUmAfUmG(moe)AmAfGmCmAmAfUmUfCmUmCmsCmsUm |
| 32 | CmsAmsAmCmUmCmAfCfCfUfGmUmAmAmUmAmAmAmUm | AmsUfsUmUmAmUfUmAmCfAmGmGmUmGfA(moe)GfUmUmGmsAmsUm |
| 33 | CmsAmsAmCmUmCmAfCfCfUfGmUmAmAmUmAmAmAmUm | AmsUfsUmUmAmUfUmAmCmAfGmGmUmGfA(moe)GfUmUmGmsAmsUm |
| 34 | CmsAmsAmCmUmCmAfCfCfUfGmUmAmAmUmAmAmAmUm | AmsUfsUmUmAmUfUmAmCmAmGfGmUmGfA(moe)GfUmUmGmsAmsUm |
| 35 | CmsAmsAmCmT(moe)CmAfCfCfUfGmUmAmAmUmAmAmAmUm | AmsUfsUmUmAmUfUmAmCmAfGmGmUmGfA(moe)GfUmUmGmsAmsUm |
| 36 | CmsAmsAmCmUmCmAfCfCfUfGmT(moe)AmAmUmAmAmAmUm | AmsUfsUmUmAmUfUmAmCmAfGmGmUmGfA(moe)GfUmUmGmsAmsUm |
| 37 | CmsAmsAmCmUmCmAfCfCfUfGmUmA(moe)AmUmAmAmAmUm | AmsUfsUmUmAmUfUmAmCmAfGmGmUmGfA(moe)GfUmUmGmsAmsUm |
| 38 | CmsAmsAmCmUmCmAfCfCfUfGmUmAmAmUmAmAmA(moe)Um | AmsUfsUmUmAmUfUmAmCmAfGmGmUmGfA(moe)GfUmUmGmsAmsUm |
| 39 | CmsAmsAmCmT(moe)CmAfCfCfUfGmUmAmAmUmAmAmAmUm | AmsUfsUmUmAmUfUmA(moe)CmAfGmGmUmGfAmGfUmUmGmsAmsUm |
| 40 | CmsAmsAmCmUmCmAfCfCfUfGmT(moe)AmAmUmAmAmAmUm | AmsUfsUmUmAmUfUmA(moe)CmAfGmGmUmGfAmGfUmUmGmsAmsUm |
| 41 | CmsAmsAmCmUmCmAfCfCfUfGmUmA(moe)AmUmAmAmAmUm | AmsUfsUmUmAmUfUmA(moe)CmAfGmGmUmGfAmGfUmUmGmsAmsUm |
| 42 | CmsAmsAmCmUmCmAfCfCfUfGmUmAmAmUmAmAmA(moe)Um | AmsUfsUmUmAmUfUmA(moe)CmAfGmGmUmGfAmGfUmUmGmsAmsUm |
| 43 | CmsGmsAmAmGmCmUfCfAfUfGmAmAmUmAmUmAmUmUm | AmsAfsUmAmUmAfUmUmCmAmUfGmAmGfC(moe)UfUmCmGmsUmsAm |
| 44 | CmsGmsAmAmG(moe)CmUfCfAfUfGmAmAmUmAmUmAmUmUm | AmsAfsUmAmUmAfUmUmCmAmUfGmAmGfC(moe)UfUmCmGmsUmsAm |
| 45 | CmsGmsAmAmGmCmUfCfAfUfGmAmA(moe)UmAmUmAmUmUm | AmsAfsUmAmUmAfUmUmCmAmUfGmAmGfC(moe)UfUmCmGmsUmsAm |
| 46 | CmsGmsAmAmGmCmUfCfAfUfGmAmA(moe)UmAmUmAmUmUm | AmsAfsUmAmUmAfUmT(moe)CmAmUfGmAmGfCmUfUmCmGmsUmsAm |
| 47 | CmsGmsAmAmGmCmUfCfAfUfGmAmAmUmAmUmAmT(moe)Um | AmsAfsUmAmUmAfUmT(moe)CmAmUfGmAmGfCmUfUmCmGmsUmsAm |
| 48 | CmsAmsGmAmGmAmAfAfUfUfCmUmAmCmUmAmCmAmUm | AmsUfsGmUmAmGfUmAmGmAmAfUmUmUfC(moe)UfCmUmGmsUmsAm |

(continued)

| Group | Sense strand (5'-3') | Antisense strand (5'-3') |
|---|---|---|
| 49 | CmsAmsGmAmGmAmAfAfUfUfCmUmA(moe)CmUmAmCmAmUm | AmsUfsGmUmAmGfUmAmGmAmAfUmUmUfC(moe)UfCmUmGmsUmsAm |
| 50 | CmsAmsGmAmGmAmAfAfUfUfCmUmAmCmUmAmCmA(moe)Um | AmsUfsGmUmAmGfUmA(moe)GmAmAfUmUmUfCmUfCmUmGmsUmsAm |
| 51 | CmsAmsAmCmUmCmAfCfCfUfGmUmA(moe)AmUmAmAmAmUm | AmsUfsUmUmAmUfUmAmCmAmGfGmUmGfA(moe)GfUmUmGmsAmsUm |
| 52 | CmsAmsAmCmUmCmAfCfCfUfGmUmAmAmUmAmAmA(moe)Um | AmsUfsUmUmAmUfUmA(moe)CmAmGfGmUmGfAmGfUmUmGmsAmsUm |
| 53 | CmsAmsGmAmGmAmAfAfUfUfCmUmAmCmUmAmCmAmUm | AmsUfsGmUmAmGfUmAmGmAmAmUfUmUfC(moe)UfCmUmGmsUmsAm |
| 54 | AmsAmsAmUmUmCmUfAfCfUfAmCmAmUmCmUmAmUmAm | UmsAfsUmAmGmAfUmGmUmAmGmUfAmGfAmAfUmUmUmsCmsUm |
| 55 | AmsAmsAmUmUmCmUfAfCfUfAmCmAmUmCmUmAmUmAm | UmsAfsUmAmGmAfUmGmUfAmGmUmAmGfA(moe)AfUmUmUmsCmsUm |
| 56 | AmsAmsAmUmUmCmUfAfCfUfAmCmAmUmCmUmAmUmAm | UmsAfsUmAmGmAfUmGmUmAmGmUfAmGfA(moe)AfUmUmUmsCmsUm |
| 57 | CmsGmsAmAmGmCmUfCfAfUfGmAmAmUmAmUmAmUmUm | AmsAfsUmAmUmAfUmUmCfAmUmGmAmGfCmUfUmCmGmsUmsAm |
| 58 | CmsGmsAmAmGmCmUfCfAfUfGmAmAmUmAmUmAmUmUm | AmsAfsUmAmUmAfUmUmCmAmUmGfAmGfCmUfUmCmGmsUmsAm |
| 59 | CmsGmsAmAmGmCmUfCfAfUfGmAmAmUmAmUmAmUmUm | AmsAfsUmAmUmAfUmUmCmAmUmGfAmGfC(moe)UfUmCmGmsUmsAm |
| 60 | CmsAmsAmCmUmCmAfCfCfUfGmUmAmAmUmAmAmAmUm | AmsUfsUmUmAmUfUmAmCfAmGmGmUmGfAmGfUmUmGmsAmsUm |
| 61 | CmsAmsAmCmUmCmAfCfCfUfGmUmAmAmUmAmAmAmUm | AmsUfsUmUmAmUfUmAmCmAmGmGfUmGfAmGfUmUmGmsAmsUm |
| 62 | CmsAmsAmCmUmCmAfCfCfUfGmUmAmAmUmAmAmAmUm | AmsUfsUmUmAmUfUmAmCmAmGmGfUmGfA(moe)GfUmUmGmsAmsUm |
| 63 | CmsAmsGmAmGmAmAfAfUfUfCmUmAmCmUmAmCmAmUm | AmsUfsGmUmAmGfUmAmGfAmAmUmUmUfCmUfCmUmGmsUmsAm |
| 64 | CmsAmsGmAmGmAmAfAfUfUfCmUmAmCmUmAmCmAmUm | AmsUfsGmUmAmGfUmAmGmAmAmUfUmUfCmUfCmUmGmsUmsAm |
| 65 | AmsAmsAmUmUmCmUfAfCfUfAmCmAmUmCmUmAmUmAm | UmsAfsUmAmGmAfUmGmUfAmGmUmAmGfAmAfUmUmUmsCmsUm |
| 66 | GmsAmsGmAmAmUmUfGfCfUfUmCmAmUmAmCmAmAmAm | UmsUfsUmGmUmAfUmGmAfAmGmCmAmAfUmUfCmUmCmsCmsUm |
| 67 | GmsAmsGmAmAmUmUfGfCfUfUmCmAmUmAmCmAmAmAm | UmsUfsUmGmUmAfUmGmAmAmGmCfAmAfUmUfCmUmCmsCmsUm |

(continued)

| Group | Sense strand (5'-3') | Antisense strand (5'-3') |
|---|---|---|
| 68 | GmsAmsGmAmAmUmUfGfCfUfUmCmAmUmAmCmAmAmAm | UmsUfsUmGmUmAfUmGmAfAmGmCmAmAfU(moe)UfCmUmCmsCmsUm |
| 69 | GmsAmsGmAmAmUmUfGfCfUfUmCmAmUmAmCmAmAmAm | UmsUfsUmGmUmAfUmGmAmAmGmCfAmAfU(moe)UfCmUmCmsCmsUm |

Preparative example 4: Synthesis of the sense strand conjugated with (CR01008×3) carrier at 3' end

(4.1) Synthesis of sense strand

According to the solid-phase nucleic acids synthesis using phosphoramidite method, nucleoside monomers were linked one by one in the direction from 3' to 5' according to the nucleotides sequence, starting from the compound CR01008Z connected to the solid phase support in cycles (compound CR01008 was considered as a nucleoside monomer).

The linking of each nucleoside monomer comprised a four-step reaction of deprotection, coupling, capping, and oxidation or sulfurization. The conditions for deprotection, coupling, capping, oxidation or sulfurization reaction, cleavage, deprotection, purification and desalination in the synthesis of the sense strand in this preparative example were the same as those for the synthesis of the sense strand of step (3.1) in preparative example 3.

In this step, a trimeric CR01008 was synthesized during the synthesis of the sense strand, denoted by (CR01008)×3 or (CR01008×3).

The structure formula of the trimeric CR01008 is given below:

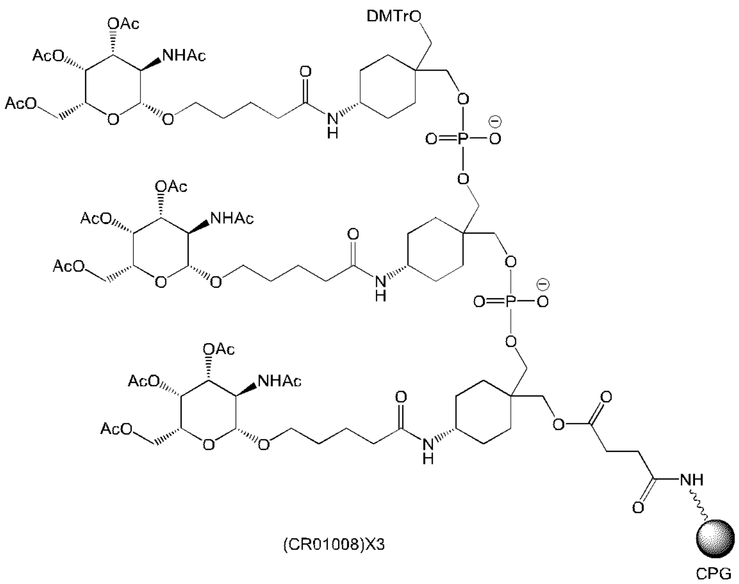

(CR01008)X3

(5.2) Synthesis of antisense strand

The antisense strand of this preparative example was synthesized according to the antisense strand synthesis method shown in step (3.2) of preparative example 3.

(5.3) Synthesis of siRNA conjugate

The compound conjugated with oligonucleotide of the present preparative example was synthesized according to the method shown in step (3.3) of preparative example 3.

Wherein, when the ligand is trimeric CR01008, the structure formula of the siRNA conjugate is shown below:

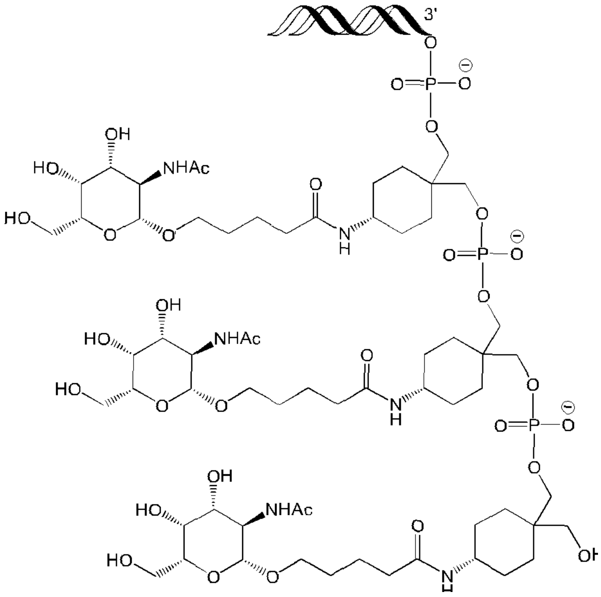

wherein,

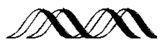

represents siRNA. The (CR01008×3) carrier was conjugated to siRNA at 3’ end of the sense strand. Compounds conjugated with oligonucleotides shown in Table 6 were prepared according to the methods provided in the present disclosure.

Table 6 Sequence information of compounds conjugated with oligonucleotides

| | Sense strand (5’-3’) | Antisense strand (5’-3’) |
|---|---|---|
| RZ002033 | CmsGmsAmAmGmCmUfCfAfUfGmAmAmUmAmUmAmUmUm_(CR01008×3) | AmsAfsUmAmUmAfUmUmCfAmUmGmAmGfC(moe)UfUmCmGmsUmsAm |
| RZ002034 | CmsGmsAmAmGmCmUfCfAfUfGmAmAmUmAmUmAmUmUm_(CR01008×3) | AmsAfsUmAmUmAfUmUmCmAfUmGmAmGfC(moe)UfUmCmGmsUmsAm |
| RZ002036 | CmsGmsAmAmG(moe)CmUfCfAfUfGmAmAmUmAmUmAmUmUm_(CR01008×3) | AmsAfsUmAmUmAfUmUmCmAfUmGmAmGfC(moe)UfUmCmGmsUmsAm |
| RZ002037 | CmsGmsAmAmGmCmUfCfAfUfGmA(moe)AmUmAmUmAmUmUm_(CR01008×3) | AmsAfsUmAmUmAfUmUmCmAfUmGmAmGfC(moe)UfUmCmGmsUmsAm |
| RZ002038 | CmsGmsAmAmGmCmUfCfAfUfGmAmA(moe)UmAmUmAmUmUm_(CR01008×3) | AmsAfsUmAmUmAfUmUmCmAfUmGmAmGfC(moe)UfUmCmGmsUmsAm |
| RZ002039 | CmsGmsAmAmGmCmUfCfAfUfGmAmAmUmAmUmAmT(moe)Um_(CR01008×3) | AmsAfsUmAmUmAfUmUmCmAfUmGmAmGfC(moe)UfUmCmGmsUmsAm |
| RZ002040 | CmsGmsAmAmG(moe)CmUfCfAfUfGmAmAmUmAmUmAmUmUm (CR01008×3) | AmsAfsUmAmUmAfUmT(moe)CmAfUmGmAmGfCmUfUmCmGmsUmsAm |

(continued)

| | Sense strand (5'-3') | Antisense strand (5'-3') |
|---|---|---|
| RZ002041 | CmsGmsAmAmGmCmUfCfAfUfGmA(moe)AmUmAmUmAmUmUm_(CR01008×3) | AmsAfsUmAmUmAfUmT(moe)CmAfUmGmAmGfCmUfUmCmGmsUmsAm |
| RZ002042 | CmsGmsAmAmGmCmUfCfAfUfGmAmA(moe)UmAmUmAmUmUm_(CR01008×3) | AmsAfsUmAmUmAfUmT(moe)CmAfUmGmAmGfCmUfUmCmGmsUmsAm |
| RZ002043 | CmsGmsAmAmGmCmUfCfAfUfGmAmAmUmAmUmAmT(moe)Um_(CR01008×3) | AmsAfsUmAmUmAfUmT(moe)CmAfUmGmAmGfCmUfUmCmGmsUmsAm |
| RZ002050 | CmsAmsGmAmGmAmAfAfUfUfCmUmAmCmUmAmCmAmUm_(CR01008×3) | AmsUfsGmUmAmGfUmAmGfAmAmUmUmUfC(moe)UfCmUmGmsUmsAm |
| RZ002051 | CmsAmsGmAmGmAmAfAfUfUfCmUmAmCmUmAmCmAmUm_(CR01008×3) | AmsUfsGmUmAmGfUmAmGmAfAmUmUmUfC(moe)UfCmUmGmsUmsAm |
| RZ002053 | CmsAmsGmAmG(moe)AmAfAfUfUfCmUmAmCmUmAmCmAmUm_(CR01008×3) | AmsUfsGmUmAmGfUmAmGmAfAmUmUmUfC(moe)UfCmUmGmsUmsAm |
| RZ002054 | CmsAmsGmAmGmAmAfAfUfUfCmT(moe)AmCmUmAmCmAmUm_(CR01008×3) | AmsUfsGmUmAmGfUmAmGmAfAmUmUmUfC(moe)UfCmUmGmsUmsAm |
| RZ002055 | CmsAmsGmAmGmAmAfAfUfUfCmUmA(moe)CmUmAmCmAmUm_(CR01008×3) | AmsUfsGmUmAmGfUmAmGmAfAmUmUmUfC(moe)UfCmUmGmsUmsAm |
| RZ002056 | CmsAmsGmAmGmAmAfAfUfUfCmUmAmCmUmAmCmA(moe)Um_(CR01008×3) | AmsUfsGmUmAmGfUmAmGmAfAmUmUmUfC(moe)UfCmUmGmsUmsAm |
| RZ002057 | CmsAmsGmAmG(moe)AmAfAfUfUfCmUmAmCmUmAmCmAmUm_(CR01008×3) | AmsUfsGmUmAmGfUmA(moe)GmAfAmUmUmUfCmUfCmUmGmsUmsAm |
| RZ002058 | CmsAmsGmAmGmAmAfAfUfUfCmT(moe)AmCmUmAmCmAmUm_(CR01008×3) | AmsUfsGmUmAmGfUmA(moe)GmAfAmUmUmUfCmUfCmUmGmsUmsAm |
| RZ002059 | CmsAmsGmAmGmAmAfAfUfUfCmUmA(moe)CmUmAmCmAmUm_(CR01008×3) | AmsUfsGmUmAmGfUmA(moe)GmAfAmUmUmUfCmUfCmUmGmsUmsAm |
| RZ002060 | CmsAmsGmAmGmAmAfAfUfUfCmUmAmCmUmAmCmA(moe)Um_(CR01008×3) | AmsUfsGmUmAmGfUmA(moe)GmAfAmUmUmUfCmUfCmUmGmsUmsAm |
| RZ002066 | GmsAmsGmAmAmUmUfGfCfUfUmCmAmUmAmCmAmAmAm_(CR01008×3) | UmsUfsUmGmUmAfUmGmAfAmGmCmAmAfT(moe)UfCmUmCmsCmsUm |

(continued)

| | Sense strand (5'-3') | Antisense strand (5'-3') |
|---|---|---|
| RZ002067 | GmsAmsGmAmAmUmUfGfCfUfUmCmAmUmAmCmAmAmAm_(CR01008×3) | UmsUfsUmGmUmAfUmGmAmAfGmCmAmAfT(moe)UfCmUmCmsCmsUm |
| RZ002068 | GmsAmsGmAmAmUmUfGfCfUfUmCmAmUmAmCmAmAmAm_(CR01008×3) | UmsUfsUmGmUmAfUmGmAmAmGfCmAmAfT(moe)UfCmUmCmsCmsUm |
| RZ002069 | GmsAmsGmAmA(moe)UmUfGfCfUfUmCmAmUmAmCmAmAmAm_(CR01008×3) | UmsUfsUmGmUmAfUmGmAmAfGmCmAmAfT(moe)UfCmUmCmsCmsUm |
| RZ002070 | GmsAmsGmAmAmUmUfGfCfUfUmC(moe)AmUmAmCmAmAmAm_(CR01008×3) | UmsUfsUmGmUmAfUmGmAmAfGmCmAmAfT(moe)UfCmUmCmsCmsUm |
| RZ002071 | GmsAmsGmAmAmUmUfGfCfUfUmCmA(moe)UmAmCmAmAmAm_(CR01008×3) | UmsUfsUmGmUmAfUmGmAmAfGmCmAmAfT(moe)UfCmUmCmsCmsUm |
| RZ002072 | GmsAmsGmAmAmUmUfGfCfUfUmCmAmUmAmCmAmA(moe)Am_(CR01008×3) | UmsUfsUmGmUmAfUmGmAmAfGmCmAmAfT(moe)UfCmUmCmsCmsUm |
| RZ002073 | GmsAmsGmAmA(moe)UmUfGfCfUfUmCmAmUmAmCmAmAmAm_(CR01008×3) | UmsUfsUmGmUmAfUmG(moe)AmAfGmCmAmAfUmUfCmUmCmsCmsUm |
| RZ002074 | GmsAmsGmAmAmUmUfGfCfUfUmC(moe)AmUmAmCmAmAmAm_(CR01008×3) | UmsUfsUmGmUmAfUmG(moe)AmAfGmCmAmAfUmUfCmUmCmsCmsUm |
| RZ002075 | GmsAmsGmAmAmUmUfGfCfUfUmCmA(moe)UmAmCmAmAmAm_(CR01008×3) | UmsUfsUmGmUmAfUmG(moe)AmAfGmCmAmAfUmUfCmUmCmsCmsUm |
| RZ002076 | GmsAmsGmAmAmUmUfGfCfUfUmCmAmUmAmCmAmA(moe)Am_(CR01008×3) | UmsUfsUmGmUmAfUmG(moe)AmAfGmCmAmAfUmUfCmUmCmsCmsUm |
| RZ002082 | CmsAmsAmCmUmCmAfCfCfUfGmUmAmAmUmAmAmAmUm_(CR01008×3) | AmsUfsUmUmAmUfUmAmCfAmGmGmUmGfA(moe)GfUmUmGmsAmsUm |
| RZ002083 | CmsAmsAmCmUmCmAfCfCfUfGmUmAmAmUmAmAmAmUm_(CR01008×3) | AmsUfsUmUmAmUfUmAmCmAfGmGmUmGfA(moe)GfUmUmGmsAmsUm |
| RZ002084 | CmsAmsAmCmUmCmAfCfCfUfGmUmAmAmUmAmAmAmUm_(CR01008×3) | AmsUfsUmUmAmUfUmAmCmAmGfGmUmGfA(moe)GfUmUmGmsAmsUm |
| RZ002085 | CmsAmsAmCmT(moe)CmAfCfCfUfGmUmAmAmUmAmAmAmUm_(CR01008×3) | AmsUfsUmUmAmUfUmAmCmAfGmGmUmGfA(moe)GfUmUmGmsAmsUm |

(continued)

| | Sense strand (5'-3') | Antisense strand (5'-3') |
|---|---|---|
| RZ002086 | CmsAmsAmCmUmCmAfCfCfUfGmT(moe)AmAmUmAmAmAmUm_(CR01008×3) | AmsUfsUmUmAmUfUmAmCmAfGmGmUmGfA(moe)GfUmUmGmsAmsUm |
| RZ002087 | CmsAmsAmCmUmCmAfCfCfUfGmUmA(moe)AmUmAmAmAmUm_(CR01008×3) | AmsUfsUmUmAmUfUmAmCmAfGmGmUmGfA(moe)GfUmUmGmsAmsUm |
| RZ002088 | CmsAmsAmCmUmCmAfCfCfUfGmUmAmAmUmAmAmA(moe)Um_(CR01008×3) | AmsUfsUmUmAmUfUmAmCmAfGmGmUmGfA(moe)GfUmUmGmsAmsUm |
| RZ002089 | CmsAmsAmCmT(moe)CmAfCfCfUfGmUmAmAmUmAmAmAmUm_(CR01008×3) | AmsUfsUmUmAmUfUmA(moe)CmAfGmGmUmGfAmGfUmUmGmsAmsUm |
| RZ002090 | CmsAmsAmCmUmCmAfCfCfUfGmT(moe)AmAmUmAmAmAmUm_(CR01008×3) | AmsUfsUmUmAmUfUmA(moe)CmAfGmGmUmGfAmGfUmUmGmsAmsUm |
| RZ002091 | CmsAmsAmCmUmCmAfCfCfUfGmUmA(moe)AmUmAmAmAmUm_(CR01008×3) | AmsUfsUmUmAmUfUmA(moe)CmAfGmGmUmGfAmGfUmUmGmsAmsUm |
| RZ002092 | CmsAmsAmCmUmCmAfCfCfUfGmUmAmAmUmAmAmA(moe)Um_(CR01008×3) | AmsUfsUmUmAmUfUmA(moe)CmAfGmGmUmGfAmGfUmUmGmsAmsUm |
| RZ002099 | CmsGmsAmAmGmCmUfCfAfUfGmAmAmUmAmUmAmUmUm_(CR01008×3) | AmsAfsUmAmUmAfUmUmCmAmUfGmAmGfC(moe)UfUmCmGmsUmsAm |
| RZ002100 | CmsGmsAmAmG(moe)CmUfCfAfUfGmAmAmUmAmUmAmUmUm_(CR01008×3) | AmsAfsUmAmUmAfUmUmCmAmUfGmAmGfC(moe)UfUmCmGmsUmsAm |
| RZ002101 | CmsGmsAmAmGmCmUfCfAfUfGmAmA(moe)UmAmUmAmUmUm_(CR01008×3) | AmsAfsUmAmUmAfUmUmCmAmUfGmAmGfC(moe)UfUmCmGmsUmsAm |
| RZ002102 | CmsGmsAmAmGmCmUfCfAfUfGmAmA(moe)UmAmUmAmUmUm_(CR01008×3) | AmsAfsUmAmUmAfUmT(moe)CmAmUfGmAmGfCmUfUmCmGmsUmsAm |
| RZ002103 | CmsGmsAmAmGmCmUfCfAfUfGmAmAmUmAmUmAmT(moe)Um_(CR01008×3) | AmsAfsUmAmUmAfUmT(moe)CmAmUfGmAmGfCmUfUmCmGmsUmsAm |
| RZ002106 | CmsAmsGmAmGmAmAfAfUfUfCmUmAmCmUmAmCmAmUm_(CR01008×3) | AmsUfsGmUmAmGfUmAmGmAmAfUmUmUfC(moe)UfCmUmGmsUmsAm |
| RZ002107 | CmsAmsGmAmGmAmAfAfUfUfCmUmA(moe)CmUmAmCmAmUm_(CR01008×3) | AmsUfsGmUmAmGfUmAmGmAmAfUmUmUfC(moe)UfCmUmGmsUmsAm |

(continued)

| | Sense strand (5'-3') | Antisense strand (5'-3') |
|---|---|---|
| RZ00210E | CmsAmsGmAmGmAmAfAfUfUfCmUmAmCmUmAmCmA(moe)Um_(CR01008×3) | AmsUfsGmUmAmGfUmA(moe)GmAmAfUmUmUfCmUfCmUmGmsUmsAm |
| RZ002112 | CmsAmsAmCmUmCmAfCfCfUfGmUmA(moe)AmUmAmAmAmUm_(CR01008×3) | AmsUfsUmUmAmUfUmAmCmAmGfGmUmGfA(moe)GfUmUmGmsAmsUm |
| RZ002113 | CmsAmsAmCmUmCmAfCfCfUfGmUmAmAmUmAmAmA(moe)Um_(CR01008×3) | AmsUfsUmUmAmUfUmA(moe)CmAmGfGmUmGfAmGfUmUmGmsAmsUm |
| RZ002115 | CmsAmsGmAmGmAmAfAfUfUfCmUmAmCmUmAmCmAmUm_(CR01008×3) | AmsUfsGmUmAmGfUmAmGmAmAmUfUmUfC(moe)UfCmUmGmsUmsAm |
| RZ002116 | AmsAmsAmUmUmCmUfAfCfUfAmCmAmUmCmUmAmUmAm_(CR01008×3) | UmsAfsUmAmGmAfUmGmUmAmGmUfAmGfAmAfUmUmUmsCmsUm |
| RZ002117 | AmsAmsAmUmUmCmUfAfCfUfAmCmAmUmCmUmAmUmAm_(CR01008×3) | UmsAfsUmAmGmAfUmGmUfAmGmUmAmGfA(moe)AfUmUmUmsCmsUm |
| RZ002118 | AmsAmsAmUmUmCmUfAfCfUfAmCmAmUmCmUmAmUmAm_(CR01008×3) | UmsAfsUmAmGmAfUmGmUmAmGmUfAmGfA(moe)AfUmUmUmsCmsUm |
| RZ002119 | CmsGmsAmAmGmCmUfCfAfUfGmAmAmUmAmUmAmUmUm_(CR01008×3) | AmsAfsUmAmUmAfUmUmCfAmUmGmAmGfCmUfUmCmGmsUmsAm |
| RZ00212 | CmsGmsAmAmGmCmUfCfAfUfGmAmAmUmAmUmAmUmUm (CR01008×3) | AmsAfsUmAmUmAfUmUmCmAmUmGfAmGfCmUfUmCmGmsUmsAm |
| RZ002121 | CmsGmsAmAmGmCmUfCfAfUfGmAmAmUmAmUmAmUmUm_(CR01008×3) | AmsAfsUmAmUmAfUmUmCmAmUmGfAmGfC(moe)UfUmCmGmsUmsAm |
| RZ002122 | CmsAmsAmCmUmCmAfCfCfUfGmUmAmAmUmAmAmAmUm_(CR01008×3) | AmsUfsUmUmAmUfUmAmCfAmGmGmUmGfAmGfUmUmGmsAmsUm |
| RZ002123 | CmsAmsAmCmUmCmAfCfCfUfGmUmAmAmUmAmAmAmUm_(CR01008×3) | AmsUfsUmUmAmUfUmAmCmAmGmGfUmGfAmGfUmUmGmsAmsUm |
| RZ002124 | CmsAmsAmCmUmCmAfCfCfUfGmUmAmAmUmAmAmAmUm_(CR01008×3) | AmsUfsUmUmAmUfUmAmCmAmGmGfUmGfA(moe)GfUmUmGmsAmsUm |
| RZ002125 | CmsAmsGmAmGmAmAfAfUfUfCmUmAmCmUmAmCmAmUm_(CR01008×3) | AmsUfsGmUmAmGfUmAmGfAmAmUmUmUfCmUfCmUmGmsUmsAm |

(continued)

| | Sense strand (5'-3') | Antisense strand (5'-3') |
|---|---|---|
| RZ002126 | CmsAmsGmAmGmAmAfAfUfUfCmUmAmCmUmAmCmAmUm_(CR01008×3) | AmsUfsGmUmAmGfUmAmGmAmAmUfUmUfCmUfCmUmGmsUmsAm |
| RZ002130 | AmsAmsAmUmUmCmUfAfCfUfAmCmAmUmCmUmAmUmAm_(CR01008×3) | UmsAfsUmAmGmAfUmGmUfAmGmUmAmGfAmAfUmUmUmsCmsUm |
| RZ002131 | GmsAmsGmAmAmUmUfGfCfUfUmCmAmUmAmCmAmAmAm_(CR01008×3) | UmsUfsUmGmUmAfUmGmAfAmGmCmAmAfUmUfCmUmCmsCmsUm |
| RZ002132 | GmsAmsGmAmAmUmUfGfCfUfUmCmAmUmAmCmAmAmAm_(CR01008×3) | UmsUfsUmGmUmAfUmGmAmAmGmCfAmAfUmUfCmUmCmsCmsUm |
| RZ002133 | GmsAmsGmAmAmUmUfGfCfUfUmCmAmUmAmCmAmAmAm_(CR01008×3) | UmsUfsUmGmUmAfUmGmAfAmGmCmAmAfU(moe)UfCmUmCmsCmsUm |
| RZ002134 | GmsAmsGmAmAmUmUfGfCfUfUmCmAmUmAmCmAmAmAm_(CR01008×3) | UmsUfsUmGmUmAfUmGmAmAmGmCfAmAfU(moe)UfCmUmCmsCmsUm |
| "_(CR01008×3)" indicates that the ligand represented by (CR01008×3) is conjugated to the 3' end of the sense strand. | | |

Unless otherwise specified, the meanings of the base compositions and modifications described in various examples of the present disclosure are as follows: uppercase letters A, U, G, C and T represent the base composition of the nucleotides, lowercase letter m represents that the nucleotide adjacent to the left side of the letter m is a 2'-O-methyl-modified (also known as 2'-methoxy-modified) nucleotide, lowercase letter f represents that the nucleotide adjacent to the left side of the letter f is a 2'-fluoro-modified nucleotide, lowercase letter d represents that the nucleotide adjacent to the left side of the letter d is a 2'-deoxy-modified ribonucleic acid (also known as: deoxyribonucleic acid), (moe) represents that the nucleotide adjacent to the left side of the combination sign (moe) is a 2'-O-methoxyethyl-modified nucleotide, and lowercase letter s represents that the two nucleotides adjacent to both sides of the letter s are linked by a phosphorothioate linkage.

Biological assay

In the present disclosure, unless otherwise indicated, the siRNA sequences used in the present disclosure were synthesized by Kunshan Alltest Biotech Co., Ltd., the PCR primers used in the present disclosure were synthesized by Sangon Biotech (Shanghai) Co., Ltd., Hepatoma HepG2 cells used in the present disclosure were purchased from Wuhan Pricella Biotechnology Co., Ltd., the experimental animals including BALB/c mice and SD rats used in the present disclosure were purchased from Zhejiang Vital River Laboratory Animal Technology Co., Ltd., healthy cynomolgus monkeys used in the present disclosure were purchased from Guangdong Landau Biotechnology Co., Ltd. and Guangxi Xiongsen Primate Experimental Animal Breeding Development Co., Ltd., B-hC3 mice used in the present disclosure were purchased from Biocytogen Pharmaceuticals (Beijing) Co., Ltd., the cynomolgus monkey nephropathy model used in the present disclosure was constructed by Tipmax (Suzhou) Pharmaceutical Technology Co., Ltd., and CFA-hIgA nephropathy mice model used in the present disclosure were constructed by the First Hospital of Peking University.

In the present disclosure, unless otherwise indicated, for data from real-time PCR assays related to *in vivo* activity assays, the ΔΔCt method was used to relatively quantify the mRNA expression of the target gene in each test group, which was calculated as follows:

$$\Delta Ct(\text{test group}) = Ct(\text{target gene in test group}) - Ct(\text{internal reference gene in test group})$$

$\Delta$Ct(control group) = Ct(target gene in control group) - Ct(internal reference gene in control group)

$$\Delta\Delta Ct(\text{test group}) = \Delta Ct(\text{test group}) - \Delta Ct(\text{mean of control group})$$

$$\Delta\Delta Ct(\text{control group}) = \Delta Ct(\text{control group}) - \Delta Ct(\text{mean of control group})$$

The mRNA expression level of the target gene in the test group was normalized using the control group as the baseline, and the mRNA expression level of the target gene in the control group was defined as 100%.

Relative retained expression level of mRNA of target gene in test group = $2^{-\Delta\Delta Ct\ (\text{test group})} \times 100\%$

Inhibition rate of mRNA of target gene in test group = 100% - relative retained expression level of mRNA of target gene in test group

Data for the *in vivo* activity assay of C3 protein at different time points covered by the present disclosure are processed as shown below.

$$\text{Relatively remained expression level of C3 protein} = \frac{Dx}{Pre-dose} \times 100\%$$

Wherein, pre-dose represents a C3 protein concentration before administration, and Dx represents a C3 protein concentration on day x after administration.

Data for the *in vivo* activity assay of CCP and CAP at different time points covered by the present disclosure are processed as shown below.

$$\text{Relatively remained activity of CAP or CCP} = \frac{Dx}{Pre-dose} \times 100\%$$

Wherein, pre-dose represents CCP or CAP activity before administration, and Dx represents CCP or CAP activity on day x after administration.

In the present disclosure, unless otherwise indicated, all data for *in vivo* activity assay is presented as $\overline{X}\pm SD$ ($\overline{X}\pm STDEV$ (standard deviation)). The experimental data were graphed and analyzed using GraphPad Prism 8.0 software.

Example 1 *In vitro* activity evaluation of compounds of (CR01008)×3 conjugated at 3' end of sense strand

In this Example, the human hepatocellular carcinoma cell line HepG2 was used to evaluate the inhibition activity of compounds of (CR01008)×3 conjugated at 3' end of sense strand on mRNA of target C3 gene by an evaluation experiment of inhibition on target gene expression.

Preparation of samples to be tested

After each of the above siRNA test products was centrifuged, they were dissolved with appropriate amount of PBS according to the specifications of each tube and configured into a 20 μM stock solution, which was further diluted into 0.5 μM and 0.05 μM working solutions by gradient dilution with PBS. Dose test was performed with final concentrations of double-stranded siRNA of 5 nM and 0.5 nM .

Transfection (96-well plate) and Measurement

HepG2 cells grown to near confluence were digested with trypsin, washed and prepared into a cell suspension. 100 μL of cell suspension was added to each well of a 96-well plate, with 12,000 cells per well. Cells were cultured in an incubator at 37°C, 5% $CO_2$. After cells were adhered to the wall for 24 h, the DMEM medium in the 96-well plate was discarded, 80 μL of Opti-MEM™ medium was added to each well of the 96-well plate, and then the 96-well plate was incubated in the incubator. 1 μL of 0.1 μM and 0.01 μM working solution was each dispersed in 9 μL of Opti-MEM to form

siRNA mixtures. 0.3 μL of RNAiMAX was dispersed in 9.7 μL of Opti-MEM and then mixed with each siRNA mixture to form transfection complexes. The transfection complexes were incubated at room temperature for 10 min, and then 20 μL of transfection complex was added to each well of the 96-well plate. After 4 h of incubation, each well was supplemented with 100 μL of DMEM medium containing 20% FBS, and the 96-well plates were placed in an incubator for 24 h of incubation. In the Mock control group, 0.3 μL of RNAiMAX was dispersed in 9.7 μL of Opti-MEM, then 10 μL of Opti-MEM was added, and the resulting mixture was incubated at room temperature for 10 min. The mixture was added to a 96-well plate at 20 μL/well. After 4 h of incubation, each well was supplemented with 100 μL of DMEM medium containing 20% FBS, and the 96-well plate was placed in an incubator for 24 h of incubation.

The 96-well plate was taken out, and the total RNA was extracted in accordance with the standard protocol for total RNA extraction by using a fully automated nucleic acid extractor (purchased from Zhejiang Hanwei Technology Co., Ltd.) and a nucleic acid extraction kit (purchased from Zhejiang Hanwei Technology Co., Ltd., GO-MNTR-100).

20 μL of reverse transcription system was prepared using a reverse transcription kit (Thermo Fisher Scientific, RevertAid First Strand cDNA Synthesis Kit, K1622) and Oligo (dT)18 primers for reverse transcription according to the instruction of the reverse transcription kit. The reverse transcription reaction was carried out and completed. The mRNA expression of target gene in HepG2 cells was measured with a real-time fluorescence quantitative PCR kit (Thermo Fisher Scientific, TaqMan Fast Advanced Master Mix , 4444557) using a fluorescence quantitative PCR instrument (Bio-Rad, CFX Opus 384). In the real-time fluorescence quantitative PCR assay, the glyceraldehyde-3-phosphate dehydrogenase (GAPDH) gene was used as the internal reference gene, and primers for the target gene and the internal reference gene were used to detect the target gene and the internal reference gene respectively. Sequences of primers used are shown in Table 7.

Table 7 Primer sequence for measurement

| Gene | | Primer | Primer sequence | Fluorescent group |
|---|---|---|---|---|
| Target gene | C3 | Upstream primer | 5'-AGTCTCCTGCTTTAGTGATGC-3' (SEQ ID NO: 11) | / |
| | | Downstream primer | 5'-GCCTTTGTTCTCATCTCGCT-3' (SEQ ID NO: 12) | / |
| | | Probe primer | 5'-CTGTTGACCTGCTCCTCGCAAATATCT-3' (SEQ ID NO:13) | 5'FAM; 3'MGB |
| Internal reference gene | GAPDH | Upstream primer | 5'-AAGAAGGTGGTGAAGCAGG-3' (SEQ ID NO:14) | / |
| | | Downstream primer | 5'-CAAAGTGGTCGTTGAGGG-3' (SEQ ID NO:15) | / |
| | | Probe primer | 5'-CAACAGCGACACCCACTC-3' (SEQ ID NO:16) | 5'VIC; 3'MGB |

A 10 μL real-time PCR reaction system per PCR reaction well was prepared according to the protocol documented in the instructions of the real-time fluorescence quantitative PCR kit, and each reaction system consisted of 4 μL of cDNA solution obtained by the reverse transcription reaction described above, 5 μL of TaqMan™ Fast Advanced Master Mix (2×), 0.15 μL of 10 μM upstream primer, 0.15 μL of 10 μM downstream primer, 0.15 μL of 10 μM probe primer, and 0.55 μL of RNase-Free $H_2O$. The prepared reaction system was placed in a real-time fluorescence quantitative PCR instrument (Bio-Rad, CFX Opus 384), and amplified by two-step method. The amplification procedure was 2 min at 50°C, predenaturation at 95°C for 20 seconds, denaturation at 95°C for 3 seconds, annealing at 60°C and extension for 30 seconds. The above denaturation, annealing and extension processes were repeated for 40 cycles. In this real-time fluorescence quantitative PCR assay, the mRNA expression level of the target gene C3 in each test group and the inhibition rate was relatively quantified using the ΔΔCt method as described in examples (FIG. 1).

Table 8 Inhibitory activity of the compounds described in this example on mRNA expression of target gene C3 in HepG2 cells after administration

| Compound | 5nM | | 0.5nM | |
|---|---|---|---|---|
| | % Relatively remained expression level | STDEV | % Relatively remained expression level | STDEV |
| Mock | 100.00 | 3.12 | 100.00 | 3.12 |
| RZ002033 | 9.16 | 0.94 | 31.05 | 0.71 |

(continued)

| Compound | 5nM | | 0.5nM | |
|---|---|---|---|---|
| | % Relatively remained expression level | STDEV | % Relatively remained expression level | STDEV |
| RZ002034 | 8.80 | 0.76 | 24.48 | 6.22 |
| RZ002099 | 7.65 | 1.92 | 32.10 | 1.97 |
| RZ002036 | 11.98 | 0.18 | 24.00 | 1.03 |
| RZ002037 | 11.26 | 0.61 | 27.13 | 0.60 |
| RZ002038 | 9.04 | 1.21 | 30.71 | 0.51 |
| RZ002039 | 10.98 | 0.37 | 36.07 | 1.88 |
| RZ002040 | 12.62 | 0.59 | 31.73 | 1.40 |
| RZ002041 | 10.48 | 1.47 | 27.68 | 6.13 |
| RZ002042 | 9.07 | 0.71 | 26.13 | 1.61 |
| RZ002043 | 8.04 | 0.71 | 30.68 | 0.16 |
| RZ002050 | 17.65 | 2.29 | 36.54 | 2.38 |
| RZ002051 | 16.27 | 2.17 | 27.43 | 6.32 |
| RZ002106 | 13.92 | 1.18 | 30.03 | 0.72 |
| RZ002053 | 14.88 | 4.01 | 26.00 | 0.90 |
| RZ002054 | 17.8 | 10.42 | 28.19 | 2.16 |
| RZ002055 | 15.57 | 2.71 | 24.53 | 2.19 |
| RZ002056 | 16.48 | 2.71 | 43.19 | 1.83 |
| RZ002057 | 17.52 | 3.31 | 40.75 | 5.49 |
| RZ002058 | 14.75 | 0.11 | 31.72 | 1.35 |
| RZ002059 | 11.28 | 1.47 | 26.63 | 4.85 |
| RZ002060 | 10.28 | 1.48 | 25.93 | 0.70 |
| RZ002066 | 20.09 | 9.16 | 42.86 | 15.61 |
| RZ002067 | 16.89 | 4.88 | 27.44 | 1.50 |
| RZ002068 | 15.99 | 9.04 | 38.27 | 2.66 |
| RZ002069 | 18.44 | 5.73 | 31.22 | 2.43 |
| RZ002070 | 16.77 | 8.10 | 34.34 | 1.93 |
| RZ002071 | 16.24 | 5.08 | 32.74 | 1.91 |
| RZ002072 | 24.64 | 4.76 | 53.87 | 6.33 |
| RZ002073 | 34.06 | 3.25 | 51.24 | 0.29 |
| RZ002074 | 14.91 | 0.16 | 44.37 | 1.31 |
| RZ002075 | 10.69 | 0.15 | 41.05 | 4.82 |
| RZ002076 | 10.76 | 1.08 | 41.10 | 1.94 |
| RZ002082 | 21.93 | 5.23 | 28.44 | 2.52 |
| RZ002083 | 19.48 | 8.68 | 25.39 | 1.18 |
| RZ002084 | 15.71 | 8.17 | 27.85 | 2.26 |
| RZ002085 | 23.27 | 5.75 | 33.20 | 2.55 |
| RZ002086 | 21.12 | 8.65 | 34.10 | 6.23 |
| RZ002087 | 21.77 | 0.85 | 36.53 | 3.15 |

(continued)

| Compound | 5nM | | 0.5nM | |
|---|---|---|---|---|
| | % Relatively remained expression level | STDEV | % Relatively remained expression level | STDEV |
| RZ002088 | 34.48 | 0.91 | 69.65 | 6.92 |
| RZ002089 | 31.49 | 5.73 | 54.13 | 8.64 |
| RZ002090 | 14.91 | 0.03 | 51.77 | 15.52 |
| RZ002091 | 11.73 | 0.65 | 36.02 | 0.00 |
| RZ002092 | 11.67 | 1.28 | 37.72 | 0.36 |

The results of Example 1 showed that CR01008 carrier-conjugated siRNAs with different modifications can significantly inhibit the mRNA expression of C3 gene in HepG2 cells at both doses of 5 nM and 0.5 nM (Table 8).

Example 2 Evaluation of the *in vivo* mRNA-lowering effect of compounds with (CR01008)×3-conjugation at 3' end of the sense strand in normal cynomolgus monkeys

This example evaluated the inhibitory activity of RZ002099, RZ002101, RZ002106, and RZ002113 on the mRNA expression of target gene C3 in cynomolgus monkeys.

Animal grouping, administration and collection of tissue sample

Healthy cynomolgus monkeys weighing 3-5 kg were grouped into four groups, with 4 mice in each group, half male and half female. Each test group was given a predetermined dose of drug conjugate, and a PBS control group was set. The administration dose was calculated based on body weight for all animals, and a single subcutaneous injection in the abdomen was given. Each drug conjugate was prepared into a solution of 9 mg siRNA/mL PBS and administered at a volume of 1 mL/kg body weight of cynomolgus monkey, i.e., the dosing amount for each drug conjugate was 9 mg siRNA/kg body weight of cynomolgus monkey. The PBS control group was given PBS solution without containing siRNA conjugate at 1 mL/kg body weight of cynomolgus monkey. On the day of administration (denoted by D0), and on day 14 after administration (denoted by D14), the liver needle biopsy was carried out in cynomolgus monkeys in each group, and the liver biopsy tissues were immediately stored in RNA later.

For each cynomolgus monkey, the liver tissue sample was taken out of the RNA later, and homogenized in an automatic tissue homogenizer-Tissuelyser II for 60 seconds. According to the standard protocol for total RNA extraction, the total RNA was extracted using an automatic nucleic acid extractor (purchased from Zhejiang Hanwei Technology Co., Ltd.) and a nucleic acid extraction kit (purchased from Zhejiang Hanwei Technology Co., Ltd., GO-MNTR-100).

For each cynomolgus monkey, 1 μg of the total RNA was taken, 20 μL of reverse transcription system was prepared using a reverse transcription kit (Thermo Fisher Scientific, RevertAid First Strand cDNA Synthesis Kit, K1622) and Oligo (dT)18 primer for reverse transcription according the instructions of the reverse transcription kit, and a reverse transcription reaction was carried out. After the reaction was completed, 60 μL of RNase-Free water was added to the reverse transcription system to obtain a cDNA solution. Next, the mRNA expression of the target gene in animals was measured in a fluorescence quantitative PCR instrument (Roche, Lightcycler 480II) using a real-time fluorescence quantitative PCR kit (Thermo Fisher Scientific, TaqMan Fast Advanced Master Mix, 4444557). In the real-time fluorescence quantitative PCR assay, the glyceraldehyde-3-phosphate dehydrogenase (GAPDH) gene was used as the internal reference gene, and primers for the target gene and the internal reference gene were used to detect the target gene and the internal reference gene respectively. Sequences of primers used are shown in Table 9.

Table 9 Primer sequence

| Gene | | Primer | Primer sequence | Fluorescent group |
|---|---|---|---|---|
| Target gene | C3 | Upstream primer | 5'-TCCTGCGATCAGAAGAGACC-3' (SEQ ID NO:17) | / |
| | | Downstream primer | 5'-GACCTTTGGCCTTAGCATGG-3' (SEQ ID NO:18) | / |
| | | Probe primer | 5'-ACCGACAAGGTGCCTTGGCCT-3' (SEQ ID NO:19) | 5'FAM; 3'MGB |

(continued)

| Gene | | Primer | Primer sequence | Fluorescent group |
|---|---|---|---|---|
| Internal reference gene | GAPDH | Upstream primer | 5'-TCGTGGAAGGACTCATGACC-3' (SEQ ID NO:20) | / |
| | | Downstream primer | 5'-GCAGGGATGATGTTCTGGAG-3' (SEQ ID NO:21) | / |
| | | Probe primer | 5'-CTCCGCGGCCATCACGCCAC-3' (SEQ ID NO:22) | 5'VIC; 3'MGB |

A 10 μL real-time PCR reaction system per PCR reaction well was prepared according to the protocol documented in the instructions of the real-time fluorescence quantitative PCR kit, and each reaction system consisted of 4 μL of cDNA solution obtained by the reverse transcription reaction described above, 5 μL of TaqMan™ Fast Advanced Master Mix (2×), 0.15 μL of 10 μM upstream primer, 0.15 μL of 10 μM downstream primer, 0.15 μL of 10 μM probe primer and 0.55 μL of RNase-Free $H_2O$. The prepared reaction system was placed in a real-time fluorescence quantitative PCR instrument Bio-Rad, CFX Opus 384), and amplified by two-step method. The amplification procedure was 2 min at 50°C, predenaturation at 95°C for 20 seconds, denaturation at 95°C for 3 seconds, annealing at 60°C, and extension for 30 seconds. The above denaturation, annealing and extension processes were repeated for 40 cycles. In this real-time fluorescence quantitative PCR assay, the mRNA expression level of the target gene in each test group and the inhibition rate was relatively quantified using the ΔΔCt method according to the method described in embodiments.

Table 10 Change in C3 mRNA level in liver of cynomolgus monkey after administration of the compounds of this example

| Group | C3 mRNA level (9mg/kg, D14) | |
|---|---|---|
| | % Relatively remained expression level | STDEV |
| PBS | 100.00 | 28.24 |
| RZ002099 | 15.51 | 5.60 |
| RZ002101 | 22.29 | 12.07 |
| RZ002106 | 9.86 | 1.44 |
| RZ002113 | 12.96 | 6.94 |

The results of Example 2 showed that RZ002099, RZ002101, RZ002106, and RZ002113 can significantly inhibit the mRNA expression of C3 gene after a single administration at 9 mg/kg (Table 10 and FIG. 1).

Example 3 Evaluation of the *in vivo* C3 protein-lowering effect of compounds with (CR01008)×3 conjugated at 3' end of the sense strand in normal cynomolgus monkeys

This example measured the C3 protein in serums of cynomolgus monkeys collected at different time points after a single administration of RZ002099, RZ002101, RZ002106, and RZ002113, using enzyme-linked immunosorbent assay (ELISA) method.

Animal grouping, administration and collection of tissue sample

Healthy cynomolgus monkeys weighing 3-5 kg were grouped according to the C3 protein level, with 4 mice in each group, half male and half female. Each test group was given a predetermined dose of drug conjugate, and a PBS control group was set. The administration dose was calculated based on body weight for all animals, and a single subcutaneous injection in the abdomen was given. Each drug conjugate was prepared into a solution form of 9 mg siRNA/mL PBS, and administered at a volume of 1 mL/kg body weight of cynomolgus monkey, i.e., the dosing amount for each drug conjugate was 9 mg siRNA/kg body weight of cynomolgus monkey. The PBS control group was given PBS solution without containing siRNA conjugate at 1 mL/kg body weight of cynomolgus monkey. The serums were collected from cynomolgus monkeys and the C3 protein level was measured with the human C3 ELISA kit (Hycult Biotech, HK366-01) before administration (denoted by pre-dose), on the day of administration (denoted by D0), on day 7 after

administration (denoted by D7), on day 14 after administration (denoted by D14), on day 21 after administration (denoted by D21), on day 28 after administration (denoted by D28), on day 35 after administration (denoted by D35), on day 42 after administration (denoted by D42), on day 49 after administration (denoted by D49), on day 56 after administration (denoted by D56), on day 63 after administration (denoted by D63), on day 70 after administration (denoted by D70), on day 77 after administration (denoted by D77), on day 84 after administration (denoted by D84), on day 91 after administration (denoted by D91), on day 98 after administration (denoted by D98), and on day 105 after administration (denoted by D105).

Table 11 Change in C3 protein level in serum of cynomolgus monkey after administration of the compounds of this example

| Group | pre-dose | | D7 | | D14 | | D21 | |
|---|---|---|---|---|---|---|---|---|
| | % Relatively remained expression level | STDEV | % Relatively remained expression level | STDEV | % Relatively remained expression level | STDEV | % Relatively remained expression level | STDEV |
| PBS | 100.00 | 0.00 | 100.20 | 6.80 | 95.70 | 14.00 | 104.10 | 18.10 |
| RZ002099 | 100.00 | 0.00 | 48.57 | 12.93 | 27.63 | 11.82 | 30.95 | 14.81 |
| RZ002101 | 100.00 | 0.00 | 47.87 | 15.67 | 26.08 | 8.71 | 22.75 | 10.48 |
| RZ002106 | 100.00 | 0.00 | 44.31 | 6.21 | 21.03 | 5.24 | 11.88 | 3.48 |
| RZ002113 | 100.00 | 0.00 | 41.55 | 13.82 | 21.54 | 9.85 | 17.12 | 8.27 |
| | | | | | | | | |
| Group | D28 | | D35 | | D42 | | D49 | |
| | % Relatively remained expression level | STDEV | % Relatively remained expression level | STDEV | % Relatively remained expression level | STDEV | % Relatively remained expression level | STDEV |
| PBS | 82.90 | 10.00 | 79.30 | 7.90 | 80.60 | 4.50 | 88.10 | 8.40 |
| RZ002099 | 24.25 | 11.70 | 26.75 | 8.80 | 30.29 | 15.16 | 37.84 | 24.22 |
| RZ002101 | 19.11 | 6.12 | 20.99 | 4.49 | 23.77 | 6.44 | 21.76 | 4.33 |
| RZ002106 | 13.34 | 6.63 | 12.15 | 4.03 | 11.87 | 4.67 | 9.01 | 1.95 |
| RZ002113 | 16.91 | 5.24 | 15.70 | 5.49 | 15.73 | 5.48 | 14.45 | 5.65 |
| | | | | | | | | |
| Group | D56 | | D63 | | D70 | | D77 | |
| | % Relatively remained expression level | STDEV | % Relatively remained expression level | STDEV | % Relatively remained expression level | STDEV | % Relatively remained expression level | STDEV |
| PBS | 79.41 | 7.84 | 84.87 | 17.19 | 87.71 | 13.88 | 103.71 | 10.45 |
| RZ002099 | 34.34 | 26.33 | / | / | / | / | / | / |
| RZ002101 | 25.18 | 3.66 | / | / | / | / | / | / |
| RZ002106 | 9.51 | 2.59 | 15.41 | 5.99 | 15.17 | 4.63 | 24.45 | 8.90 |
| RZ002113 | 19.25 | 8.15 | / | / | / | / | / | / |
| | | | | | | | | |

(continued)

| Group | D84 | | D91 | | D98 | | D105 | |
|---|---|---|---|---|---|---|---|---|
| | % Relatively remained expression level | STDEV | % Relatively remained expression level | STDEV | % Relatively remained expression level | STDEV | % Relatively remained expression level | STDEV |
| PBS | 99.52 | 15.57 | 91.57 | 3.00 | 95.00 | 1.80 | 117.08 | 9.46 |
| RZ002099 | / | / | / | / | / | / | / | / |
| RZ002101 | / | / | / | / | / | / | / | / |
| RZ002106 | 38.93 | 16.88 | 34.23 | 13.01 | 36.06 | 14.85 | 36.61 | 13.59 |
| RZ002113 | / | / | / | / | / | / | / | / |

The results of Example 3 showed that RZ002099, RZ002101, RZ002106 and RZ002113 can significantly reduce the C3 protein level in the serum of cynomolgus monkeys after a single administration at 9 mg/kg. Among them, RZ002106 can achieve 90% C3 protein reduction, and still showed 80% protein reduction on D70, and C3 protein levels did not return to pre-dose levels on D105 (FIG. 2 and Table 11).

Example 4 Effect of compounds with (CR01008)×3 conjugated at 3' end of the sense strand on activity of complement pathways in normal cynomolgus monkeys

This example evaluated the activity of complements in serums of cynomolgus monkeys collected at different time points after a single administration of RZ002099, RZ002101, RZ002106, and RZ002113.

Animal grouping, administration and collection of tissue sample

Healthy cynomolgus monkeys weighing 3-5 kg were grouped, with 4 mice in each group, half male and half female. Each test group was given a predetermined dose of drug conjugate, and a PBS control group was set. The administration dose was calculated based on body weight for all animals, and a single subcutaneous injection in the abdomen was given. Each drug conjugate was prepared into a solution form of 9 mg siRNA/mL PBS, and administered at a volume of 1 mL/kg body weight of cynomolgus monkey, i.e., the dosing amount for each drug conjugate was 9 mg siRNA/kg body weight of cynomolgus monkey. The PBS control group was given PBS solution without containing siRNA conjugate at 1 mL/kg body weight of cynomolgus monkey. The serums were collected from cynomolgus monkeys before administration (denoted by pre-dose), on the day of administration (denoted by D0), on day 7 after administration (denoted by D7), on day 14 after administration (denoted by D14), on day 21 after administration (denoted by D21), on day 28 after administration (denoted by D28), on day 35 after administration (denoted by D35), on day 42 after administration (denoted by D42), on day 49 after administration (denoted by D49), on day 56 after administration (denoted by D56), on day 63 after administration (denoted by D63), on day 70 after administration (denoted by D70), on day 77 after administration (denoted by D77), on day 84 after administration (denoted by D84), on day 91 after administration (denoted by D91), on day 98 after administration (denoted by D98), and on day 105 after administration (denoted by D105), and the activities of the complement system alternative pathway (CAP) and the complement system classical pathway (CCP) were detected with the Wieslab® complement system alternative pathway kit (COMPL AP330 RUO, IBL America) and the complement classical pathway kit (COMPL CP310 RUO, IBL America), respectively.

Table 12 Change in CAP activity in serum of cynomolgus monkey after administration of the compounds of this example

| Group | pre-dose | | D7 | | D14 | | D21 | |
|---|---|---|---|---|---|---|---|---|
| | % Relativel y remaine d activity | STDE V | % Relativel y remaine d activity | STDE V | % Relativel y remaine d activity | STDE V | % Relativel y remaine d activity | STDEV |
| PBS | 100.00 | 0.00 | 88.36 | 21.09 | 101.24 | 11.59 | 92.70 | 4.88 |
| RZ00209 9 | 100.00 | 0.00 | 50.06 | 38.25 | 37.05 | 44.75 | 53.62 | 38.25 |

(continued)

| Group | pre-dose | | D7 | | D14 | | D21 | |
|---|---|---|---|---|---|---|---|---|
| | % Relatively remained activity | STDEV | % Relatively remained activity | STDEV | % Relatively remained activity | STDEV | % Relatively remained activity | STDEV |
| RZ002101 | 100.00 | 0.00 | 70.48 | 21.06 | 48.03 | 13.21 | 35.31 | 13.64 |
| RZ002106 | 100.00 | 0.00 | 70.73 | 32.47 | 20.20 | 13.96 | 9.87 | 8.83 |
| RZ002113 | 100.00 | 0.00 | 74.86 | 27.76 | 32.66 | 34.63 | 27.77 | 27.59 |
| | | | | | | | | |
| Group | D28 | | D35 | | D42 | | D49 | |
| | % Relatively remained activity | STDEV | % Relatively remained activity | STDEV | % Relatively remained activity | STDEV | % Relatively remained activity | STDEV |
| PBS | 83.52 | 15.70 | 88.55 | 8.63 | 93.52 | 6.11 | 85.74 | 6.29 |
| RZ002099 | 33.90 | 48.51 | 65.90 | 33.90 | 44.33 | 42.25 | 53.30 | 42.55 |
| RZ002101 | 21.65 | 17.68 | 38.20 | 22.71 | 35.92 | 17.57 | 46.36 | 15.37 |
| RZ002106 | 10.69 | 11.51 | 4.69 | 4.04 | 7.55 | 10.01 | 9.04 | 10.40 |
| RZ002113 | 22.33 | 26.74 | 21.20 | 26.92 | 18.09 | 22.65 | 37.16 | 37.55 |
| | | | | | | | | |
| Group | D56 | | D63 | | D70 | | D77 | |
| | % Relatively remained activity | STDEV | % Relatively remained activity | STDEV | % Relatively remained activity | STDEV | % Relatively remained activity | STDEV |
| PBS | 87.26 | 12.82 | 84.05 | 13.23 | 85.94 | 19.26 | 90.42 | 10.01 |
| RZ002099 | 65.11 | 27.63 | / | / | / | / | / | / |
| RZ002101 | 58.12 | 17.72 | / | / | / | / | / | / |
| RZ002106 | 8.63 | 10.62 | 30.58 | 30.75 | 31.67 | 29.63 | 66.68 | 38.17 |
| RZ002113 | 48.77 | 33.16 | / | / | / | / | / | / |
| | | | | | | | | |
| Group | D84 | | D91 | | D98 | | D105 | |
| | % Relatively remained activity | STDEV | % Relatively remained activity | STDEV | % Relatively remained activity | STDEV | % Relatively remained activity | STDEV |
| PBS | 82.42 | 17.05 | 77.47 | 18.50 | 82.48 | 12.97 | 91.80 | 10.47 |
| RZ002099 | / | / | / | / | / | / | / | / |
| RZ002101 | / | / | / | / | / | / | / | / |
| RZ002106 | 59.91 | 37.62 | 63.49 | 32.68 | 74.24 | 36.30 | 72.33 | 31.73 |
| RZ002113 | / | / | / | / | / | / | / | / |

Table 13 Change in CCP activity in serum of cynomolgus monkey after administration of the compounds of this example

| Group | pre-dose | | D7 | | D21 | | D28 | | D35 | | D42 | |
|---|---|---|---|---|---|---|---|---|---|---|---|---|
| | % Relati vely remain ed activit y | ST DE V | % Relati vely remain ed activit y | STDEV | % Relati vely remain ed activit y | STDEV | % Relati vely remain ed activit y | STDEV | % Relati vely remain ed activit y | STDEV | % Relati vely remain ed activit y | STDEV |
| PBS | 100.00 | 0.0 0 | 104.01 | 11. 16 | 99.79 | 7.3 6 | 104.11 | 16. 23 | 97.08 | 6.6 2 | 91.57 | 5.2 4 |
| RZ0020 99 | 100.00 | 0.0 0 | 113.28 | 16. 70 | 108.76 | 10. 80 | 109.49 | 21. 92 | 103.35 | 3.8 5 | 91.43 | 13. 76 |
| RZ0021 01 | 100.00 | 0.0 0 | 112.86 | 9.2 9 | 93.61 | 6.0 7 | 108.28 | 8.2 3 | 87.59 | 6.2 0 | 86.39 | 2.7 4 |
| RZ0021 06 | 100.00 | 0.0 0 | 102.62 | 8.5 0 | 89.24 | 6.8 8 | 91.60 | 5.9 3 | 81.16 | 3.7 9 | 79.86 | 4.1 7 |
| RZ0021 13 | 100.00 | 0.0 0 | 107.08 | 6.6 6 | 92.17 | 7.2 2 | 104.28 | 9.2 5 | 101.74 | 11. 14 | 98.14 | 3.9 9 |

The results of Example 4 showed that RZ002099, RZ002101, RZ002106 and RZ002113 all can significantly inhibit the complement alternative pathway in the serum of cynomolgus monkeys after a single administration at a dose of 9 mg/kg, but had no significant effect on the complement classical pathway in the serum (FIG. 3, FIG. 4, Table 12 and Table 13).

Example 5 *In vivo* pharmacodynamic evaluation of compounds with (CR01008)×3 conjugated at 3' end of the sense strand in BALB/c-SEAP mice

In this example, the C3 transcript sequence (NM_000064.4) was inserted into the transposon plasmid (Shanghai LoGEN Biotechnology Co., Ltd.), and 1.6 mL of the mixed plasmid injection (25 μg of the transposon plasmid fused with the C3 transcript, and 25 μg of Super PiggyBac Transposase plasmid) was injected within 3 to 5 seconds into mice via tail vein on the basis of hydrokinetics, and a mouse model stably expressing the SEAP (secreted placental alkaline phosphatase) reporter gene was obtained after 2 weeks.

Animal grouping, administration and collection of serum sample

Stably transfected female BALB/c-SEAP mice were grouped according to the SEAP level, with 6 mice in each group. Each test group was given a predetermined dose of drug, and a PBS control group was set. The administration dose was calculated based on body weight for all mice, and the dosing volume was 10 mL/kg body weight of mouse.

RZ002106 was given by a single subcutaneous injection in the abdomen at various doses, wherein RZ002106 was given as solutions of 0.9 mg, 0.3 mg, 0.1 mg and 0.03 mg siRNA/mL PBS, that is, the dosing amount was 9 mg, 3 mg, 1 mg and 0.3 mg siRNA/kg body weight of mouse. The PBS control group was given a PBS solution without containing siRNA conjugate at 10 mL/kg body weight of mouse. The serums were collected from mice of PBS control group and RZ002106 groups (single administration at various doses) before administration (denoted by pre-dose), on the day of administration (denoted by D0), on day 7 after administration (denoted by D7), on day 14 after administration (denoted by D14), on day 21 after administration (denoted by D21), on day 28 after administration (denoted by D28), on day 35 after administration (denoted by D35), on day 42 after administration (denoted by D42), on day 49 after administration (denoted by D49), on day 56 after administration (denoted by D56), on day 63 after administration (denoted by D63) and on day 70 after administration (denoted by D70).

RZ002106 was administered by subcutaneous injection in the abdomen once every two weeks for a total of three administrations (Q2W×3), wherein RZ002106 was prepared into a solution of 0.3 mg siRNA/mL PBS, that is, the dosing amount was 3 mg siRNA/kg body weight of mouse. The serums were collected from mice of PBS control group and RZ002106 group (Q2W×3 administration) before administration (denoted by pre-dose), on the day of administration (denoted by D0), on day 7 after administration (denoted by D7), on day 14 after administration (denoted by D14), on day 21 after administration (denoted by D21), on day 28 after administration (denoted by D28), on day 35 after administration (denoted by D35), on day 42 after administration (denoted by D42), on day 49 after administration (denoted by D49), on day 56 after administration (denoted by D56), on day 63 after administration (denoted by D63), on day 70 after administration (denoted by D70), on day 77 after administration (denoted by D77), on day 84 after administration (denoted by D84), on day 91 after administration (denoted by D91) and on day 98 after administration (denoted by D98).

The SEAP levels in serums of mice of all groups were measured using the Phospha-Light™ SEAP reporter gene assay system (Thermo, T1017), and the results are shown below.

Table 14 Relative expression level of SEAP in the serum of mice given a single administration of siRNA conjugates

| | Relative level (%) | | | | | | | | | | |
|---|---|---|---|---|---|---|---|---|---|---|---|
| Group | pre-dos e | D7 | D14 | D21 | D28 | D35 | D42 | D49 | D56 | D63 | D70 |
| PBS | 100.00 ±0.00 | 100. 00±1 1.25 | 100.0 0 ±14. 22 | 100.0 0 ±17. 73 | 100.0 0 ±17. 12 | 100. 00±1 6.26 | 100.0 0 ±20. 01 | 100.00 ±15.9 5 | 100.0 0 ±16. 26 | 100.0 0 ±20. 98 | 100.0 0 ±17. 56 |
| RZ0021 06 9mg/kg | 100.00 ±0.00 | 2.28 ±0.3 7*** | 0.38± 0.12* ** | 0.49± 0.18* ** | 1.25± 0.38* ** | 4.14 ±1.5 5*** | 10.37 ±2.33 *** | 21.75 ±5.22 *** | 51.35 ±12.9 4** | 72.00 ±17.4 2 | 89.97 ±22.3 1 |
| RZ0021 06 3mg/kg | 100.00 ±0.00 | 2.52 ±0.7 9*** | 0.80± 0.32* ** | 1.64± 0.75* ** | 4.61± 2.08* ** | 13.2 8±4. 87** | 22.35 ±8.60 ** | 45.05 ±13.6 8*** | 64.72 ±25.6 3* | 77.21 ±12.9 7 | 93.62 ±16.8 9 |
| RZ0021 06 1mg/kg | 100.00 ±0.00 | 7.00 ±1.6 2* | 6.43± 1.89* ** | 10.41 ±2.66 *** | 26.45 ±6.26 * | 46.5 0±6. 67* | 51.31 ±10.0 0 | 88.38 ±22.4 0 | 80.52 ±12.9 9 | 106.7 0 ±19. 98 | 113.4 6 ±61. 39 |
| RZ0021 06 0.3mg/k g | 100.00 ±0.00 | 27.5 6±9. 89 | 33.56 ±10.0 6*** | 39.74 ±7.96 *** | 66.11 ±12.3 2 | 81.7 9±8. 85 | 95.29 ±32.8 9 | 107.97 ±26.9 0 | 84.71 ±14.3 0 | 113.4 2 ±22. 95 | 102.9 2 ±27. 82 |

**[00409]** Note: "*" represents P≤0.05, "**" represents P≤0.01, and "***" represents P≤0.001, compared to the PBS group.

Table 15 Relative expression level of SEAP (%) in serum of mice given siRNA conjugates at different frequencies (Mean±SD)

| | Relative level (%) | | |
|---|---|---|---|
| Time point | PBS | RZ002106 9mg/kg | RZ002106 3mg/kg Q2W*3 |
| pre-dose | 100.00±0.00 | 100.00±0.00 | 100.00±0.00 |
| D7 | 100.00±11.25 | 2.28±0.37 | 2.63±0.71**** |
| D14 | 100.00±14.22 | 0.38±0.12*** | 0.85±0.15**** |
| D21 | 100.00±17.73 | 0.49±0.18*** | 0.30±0.05**** |
| D28 | 100.00±17.12 | 1.25±0.38**** | 0.42±0.10 |
| D35 | 100.00±16.26 | 4.14±1.55**** | 0.29±0.08**** |
| D42 | 100.00±20.01 | 10.37±2.33**** | 0.38+0.11**** |
| D49 | 100.00±15.95 | 21.75±5.22*** | 0.95±0.27**** |
| D56 | 100.00±16.26 | 51.35±12.94** | 2.25±0.78**** |
| D63 | 100.00±20.98 | 72.00±17.42 | 6.07±2.19** |
| D70 | 100.00±17.56 | 89.97±22.31 | 21.92±5.45**** |
| D77 | 100.00±25.35 | / | 40.11±7.34** |
| D84 | 100.00±19.45 | / | 64.93±18.08** |
| D91 | 100.00±14.97 | / | 84.96±30.48 |
| D98 | 100.00±27.70 | / | 83.16±22.76 |

The results of Example 5 showed that the inhibitory effect of RZ002106 on SEAP was dose-dependent, and the duration of the inhibition on SEAP was prolonged as the single administration dose increased. Compared to the PBS control group, the SEAP expression in serum of mice was significantly reduced (D7 to D56) after single administration of RZ002106 at 9 mg/kg and 3 mg/kg (P<0.01), and the maximum inhibitory effect was reached on D14, with relative expression levels of 0.38% and 0.80%, respectively. A single administration of RZ002106 at 1 mg/kg significantly reduced the SEAP expression in serum of mice from D7 to D35 (P<0.05), and the maximum inhibitory effect was reached on D14, with a relative expression level of 6.43%. A single administration of RZ002106 at 0.3 mg/kg obviously reduced the SEAP expression in serum of mice on D7 and D21, with significant differences on D14 and D21 (P<0.001), and the maximum inhibitory effect was reached on D7, with a relative expression level of 27.56%. A single administration of RZ002106 at 3 mg/kg significantly reduced the SEAP expression in serum of mice from D7 to D21 (P<0.001), and the maximum inhibitory effect was reached on D14, with a relative expression level of 10.55% (FIG. 5 and Table 14).

The results of Example 1 showed that the administration of RZ002106 Q2W×3 at 3 mg/kg (3 mg/kg, once administration every 2 weeks for a total of 3 administrations) significantly reduced SEAP expression in serum of mice (P<0.0001) from D7 to D84 compared to the PBS control group, and the maximal reduction of SEAP was reached on D35, with a relative expression level of 0.29%. An inhibitory effect >90% was observed on D63. Under the condition of a total dose of 9 mg/kg, the RZ002106 3 mg/kg Q2W×3 multiple-administration group exhibited a better inhibitory activity and longer duration compared to the 9 mg/kg single administration group (FIG. 6 and Table 15).

Example 6 *In vivo* pharmacodynamic evaluation of compounds with (CR01008)×3 conjugated at 3' end of the sense strand in transgenic B-hC3 mice

This example measured the C3 protein expression in serums of B-hC3 mice at different time points after administration of RZ002106, a CR01008 carrier conjugate, by enzyme-linked immunosorbent assay (ELISA).

Animal grouping, administration and collection of serum sample

B-hC3 mice (purchased from Biocytogen Pharmaceuticals (Beijing) Co., Ltd.) were grouped according to the C3 protein level in the serum of mice, with 8 mice in each group. Each test group was given a predetermined dose of drug, and a PBS control group was set. The administration dose was calculated based on body weight for all mice, and the dosing volume was 10 mL/kg body weight of mouse.

RZ002106 was given by a single subcutaneous injection in the abdomen at various doses, wherein RZ002106 was given as solutions of 0.9 mg, 0.3 mg and 0.1 mg siRNA/mL PBS, that is, the dosing amount was 9 mg, 3 mg and 1 mg siRNA/kg body weight of mouse. The PBS control group was given a PBS solution without containing siRNA conjugate at 10 mL/kg body weight of mouse. A small amount of liver tissue was collected from 4 mice of each group (PBS group and RZ002106 groups (single administration at various doses)) by surgery on the day of administration (denoted by D0) and on day 14 after administration (denoted by D14), and the liver tissue was stored in RNA later. For each mouse, the liver tissue sample was taken out of the RNA later, and homogenized in an automatic tissue homogenizer-Tissuelyser II for 60 seconds. According to the standard protocol for total RNA extraction, the total RNA was extracted using an automatic nucleic acid extractor (purchased from Zhejiang Hanwei Technology Co., Ltd.) and a nucleic acid extraction kit (purchased from Zhejiang Hanwei Technology Co., Ltd., GO-MNTR-100). 1 μg of the total RNA was taken, 20 μL of reverse transcription system was prepared using a reverse transcription kit (Thermo Fisher Scientific, RevertAid First Strand cDNA Synthesis Kit, K1622) and Oligo (dT)18 primer for reverse transcription according the instructions of the reverse transcription kit, and a reverse transcription reaction was carried out. After the reaction was completed, 60 μL of RNase-Free water was added to the reverse transcription system to obtain a cDNA solution. Next, the mRNA expression of the target gene in animals was measured in a fluorescence quantitative PCR instrument (Bio-Rad, CFX Opus 384) using a real-time fluorescence quantitative PCR kit (Thermo Fisher Scientific, TaqMan Fast Advanced Master Mix, 4444557). In the real-time fluorescence quantitative PCR assay, the GAPDH gene was used as the internal reference gene, and primers for the target gene and the internal reference gene were used to detect the target gene and the internal reference gene respectively. Sequences of primers used are shown in Table 16.

Table 16 Primer sequence

| Gene | | Primer | Primer sequence | Fluorescent group |
|---|---|---|---|---|
| Target gene | C3 | Upstream primer | 5'-AGTCTCCTGCTTTAGTGATGC-3' (SEQ ID NO: 23) | / |
| | | Downstream primer | 5'-GCCTTTGTTCTCATCTCGCT-3' (SEQ ID NO: 24) | / |
| | | Probe primer | 5'-CTGTTGACCTGCTCCTCGCAAATAT-3' (SEQ ID NO: 25) | 5'6-FAM; 3'MGB |
| Internal reference gene | GAPDH | Upstream primer | 5'-CCTTCCGTGTTCCTACCC-3' (SEQ ID NO:26) | / |
| | | Downstream primer | 5'-GAGACAACCTGGTCCTCA-3' (SEQ ID NO: 27) | / |
| | | Probe primer | 5'-CCGCCTGGAGAAACCTGC-3' (SEQ ID NO: 28) | 5'6-FAM; 3'MGB |

A 10 μL real-time PCR reaction system per PCR reaction well was prepared according to the protocol documented in the instructions of the real-time fluorescence quantitative PCR kit, and each reaction system consisted of 4 μL of cDNA solution obtained by the reverse transcription reaction described above, 5 μL of TaqMan™ Fast Advanced Master Mix (2×), 0.15 μL of 10 μM upstream primer, 0.15 μL of 10 μM downstream primer (see Table 16 for primer information), 0.15 μL of 10 μM probe primer and 0.55 μL of RNase-Free $H_2O$. The prepared reaction system was placed in a real-time fluorescence quantitative PCR instrument (Bio-Rad, CFX Opus 384), and amplified by two-step method. The amplification procedure was 2 min at 50°C, predenaturation at 95°C for 20 seconds, denaturation at 95°C for 3 seconds, annealing at 60°C and extension for 30 seconds. The above denaturation, annealing and extension processes were repeated for 40 cycles. In this real-time fluorescence quantitative PCR assay, the mRNA expression level of the target gene in each test group and the inhibition rate was relatively quantified using the ΔΔCt method as described in the examples.

The serums were collected from the remaining mice of PBS group and RZ002106 groups (single administration at various doses) before administration (denoted by pre-dose), on the day of administration (denoted by D0), on day 7 after administration (denoted by D7), on day 14 after administration (denoted by D14), on day 21 after administration (denoted by D21), on day 28 after administration (denoted by D28), on day 35 after administration (denoted by D35), on day 42 after administration (denoted by D42), on day 49 after administration (denoted by D49), on day 56 after administration (denoted by D56), on day 63 after administration (denoted by D63), on day 70 after administration (denoted by D70), on day 77 after administration (denoted by D77), on day 84 after administration (denoted by D84), on day 91 after administration (denoted by D91) and on day 98 after administration (denoted by D98), and the C3 protein expression level was measured with the

human C3 protein ELISA kit (Hycult, HK366).

RZ002106 was given by a single subcutaneous injection in the abdomen either once every two weeks for a total of three administrations (Q2W×3) or once every four weeks for a total of three administrations (Q4W×3), wherein the drug conjugate was given as a solution of 0.3 mg siRNA/mL PBS, , that is, the dosing amount was 3 mg siRNA/kg body weight of mouse. The serums were collected from mice of PBS group, RZ002106 Q2W×3 administration group and RZ002106 Q4W×3 administration group before administration (denoted by pre-dose), on the day of administration (denoted by D0), on day 7 after administration (denoted by D7), on day 14 after administration (denoted by D14), on day 21 after administration (denoted by D21), on day 28 after administration (denoted by D28), on day 35 after administration (denoted by D35), on day 42 after administration (denoted by D42), on day 49 after administration (denoted by D49), on day 56 after administration (denoted by D56), on day 63 after administration (denoted by D63), on day 70 after administration (denoted by D70), on day 77 after administration (denoted by D77), on day 84 after administration (denoted by D84), on day 91 after administration (denoted by D91), on day 98 after administration (denoted by D98), on day 105 after administration (denoted by D105), on day 112 after administration (denoted by D112), on day 119 after administration (denoted by D119), on day 126 after administration (denoted by D126), on day 133 after administration (denoted by D133) and on day 140 after administration (denoted by D140).

The experimental results are shown below.

Table 17 Relative expression level (%) in liver of B-hC3 mice (Mean±SD)

| Group | Relative level% |
|---|---|
| PBS | 100.00±21.24 |
| RZ002106 9 mg/kg | 12.95±3.16** |
| RZ002106 3 mg/kg | 17.22±5.58** |
| RZ002106 1 mg/kg | 34.30±14.92** |
| Note: "**" represents P≤0.01 compared to the PBS group. | |

Table 18 Relative expression level (%) of C3 protein in the serum of B-hC3 mice given a single administration of siRNA conjugate (Mean±SD)

| Time point | Relative level (%) | | | |
|---|---|---|---|---|
| | PBS | RZ002106 9mg/kg | RZ002106 3mg/kg | RZ002106 1mg/kg |
| pre-dose | 100.00±7.52 | 95.69±9.08 | 95.55±7.19 | 101.74±8.92 |
| D7 | 100.00±8.75 | 37.23±3.66*** | 41.92±3.80*** | 52.57±5.31*** |
| D14 | 100.00±6.95 | 39.18±3.91*** | 42.57±4.00*** | 56.00±5.83*** |
| D21 | 100.00±8.12 | 36.63±3.63*** | 44.34±6.20*** | 54.13±4.27*** |
| D28 | 100.00±5.97 | 41.54±2.86*** | 46.76±5.08*** | 60.15±5.19*** |
| D35 | 100.00±10.82 | 43.16±4.03*** | 53.66±7.50*** | 69.69±9.44*** |
| D42 | 100.00±6.52 | 45.76±5.10*** | 56.58±7.52** | 73.89±11.63 |
| D49 | 100.00±8.66 | 51.62±5.88*** | 61.25±8.49** | 88.52±12.76 |
| D56 | 100.00±13.17 | 51.94±6.00*** | 63.75±9.79*** | 91.41±16.90 |
| D63 | 100.00±11.36 | 57.67±8.38*** | 70.01±12.96* | 85.71±17.03 |
| D70 | 100.00±10.93 | 64.74±9.15*** | 74.77±12.22* | 87.46±17.71 |
| D77 | 100.00±9.47 | 67.80±9.59*** | 75.61±10.36** | 105.29±20.70 |
| D84 | 100.00±13.56 | 76.35±13.09* | 80.73±9.65 | 115.97±14.44 |
| D91 | 100.00±9.48 | 83.81±13.76 | 89.27±17.38 | 100.33±19.95 |

(continued)

| Time point | Relative level (%) | | | |
|---|---|---|---|---|
| | PBS | RZ002106 9mg/kg | RZ002106 3mg/kg | RZ002106 1mg/kg |
| D98 | 100.00±13.10 | 88.16±13.67 | 86.26±15.90 | 110.07±24.14 |
| Note: "*" represents P≤0.05, "**" represents P≤0.01, and "***" represents P≤0.001, compared to the PBS group. | | | | |

Table 19 Relative expression level (%) of C3 protein in serum of B-hC3 mice given siRNA conjugate at different frequencies (Mean±SD)

| Time point | Relative C3 protein level (%) | | | |
|---|---|---|---|---|
| | PBS | RZ002106 9mg/kg | RZ002106 3mg/kg Q2W×3 | RZ002106 1mg/kg Q4W×3 |
| pre-dose | 100.00±7.52 | 95.69±9.08 | 103.65±10.16 | 106.59±2.31 |
| D7 | 100.00±8.75 | 37.23±3.66*** | 41.20±3.41*** | 40.02±5.48*** |
| D14 | 100.00±6.95 | 39.18±3.91*** | 43.74±3.95*** | 41.80±3.94*** |
| D21 | 100.00±8.12 | 36.63±3.63*** | 33.47±2.79*** | 37.66±4.04*** |
| D28 | 100.00±5.97 | 41.54±2.86*** | 36.35±2.60*** | 42.29±5.34*** |
| D35 | 100.00±10.82 | 43.16±4.03*** | 38.85±3.74*** | 38.58±3.65*** |
| D42 | 100.00±6.52 | 45.76±5.10*** | 38.65±5.14** | 39.93±3.56** |
| D49 | 100.00±8.66 | 51.62±5.88 | 40.46±6.43** | 42.36±5.45** |
| D56 | 100.00±13.17 | 51.94±6.00*** | 40.94±7.57** | 42.07±6.39** |
| D63 | 100.00±11.36 | 57.67±8.38*** | 42.41±8.20* | 38.23±4.75*** |
| D70 | 100.00±10.93 | 64.74±9.15*** | 45.47±8.25* | 38.71±5.55 |
| D77 | 100.00±9.47 | 67.80±9.59 | 53.98±10.11*** | 43.59±5.91*** |
| D84 | 100.00±13.56 | 76.35±13.09 | 55.66±10.45* | 42.58±4.85*** |
| D91 | 100.00±9.48 | 83.81±13.76 | 56.24±12.82* | 43.41±3.25 |
| D98 | 100.00±13.10 | 88.16±13.67 | 54.45±13.72*** | 44.11±5.30 |
| D105 | 100.00±9.37 | / | 56.67±11.61** | 44.59±2.58*** |
| D112 | 100.00±12.02 | / | 58.87±16.79** | 42.21±4.40 |
| D119 | 100.00±10.75 | / | 65.30±17.10 | 50.21±3.07 |
| D126 | 100.00±6.33 | / | 65.45±17.67 | 50.06±4.25** |
| D133 | 100.00±18.92 | / | 79.99±22.63 | 57.78±4.42 |
| D140 | 100.00±10.33 | / | 82.20±24.37 | 60.69±7.83** |
| Note: "/" indicates no value, Q2W×3 indicates once administration every 2 weeks for a total of 3 administrations, and Q4W×3 indicates once administration every 4 weeks for a total of 3 administrations. "*" represents P≤0.05, "**" represents P≤0.01, and "***" represents P≤0.001, compared to the PBS group. | | | | |

The results of Example 6 showed that compared to the PBS control group, a single administration of RZ002106 at 9 mg/kg, 3 mg/kg, and 1 mg/kg significantly inhibited the C3 mRNA expression in liver tissues on D14 (P<0.01), with relative expression levels of 12.95%, 17.22%, and 34.30%, respectively, and this inhibition was dose-dependent (FIG. 7 and Table 17). Compared to the PBS control group, a single administration of RZ002106 at 9 mg/kg, 3 mg/kg, and 1 mg/kg can significantly reduce C3 protein expression in serum of mice (P<0.05), and the maximum inhibitory effect was reached on D7, with relative levels of 37.23%, 41.92%, and 52.57%, respectively. The inhibitory effect of RZ002106 on C3 protein was dose-dependent until the experimental endpoint (D98) (FIG. 8 and Table 18).

The C3 protein expression in serum of mice was significantly reduced after administration of RZ002106 (3 mg/kg, Q2W×3) from D7 to D119 (P<0.05), the maximum inhibitory effect was reached on D21, with a relative expression level of 33.47%, and the inhibitory effect on D70 was still greater than 50%. The C3 protein expression in serum of mice was

significantly reduced after administration of RZ002106 (3 mg/kg, Q2W×3) from D7 to D140 (P<0.05), the maximum inhibitory effect was reached on D21 with a relative expression level of 37.66%, and the inhibitory effect on C3 protein was still greater than 50% on D112, and was 39.31% on D149 (FIG. 9 and Table 19). Under the condition of a total dose of 9 mg/kg, the RZ002106 3 mg/kg Q4W×3 administration group exhibited a longer duration of inhibitory effect on serum C3 protein of model mice compared to the RZ002106 3 mg/kg Q2W×3 administration group and the 9 mg/kg single administration group.

Example 7 Pharmacodynamic study of compounds with (CR01008)×3 conjugated at 3' end of the sense strand in cynomolgus monkey nephropathy model

In this example, a cynomolgus monkey nephropathy model based on the combined induction of naive cynomolgus monkeys by BSA, CCl4 and LPS was obtained from Taipumed (Suzhou) Pharmaceutical Technology Co., Ltd. Blood creatinine (Crea)\24-h urine protein (uTP) and urine creatinine (Ucrea) were measured using a blood biochemistry analyzer, and 24-h urine protein/creatinine ratio (uPCR) and glomerular filtration rate (eGFR) were calculated.

Animal grouping, administration and collection of serum and tissue sample

According to the urinary parameters during the modeling period, 20 model animals were randomly divided into 4 administration groups, with 5 animals in each group. Each test group was given a predetermined dose of the drug, and a 0.9% saline control group was set. The administration dose was calculated based on body weight for all monkeys, and the dosing volume was 1 mL/kg body weight of monkey. RZ002106 was given by a single subcutaneous injection at various doses, wherein the drug was prepared into solutions of 9 mg, 3 mg and 1 mg siRNA/mL 0.9% saline, that is, the dosing amount was 9 mg, 3 mg and 1 mg siRNA/kg body weight of cynomolgus monkey. The 0.9% saline control group was given a 0.9% saline solution without containing siRNA conjugate at 1 mL/kg body weight of cynomolgus monkey.

Serum was collected from cynomolgus monkeys in all groups before administration (denoted by pre-dose), on the day of administration (denoted by D0), and at 2 weeks, 4 weeks, and 6 weeks after administration, for subsequent serum Crea measurement by blood biochemistry analyzer. Urine was collected for 3 consecutive days at each time point, and 24-hour urine was collected each day. The average of the urine parameters for three days was used as the value for that time point. After each collection, the urine was centrifuged at 4°C and 2000 rpm for 10 min, and the supernatant was aliquoted into 1.5 mL centrifuge tubes (300 μL per tube). The samples were stored in a refrigerator at -80°C for subsequent measurements of uTP and Ucrea, and the uPCR (UPCR=uTP/Ucrea) and eGFR (eGFR=urine creatinine/blood creatinine*1.73/animal body weight^0.667*0.118) were calculated.

The experimental results are shown in the following table:

Table 20 uTP levels in the cynomolgus monkey nephropathy model after administration of siRNA conjugates at various doses (Mean ± SEM)

| Time point | uTP (mg) (Mean±SEM) | | | |
|---|---|---|---|---|
| | 0.9% saline | RZ002106 9mg/kg | RZ002106 3mg/kg | RZ002106 1mg/kg |
| Pre-dose | 75.65±15.58 | 75.47±7.4 | 71.01±7.82 | 83.3±9.15 |
| 2 weeks after administr ation | 83.41±15.4 | 59.74±7.92 | 65.79±5.96 | 83.14±9.07 |
| 4 weeks after administr ation | 94.15±17.52 | 48.56±5.89 | 54.6±8.72 | 75.98±7.05 |
| 6 weeks after administr ation | 98.27±11.52 | 53.19±10.06 | 57.19±6.86 | 78.88±8.16 |

Table 21 UPCR levels in the cynomolgus monkey nephropathy model after administration of siRNA conjugates at various doses (Mean ± SEM)

| Time point | UPCR (mg/g) (Mean±SEM) | | | |
|---|---|---|---|---|
| | 0.9% saline | RZ002106 9mg/kg | RZ002106 3mg/kg | RZ002106 1mg/kg |
| Pre-dose | 453.28±55.57 | 461.86±58.36 | 450.98±78.94 | 463.31±58.07 |
| 2 weeks after administr ation | 489.16±67.99 | 400.24±24.87 | 411.92±66.53 | 471.25±67.9 |
| 4 weeks after administration | 481.73±79.3 | 260.35±18.6 | 337.62±79.88 | 407.67±68.04 |
| 6 weeks after administr ation | 507.66±74.77 | 255.21±21.57 | 334.56±80.68 | 442.24±79.92 |

Table 22 eGFR levels in the cynomolgus monkey nephropathy model after administration of siRNA conjugates at various doses (Mean ± SEM)

| Time point | eGFR (mL/min/1.73m$^2$) (Mean±SEM) | | | |
|---|---|---|---|---|
| | 0.9% saline | RZ002106 9mg/kg | RZ002106 3mg/kg | RZ002106 1mg/kg |
| Pre-dose | 218.94±54.77 | 234.54±57.34 | 205.44±35.65 | 204.58±27.14 |
| 2 weeks after administr ation | 222.91±45.7 | 332.02±74.98 | 265.34±41.17 | 209.24±48.78 |
| 4 weeks after administr ation | 200.84±32.58 | 374.08±71.91 | 272.17±45.51 | 226.38±47.51 |
| 6 weeks after administr ation | 197.86±35.03 | 379.21±72.72 | 276.19±49.5 | 225.29±28.68 |

Table 23 uTP levels in the cynomolgus monkey nephropathy model after administration of siRNA conjugates at various doses (Mean ± SEM)

| Time point | Crea (μmol/L) (Mean±SEM) | | | |
|---|---|---|---|---|
| | 0.9% saline | RZ002106 9mg/kg | RZ002106 3mg/kg | RZ002106 1mg/kg |
| Pre-dose | 190.6±17.06 | 177.64±11.28 | 180.93±16.65 | 177.38±8.18 |
| 2 weeks after administr ation | 197.73±17.65 | 162.59±13.91 | 167.17±18.96 | 174.14±9.59 |
| 4 weeks after administr ation | 199.64±16.22 | 122.46±8.32 | 139.17±17.89 | 159.59±8.19 |
| 6 weeks after administr ation | 200.22±13.74 | 119.31±8.2 | 142.16±16.08 | 161.01±6.11 |

The results of Example 7 showed that compared to the 0.9% saline control group, a single administration of RZ002106 at 9 mg/kg, 3 mg/kg and 1 mg/kg significantly reduced urine uTP and UPCR levels at 2 weeks, 4 weeks, and 6 weeks after administration in a dose-dependent manner (FIG. 10 and Table 20, FIG. 11 and Table 21). Compared to the 0.9% saline control group, a single administration of RZ002106 at 9 mg/kg, 3 mg/kg and 1 mg/kg significantly increased eGFR at 2 weeks, 4 weeks, and 6 weeks after administration in a dose-dependent manner (FIG. 12 and Table 22). Compared to the 0.9% saline control group, a single administration of RZ002106 at 9 mg/kg, 3 mg/kg and 1 mg/kg significantly reduced serum Crea at 2 weeks, 4 weeks, and 6 weeks after administration in a dose-dependent manner (FIG. 13 and Table 23).

Example 8 *In vivo* toxicological evaluation of compounds with (CR01008)×3 conjugated at 3' end of the sense strand in SD rats

In this example, a subcutaneous injection at 300 mg/kg, 100 mg/kg and 30 mg/kg was given once every 2 weeks for continuous 4 weeks, and the toxicity and metabolic profile of the RZ002106 conjugate in SD rats was evaluated.

Animal grouping, administration, and collection of tissue sample:

6- to 8-week-old SD rats (purchased from Zhejiang Charles River Experimental Animal Technology Co., Ltd.) were randomly grouped based on body weight. Each test group was given a predetermined dose of drug conjugate, and a PBS control group was set. The administration dose was calculated based on body weight for all rats, and a single subcutaneous injection in the abdomen was given. The RZ002106 conjugate was prepared into solutions of 60 mg, 20 mg and 6 mg siRNA/mL PBS, which were then administered at a dosing volume of 5 mL/kg body weight of rat, i.e., the dosing amount of drug conjugate was 300 mg, 100 mg and 30 mg siRNA/kg body weight of rat. The PBS control group was given an equal volume of PBS solution without containing siRNA conjugate. The rats were given a total of 3 administrations, and observed for 4 weeks. The day of administration was denoted as D0, and on the final day of administration period (denoted as D29), all mice were subjected to gross dissection and histopathological examination of tissues.

No death or near-death conditions were observed in animals of each group during the experimental period. Clinical observations and gross dissection of animals in each dose group of the tested compound did not show any abnormal changes associated with the test compound. Histopathological examination revealed light to mild basophilic granules in the kidneys and light to mild vacuolar degeneration in cells surrounding the hepatic central vein/centrilobular zone. The no-observed-adverse-effect level (NOAEL) was 300 mg/kg (FIG. 14).

Example 9 *In vivo* toxicological evaluation of compounds with (CR01008)×3 conjugated at 3' end of the sense strand in cynomolgus monkeys

In this example, a subcutaneous injection at 300 mg/kg, 100 mg/kg and 30 mg/kg was given once every 2 weeks for continuous 4 weeks, and the toxicity and metabolic profile of the RZ002106 conjugate in cynomolgus monkeys was evaluated.

Animal grouping, administration, and collection of tissue sample:

6 non-naive cynomolgus monkeys (3 female and 3 male monkeys) were randomly divided into 3 groups based on body weight, with 1 female and 1 male monkeys in each group. Each test group was given a predetermined dose of drug conjugate. The administration dose was calculated based on body weight for all cynomolgus monkeys, and a single subcutaneous injection in the abdomen was given. The RZ002106 conjugate was administered as solutions of 150 mg, 50 mg and 15 mg siRNA/mL PBS, at a dosing volume of 2 mL/kg body weight of cynomolgus monkey, i.e., the dosing amount of drug conjugate was 300 mg, 100 mg and 30 mg siRNA/kg body weight of cynomolgus monkey. The cynomolgus monkeys were given a total of 3 administrations, and observed for 4 weeks. All animals were euthanized at the end of the administration period (D30) for gross dissection, and major organ tissues were subjected to histopathological examination.

No death or near-death conditions were observed in animals of each group during the experimental period. Clinical observations and gross anatomical observation of animals in each dose group of the tested compound did not show any abnormal changes associated with the test compound. Histopathological examination of animals in each dose group revealed macrophages vacuolization in the medulla of mesenteric lymph nodes/ inguinal lymph nodes/ submandibular lymph nodes, and mild basophilic granules in Kupffer cells within the liver sinusoids. The no-observed-adverse-effect level (NOAEL) was 300 mg/kg (FIG. 15).

Example 10 *In vivo* pharmacodynamic evaluation of compounds with (CR01008)×3 conjugated at 3' end of the sense strand in CFA-hIgA mice

In this example, a CFA-hIgA mice nephropathy model was constructed by the Peking University First Hospital. The inhibitory effect of RZM02009 on C3 mRNA in the liver of the CFA-IgA mice model was detected by QPCR method, the expression level of C3 protein and the activity of complement alternative pathway (CAP) in the serum of mice were measured by ELISA at different time points.

| Group | Sense strand (5'-3') | Antisense strand (5'-3') |
|---|---|---|
| RZM02009 | AmsGmsAmAmGmAmAfGfAfUfAmUmUmAmUmCmUmCmUm_(CR01008×3) | AmsGfsAmGmAmUfAmAmUmAmUmCfUmUfC(moe)UfUmCmUmsGmsGm |

Animal grouping, administration and collection of serum and tissue sample

24 model animals were randomly divided into 4 administration groups, with 6 animals in each group. The administration dose was calculated based on body weight for all mice, and the dosing volume was 10 mL/kg body weight of mouse.

RZM02009 was given by a single subcutaneous injection in the abdomen at various doses, wherein the drug conjugate was administered as solutions of 9 mg, 3 mg and 1 mg siRNA/mL 0.9% saline, that is, the dosing amount of the drug conjugate was 9 mg, 3 mg and 1 mg siRNA/kg body weight of mouse. The 0.9% saline control group was given a 0.9% saline solution without containing siRNA conjugate at 1 mL/kg body weight of mouse. Serum was collected from all mice before administration (denoted by pre-dose), on the day of administration (denoted by D0), on day 7 after administration (denoted by D7), on day 14 after administration (denoted by D14), on day 21 after administration (denoted by D21) and on day 28 after administration (denoted by D28). The C3 protein expression level in serum was measured with the mouse complement C3 ELISA kit (Abcam, 157711). The activity of complement alternative pathway (CAP) in serum of mice on D28 was detected with a kit (Hycult Biotech, HIT422).

The experimental results are shown in the following table:

Table 24 Inhibitory activity (%) of siRNA conjugates on C3 mRNA in the liver of CFA-hIgA mice after administration

| Group | Relatively remained C3 mRNA expression level (%, Mean±SD) |
|---|---|
| 0.9% saline | 100.00±22.13 |
| RZM02009 9 mg/kg | 1.69±0.62*** |
| RZM02009 3 mg/kg | 3.40±0.94*** |
| RZM02009 1 mg/kg | 10.61±4.31 |
| Note: "***" represents P≤0.001 compared to 0.9% saline control group. | |

Table 25 Expression levels of C3 protein (%, Mean ± SD) in the livers of CFA-hIgA mice after administration of siRNA conjugates

| Group | Pre-dose | D7 | D14 | D21 | D28 |
|---|---|---|---|---|---|
| 0.9% saline | 100.00±9.28 | 100.00±18.04 | 100.00±12.20 | 100.00±14.55 | 100.00±15.41 |
| RZM02009 9 mg/kg | 89.49±11.08 | 17.35±2.21*** | 17.00±3.46*** | 19.90±3.88*** | 21.5 1±3.90*** |
| RZM02009 3 mg/kg | 97.24±9.36 | 20.67±2.32*** | 21.98±2.12*** | 23.18±3.34*** | 24.85±2.73*** |
| RZM02009 1 mg/kg | 82.60±21.35 | 20.16±4.40*** | 22.24±7.38*** | 24.80±6.06*** | 31.83±9.94*** |
| Note: "*" represents P≤0.05, "**" represents P≤0.01 and "***" represents P≤0.001, compared to 0.9% saline control group. | | | | | |

Table 26 CAP activity level (%) in serum of CFA-hIgA mice after administration of siRNA conjugates

| Group | Relative activity of CAP (%, Mean±SD) |
|---|---|
| 0.9% saline | 100.00±8.43 |
| RZM02009 9 mg/kg | 14.21±4.98*** |
| RZM02009 3 mg/kg | 22.43±10.34*** |
| RZM02009 1 mg/kg | 53.66±19.97* |
| Note: "*" represents P≤0.05 and "***" represents P≤0.0001, compared to 0.9% saline control group. | |

The results of Example 10 showed that a single administration of RZM02009 at 9 mg/kg, 3 mg/kg, and 1 mg/kg significantly reduced C3 mRNA levels in the livers of the model mice on day 28 after administration compared to the 0.9% saline control group, with relative expression levels of 1.69%, 3.40% and 10.61%, respectively, and this reduction effect was dose-dependent (FIG. 16 and Table 24). Compared to the 0.9% saline control group, a single administration of RZM02009 at 9 mg/kg, 3 mg/kg, and 1 mg/kg significantly reduced C3 protein expression in serum of mice ($P < 0.05$), and the maximum inhibitory effect was reached on D7, with relative levels of 17.35%, 20.67%, and 20.16%, respectively. Until the experimental endpoint (D28), the inhibitory effect of RZM02009 on C3 protein was dose-dependent (FIG. 17 and Table 25).

The results of Example 10 showed that compared to the 0.9% saline control group, a single administration of RZM02009 at 9 mg/kg, 3 mg/kg, and 1 mg/kg significantly inhibited the CAP activity in serum of the model mice after administration on D28, with relative expression levels of 14.21%, 22.43%, and 53.66%, respectively, and this inhibition was dose-dependent (FIG. 18 and Table 26).

The above specific embodiments are merely schematic illustrations of the contents of the present disclosure and do not represent limitations of the contents of the present disclosure. For those of ordinary skills in the art, without departing from the spirit and substance of the present disclosure, various variations and modifications may be made, which are also regarded as the scope of protection of the present disclosure.

## Claims

1. A compound having a structure represented by formula (I), or a pharmaceutically acceptable salt thereof:

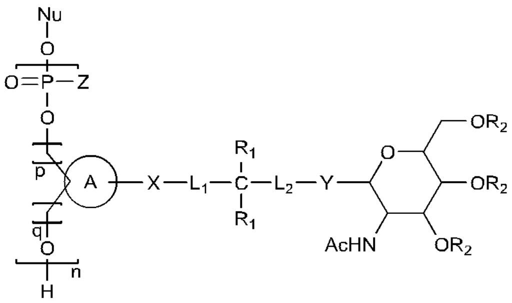

formula (I)

wherein, in the structure,

each A is independently an unsubstituted or substituted 4- to 10-membered aliphatic ring,
n is selected from the group consisting of 1, 2, 3 and 4,
each Z is independently selected from the group consisting of hydroxyl and mercapto,
each p is independently selected from the group consisting of 1, 2 and 3,
each q is independently selected from the group consisting of 1, 2 and 3,
each X is independently selected from the group consisting of NH, O and S,
each $L_1$ is independently selected from the group consisting of

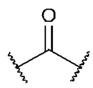, 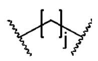

and

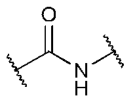,

wherein j is selected from the group consisting of 1, 2, 3, 4, 5, 6, 7, 8, 9 and 10,
each $R_1$ is independently selected from the group consisting of H, $C_1$-$C_6$ alkyl, $C_1$-$C_6$ haloalkyl and $C_1$-$C_6$ alkoxy,
each $L_2$ is independently selected from the group consisting of $C_1$-$C_{30}$ alkylidene and

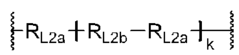,

wherein each $R_{L2a}$ is independently $C_1$-$C_{10}$ alkylidene, each $R_{L2b}$ is independently selected from the group consisting of O, S, NH and -NH-C(O)-, and k is selected from the group consisting of 1, 2, 3, 4, 5, 6, 7, 8, 9 and 10,
each Y is independently selected from the group consisting of NH, O and S, and
each $R_2$ is independently selected from the group consisting of H,

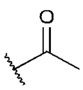, 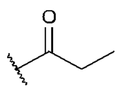,

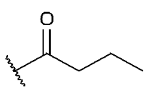, 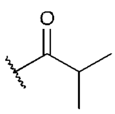, 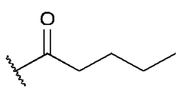, 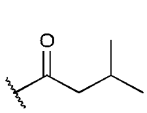, 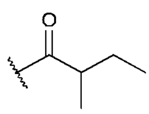, 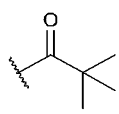, 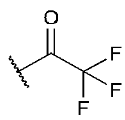

and

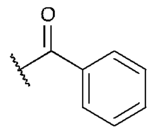

,

wherein Nu represents a double-stranded oligonucleotide or a pharmaceutically acceptable salt thereof for reducing the expression of complement 3 (C3), the double-stranded oligonucleotide comprises a sense strand and an antisense strand, wherein the sense strand and the antisense strand form a double-stranded region, the antisense strand comprises a complementary region that has complementarity to a target sequence of C3 mRNA, and the complementary region is 17 to 35 contiguous nucleotides in length.

2. The compound according to claim 1, wherein the compound has a structure represented by formula (II), or the pharmaceutically acceptable salt thereof,

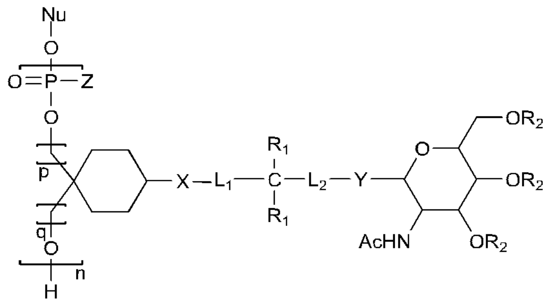

formula (II)

in formula (II), p, q, n, Z, X, Y, $L_1$, $L_2$ and $R_1$ are as defined in claim 1, and $R_2$ is H.

3. The compound according to any one of claims 1 to 2, wherein the compound has a structure represented by formula (III), or the pharmaceutically acceptable salt thereof,

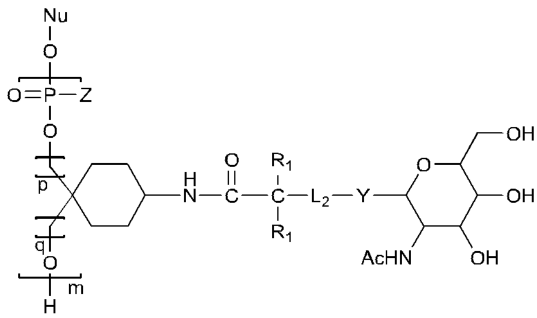

formula (III)

in formula (III), m is selected from the group consisting of 1, 2, 3 and 4, and the other substituents are as defined in claim 1;
optionally, the compound has a structure represented by formula (IV), or the pharmaceutically acceptable salt thereof,

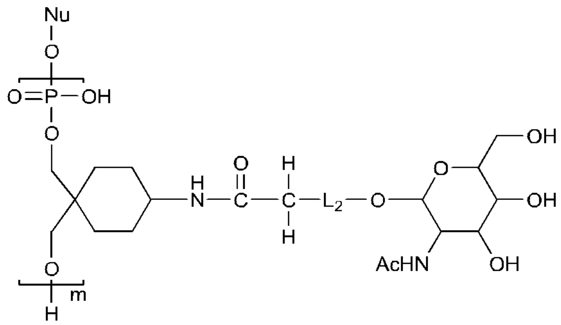

formula (IV),

in formula (IV), Nu is as defined in claim 1, m is selected from the group consisting of 1, 2, 3 and 4, and $L_2$ is dependently selected from the group consisting of

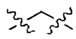, 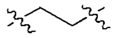,

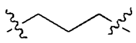, 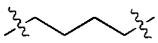, 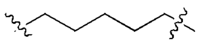, 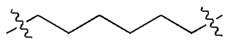, 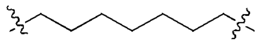,

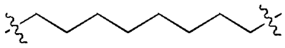, 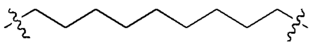, 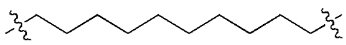,

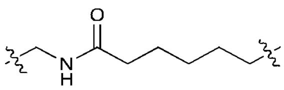, 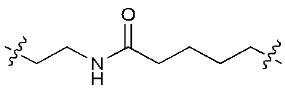, 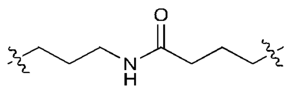,

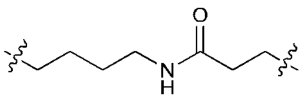 and 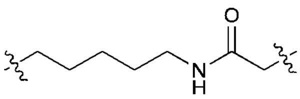.

4. The compound according to any one of claims 1 to 3, wherein the compound has a structure selected from the group consisting of

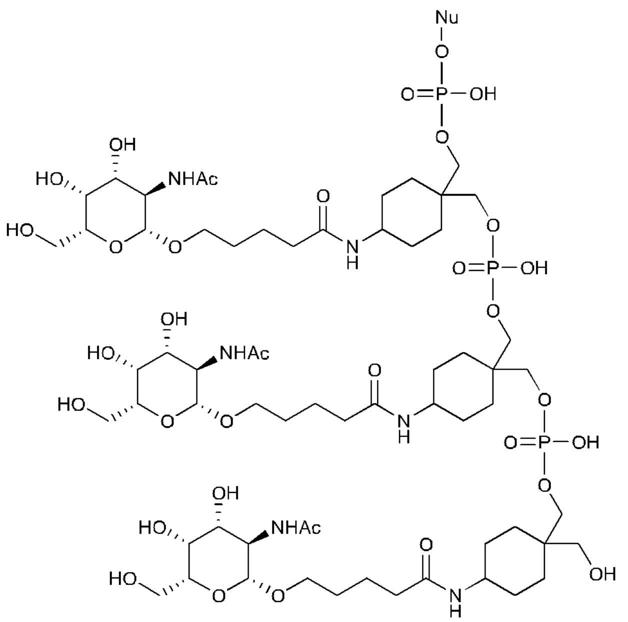

and

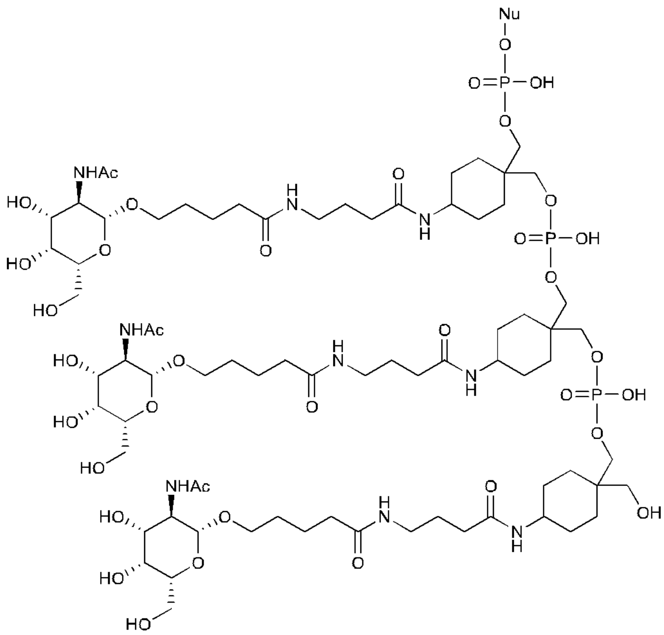

, or the pharmaceutically acceptable salt thereof,
wherein Nu is as defined in claim 1, and in the above oligonucleotide-conjugated compound, the sense strand of Nu is connected at 3' end to a phosphate group.

**5.** The compound according to any one of claims 1 to 4, wherein

the antisense strand of the double-stranded oligonucleotide represented by Nu comprises a nucleotide sequence of or differing by 1 or 2 nucleotides from any one of the sequences set forth in SEQ ID NOs: 2, 4, 6, 8 and 10, and/or, the sense strand of the double-stranded oligonucleotide comprises a nucleotide sequence of or differing by 1 or 2 nucleotides from any one of the sequences set forth in SEQ ID NOs: 1, 3, 5, 7 and 9;
optionally, the double-stranded oligonucleotide is one or more selected from the group consisting of:

1) an antisense strand having a nucleotide sequence of or differing by 1 or 2 nucleotides from the nucleotide sequence set forth in SEQ ID NO: 2 and a sense strand having a nucleotide sequence of or differing by 1 or 2 nucleotides from the nucleotide sequence set forth in SEQ ID NO: 1,
2) an antisense strand having a nucleotide sequence of or differing by 1 or 2 nucleotides from the nucleotide sequence set forth in SEQ ID NO: 4 and a sense strand having a nucleotide sequence of or differing by 1 or 2 nucleotides from the nucleotide sequence set forth in SEQ ID NO: 3,
3) an antisense strand having a nucleotide sequence of or differing by 1 or 2 nucleotides from the nucleotide sequence set forth in SEQ ID NO: 6 and a sense strand having a nucleotide sequence of or differing by 1 or 2 nucleotides from the nucleotide sequence set forth in SEQ ID NO: 5,
4) an antisense strand having a nucleotide sequence of or differing by 1 or 2 nucleotides from the nucleotide sequence set forth in SEQ ID NO: 8 and a sense strand having a nucleotide sequence of or differing by 1 or 2 nucleotides from the nucleotide sequence set forth in SEQ ID NO: 7, and
5) an antisense strand having a nucleotide sequence of or differing by 1 or 2 nucleotides from the nucleotide sequence set forth in SEQ ID NO: 10 and a sense strand having a nucleotide sequence of or differing by 1 or 2 nucleotides from the nucleotide sequence set forth in SEQ ID NO: 9.

**6.** The compound according to any one of claims 1 to 5, wherein each nucleotide in the double-stranded oligonucleotide is independently selected from the group consisting of:

2'-fluoro modified nucleotide, 2'-deoxy modified nucleotide, 2'-O-methyl modified nucleotide, 2'-O-$(CH_2)_n$-O-R modified nucleotide, 2'-amino modified nucleotide, abasic nucleotide, and a nucleotide analogue, wherein the nucleotide analogue is one or more selected from the group consisting of PNA, MNA, BNA, LNA, GNA, TNA and

UNA, wherein, n is selected from the group consisting of 1 and 2, R is selected from the group consisting of optionally substituted $C_{1-6}$ alkyl and optionally substituted $C_{1-6}$ alkoxy, when R comprises a substituent, the substituent is selected from the group consisting of halogen, $C_{1-6}$ alkoxy, hydroxyl and amino,
optionally, the 2'-O-$(CH_2)_n$-O-R modified nucleotide is selected from the group consisting of 2'-O-methoxyethyl-modified nucleotide and 2'-O-ethoxymethyl-modified nucleotide, and
optionally, the double-stranded oligonucleotide comprises at least one 2'-O-methoxyethyl modified nucleotide.

**7.** The compound according to any one of claims 1 to 6, wherein the antisense strand comprises at least one 2'-O-methoxyethyl modified nucleotide,

optionally, in the direction from 5' end to 3' end, nucleotides at positions 7 to 10 of the nucleotide sequence of the sense strand of the double-stranded oligonucleotide are 2'-fluoro modified nucleotides, and nucleotides at the other positions in the sense strand are 2'-O-methyl modified nucleotides; nucleotides at positions 2, 6, 14, and 16 of the nucleotide sequence of the antisense strand are 2'-fluoro modified nucleotides, any one of nucleotides at positions 9 to 12 is a 2'-fluoro modified nucleotide, at least one of nucleotides at positions 8 and 15 is a 2'-O-methoxyethyl modified nucleotide, and nucleotides at the other positions in the antisense strand are 2'-O-methyl modified nucleotides;
optionally, in the direction from the 5' end to 3' end, at least one of linkages between the following nucleotides of the sense strand is a phosphorothioate linkage: a linkage between the first nucleotide and the second nucleotide at 5' end of the sense strand, and a linkage between the second nucleotide and the third nucleotide at 5' end of the sense strand; and
optionally, in the direction from the 5' end to 3' end, at least one of linkages between the following nucleotides of the antisense strand is a phosphorothioate linkage: a linkage between the first nucleotide and the second nucleotide at 5' end of the antisense strand, a linkage between the second nucleotide and the third nucleotide at 5' end of the antisense strand, a linkage between the first nucleotide and the second nucleotide at 3' end of the antisense strand, and a linkage between the second nucleotide and the third nucleotide at 3' end of the antisense strand.

**8.** The compound according to any one of claims 1 to 7, wherein each nucleotide in the double-stranded oligonucleotide is a modified nucleotide,

optionally, the double-stranded oligonucleotide is one or more sets selected from the group consisting of set 1, set 2, set 3 and set 4,

| | sense strand (5'-3') | antisense strand (5'-3') |
|---|---|---|
| set 1 | CmsGmsAmAmGmCmUfCfAfUfGmAmAmUmAmUmAmUmUm | AmsAfsUmAmUmAfUmUmCmAmUfGmAmGfC(moe)UfUmCmGmsUmsAm |
| set 2 | CmsGmsAmAmGmCmUfCfAfUfGmAmA(moe)UmAmUmAmUmUm | AmsAfsUmAmUmAfUmUmCmAmUfGmAmGfC(moe)UfUmCm GmsUmsAm |
| set 3 | CmsAmsGmAmGmAmAfAfUfUfCmUmAmCmUmAmCmAmUm | AmsUfsGmUmAmGfUmAmGmAmAfUmUmUfC(moe)UfCmUmGmsUmsAm |
| set 4 | CmsAmsAmCmUmCmAfCfCfUfGmUmAmAmUmAmAmA(moe)Um | AmsUfsUmUmAmUfUmA(moe)CmAmGfGmUmGfAmGfUmUmGmsAmsUm |

optionally, the double-stranded oligonucleotide comprises a sense strand (5'-3') of CmsAmsGmAmGmAmAfAfUfUfCmUmAmCmUmAmCmAmUm and an antisense strand (5'-3') of AmsUfsGmUmAmGfUmAmGmAmAfUmUmUfC(moe)UfCmUmGmsUmsAm.

**9.** The compound according to any one of claims 1 to 8, wherein the compound is any one selected from the compounds shown in Table 6;

optionally, the compound is any one selected from the group consisting of RZ002099, RZ002101, RZ002106 and RZ002113:

| | sense strand (5'-3') | antisense strand (5'-3') |
|---|---|---|
| RZ002099 | CmsGmsAmAmGmCmUfCfAfUfGmAmAmUmAmUmAmUmUm_(CR01008×3) | AmsAfsUmAmUmAfUmUmCmAmUfGmAmGfC(moe)UfUmCmGmsUmsAm |
| RZ002101 | CmsGmsAmAmGmCmUfCfAfUfGmAmA(moe)UmAmUmAmUmUm_(CR01008×3) | AmsAfsUmAmUmAfUmUmCmAmUfGmAmGfC(moe)UfUmCmGmsUmsAm |
| RZ002106 | CmsAmsGmAmGmAmAfAfUfUfCmUmAmCmUmAmCmAmUm_(CR01008×3) | AmsUfsGmUmAmGfUmAmGmAmAfUmUmUfC(moe)UfCmUmGmsUmsAm |
| RZ002113 | CmsAmsAmCmUmCmAfCfCfUfGmUmAmAmUmAmAmA(moe)Um_(CR01008×3) | AmsUfsUmUmAmUfUmA(moe)CmAmGfGmUmGfAmGfUmUmGmsAmsUm |

optionally, the compound is RZ002106.

10. A pharmaceutical composition comprising the compound according to any one of claims 1 to 9 and a pharmaceutically acceptable excipient.

11. Use of the compound according to any one of claims 1 to 9 or the pharmaceutical composition according to claim 10 in the manufacture of a medicament for alleviating, preventing and/or treating a C3 gene-mediated disease or condition, optionally, the C3 gene-mediated disease or condition includes, but is not limited to, IgA nephropathy, atypical haemolytic uremic syndrome (aHUS), including paroxysmal nocturnal haemoglobinuria (PNH), C3 glomerulopathy, lupus nephitis and/or membranous nephritis.

12. A kit comprising the compound according to any one of claims 1 to 9 or the pharmaceutical composition according to claim 10.

13. A method for inhibiting expression of C3 gene in a subject in need thereof, comprising administering to the subject the compound according to any one of claims 1 to 9 or the pharmaceutical composition according to claim 10.

14. A method for alleviating, treating and/or preventing a C3-mediated disease or condition in a subject in need thereof, comprising administering to the subject the compound according to any one of claims 1 to 9 or the pharmaceutical composition according to claim 10,

optionally, the C3 gene-mediated disease or condition includes a disease related to mRNA expression level of C3 gene, and
optionally, the C3 gene-mediated disease or condition includes IgA nephropathy, atypical haemolytic uremic syndrome, paroxysmal nocturnal haemoglobinuria (PNH), C3 glomerulopathy, lupus nephitis and membranous nephritis.

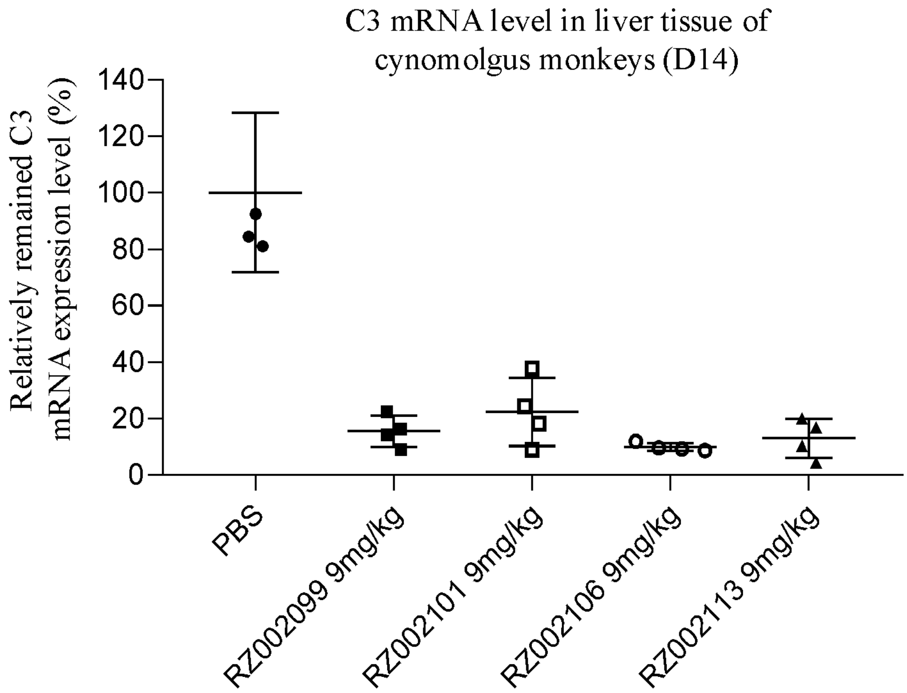
C3 mRNA level in liver tissue of
cynomolgus monkeys (D14)
Relatively remained C3
mRNA expression level (%)
140
120
100
80
60
40
20
0
PBS
RZ002099 9mg/kg
RZ002101 9mg/kg
RZ002106 9mg/kg
RZ002113 9mg/kg

**FIG. 1**

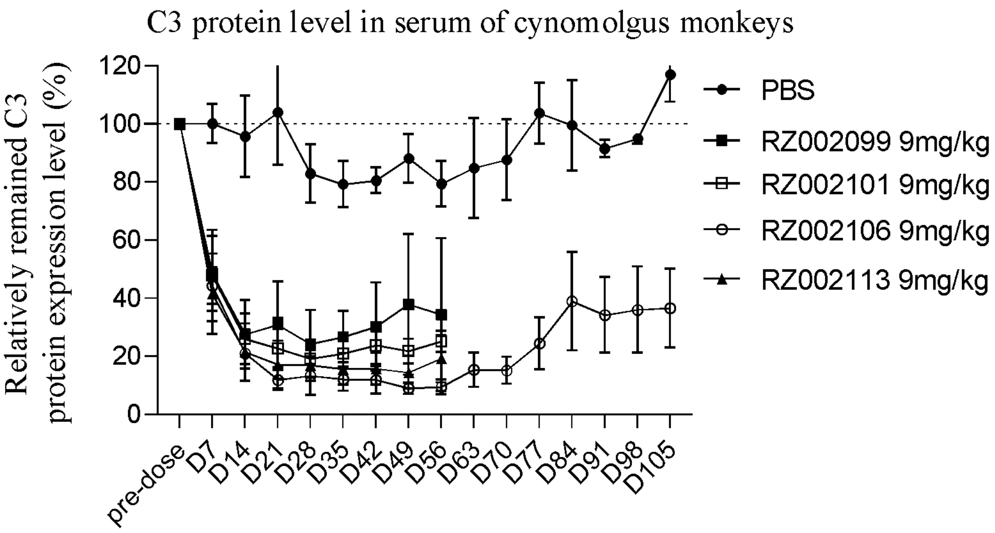
C3 protein level in serum of cynomolgus monkeys
Relatively remained C3
protein expression level (%)
120
100
80
60
40
20
0
pre-dose
D7
D14
D21
D28
D35
D42
D49
D56
D63
D70
D77
D84
D91
D98
D105
PBS
RZ002099 9mg/kg
RZ002101 9mg/kg
RZ002106 9mg/kg
RZ002113 9mg/kg

**FIG. 2**

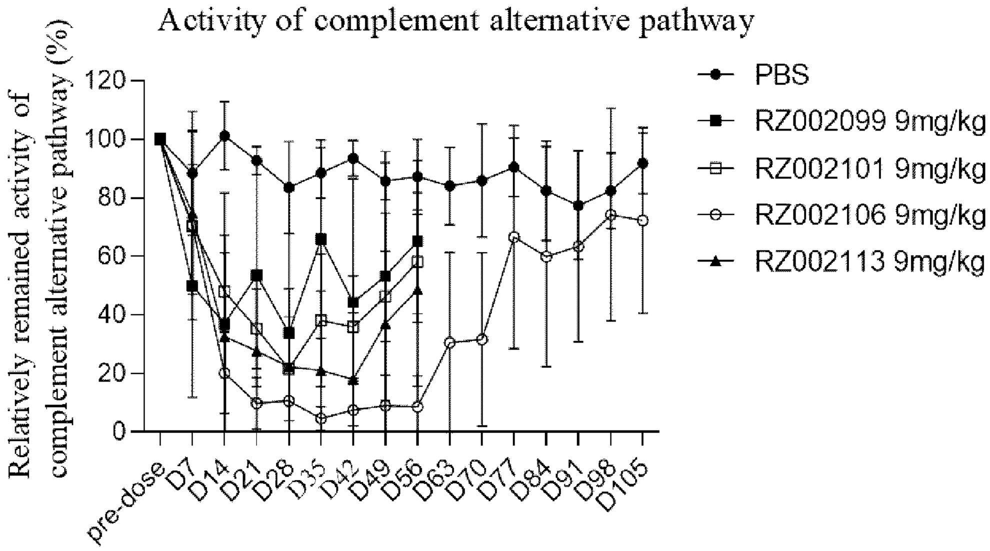
Activity of complement alternative pathway
Relatively remained activity of complement alternative pathway (%)
120
100
80
60
40
20
0
pre-dose
D7
D14
D21
D28
D35
D42
D49
D56
D63
D70
D77
D84
D91
D98
D105
PBS
RZ002099 9mg/kg
RZ002101 9mg/kg
RZ002106 9mg/kg
RZ002113 9mg/kg

FIG. 3

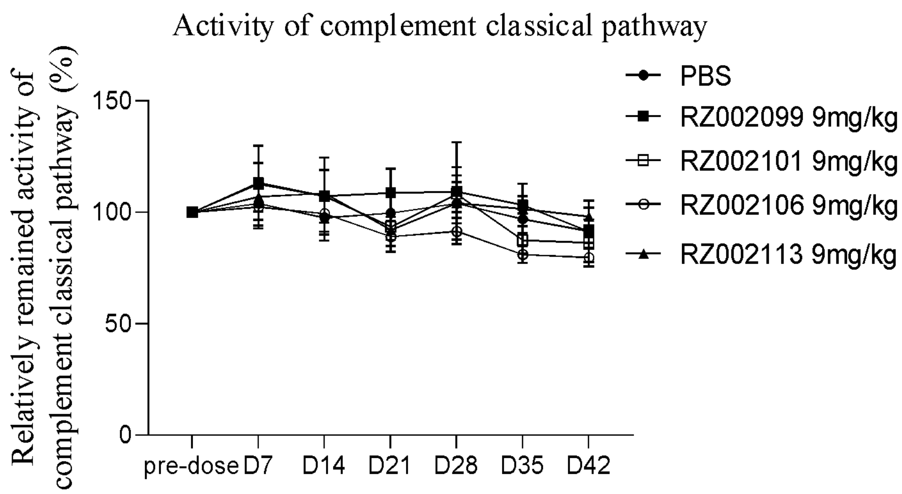
Activity of complement classical pathway
Relatively remained activity of complement classical pathway (%)
150
100
50
0
pre-dose
D7
D14
D21
D28
D35
D42
PBS
RZ002099 9mg/kg
RZ002101 9mg/kg
RZ002106 9mg/kg
RZ002113 9mg/kg

FIG. 4

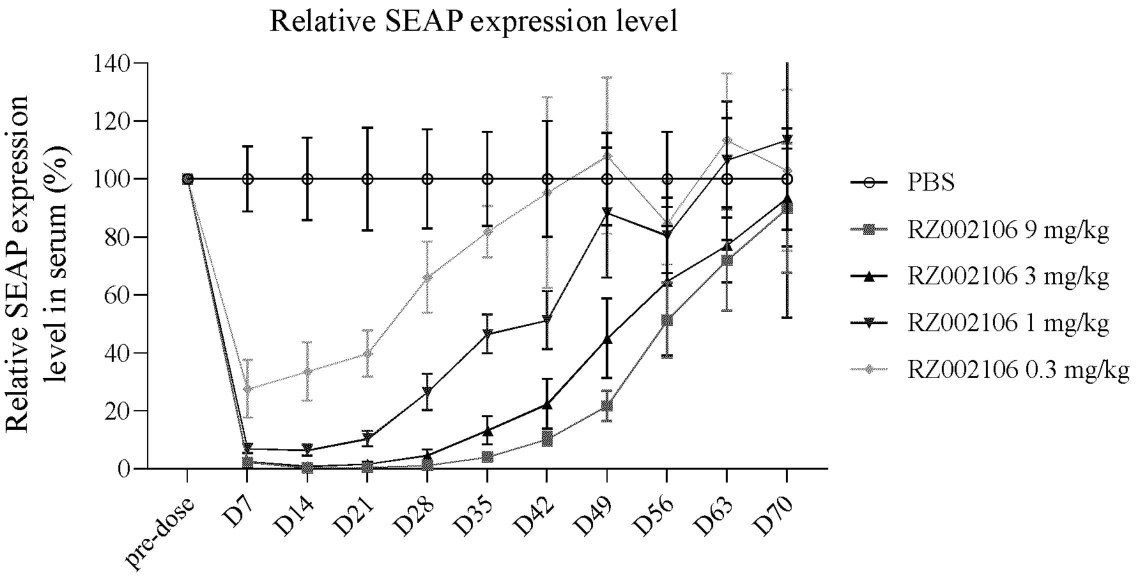
Relative SEAP expression level
Relative SEAP expression level in serum (%)
140
120
100
80
60
40
20
0
pre-dose
D7
D14
D21
D28
D35
D42
D49
D56
D63
D70
PBS
RZ002106 9 mg/kg
RZ002106 3 mg/kg
RZ002106 1 mg/kg
RZ002106 0.3 mg/kg

**FIG. 5**

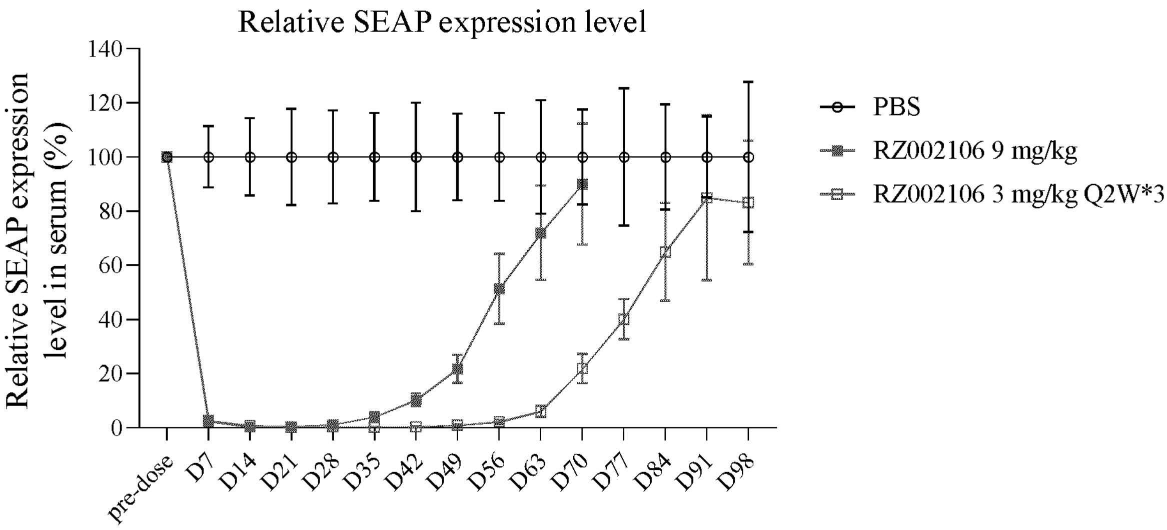
Relative SEAP expression level
Relative SEAP expression level in serum (%)
140
120
100
80
60
40
20
0
pre-dose
D7
D14
D21
D28
D35
D42
D49
D56
D63
D70
D77
D84
D91
D98
PBS
RZ002106 9 mg/kg
RZ002106 3 mg/kg Q2W*3

**FIG. 6**

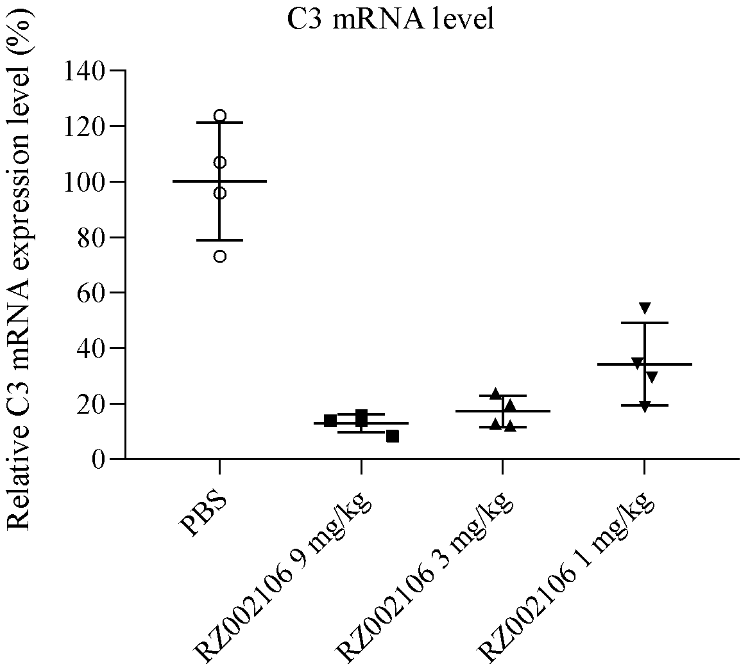
C3 mRNA level
Relative C3 mRNA expression level (%)
140
120
100
80
60
40
20
0
PBS
RZ002106 9 mg/kg
RZ002106 3 mg/kg
RZ002106 1 mg/kg

**FIG. 7**

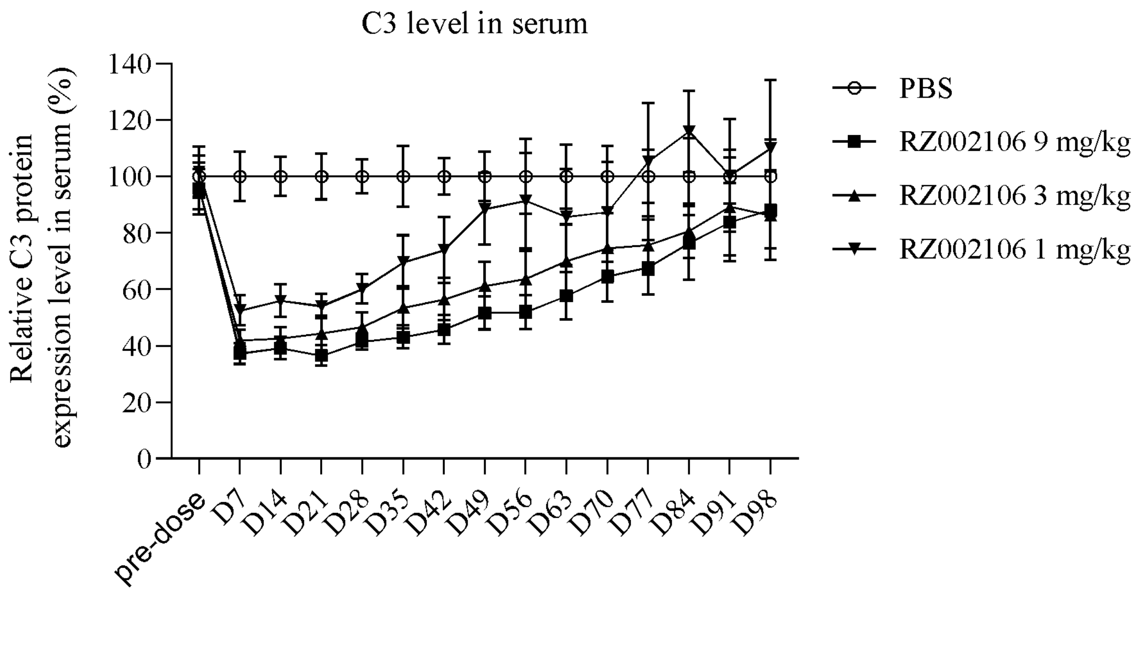
C3 level in serum
Relative C3 protein expression level in serum (%)
140
120
100
80
60
40
20
0
pre-dose
D7
D14
D21
D28
D35
D42
D49
D56
D63
D70
D77
D84
D91
D98
PBS
RZ002106 9 mg/kg
RZ002106 3 mg/kg
RZ002106 1 mg/kg

**FIG. 8**

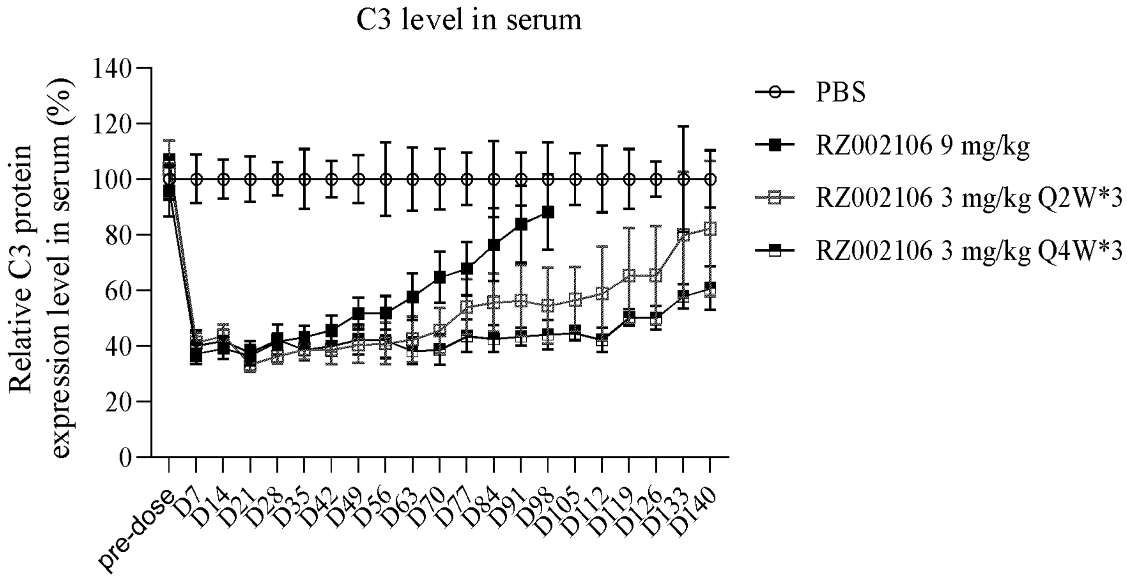
C3 level in serum
Relative C3 protein expression level in serum (%)
140
120
100
80
60
40
20
0
pre-dose D7 D14 D21 D28 D35 D42 D49 D56 D63 D70 D77 D84 D91 D98 D105 D112 D119 D126 D133 D140
PBS
RZ002106 9 mg/kg
RZ002106 3 mg/kg Q2W*3
RZ002106 3 mg/kg Q4W*3

**FIG. 9**

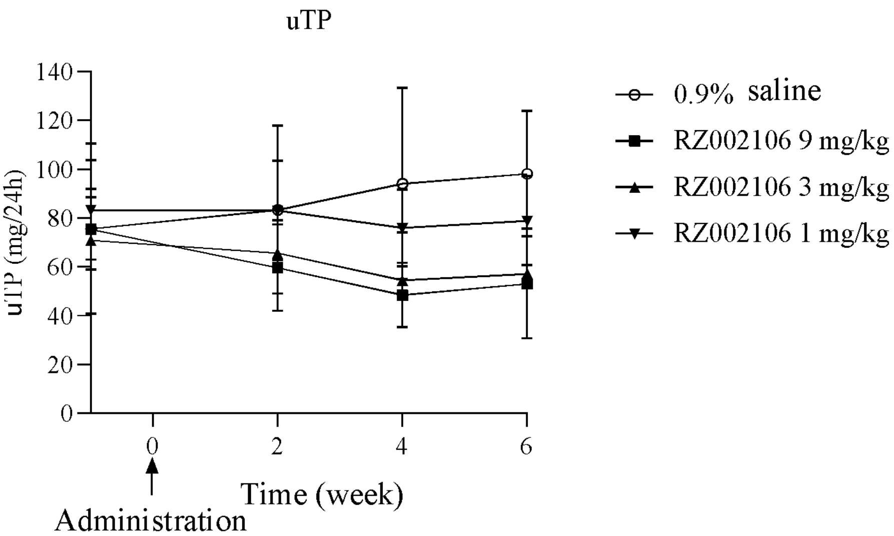
uTP
uTP (mg/24h)
140
120
100
80
60
40
20
0
0
2
4
6
Time (week)
Administration
0.9% saline
RZ002106 9 mg/kg
RZ002106 3 mg/kg
RZ002106 1 mg/kg

**FIG. 10**

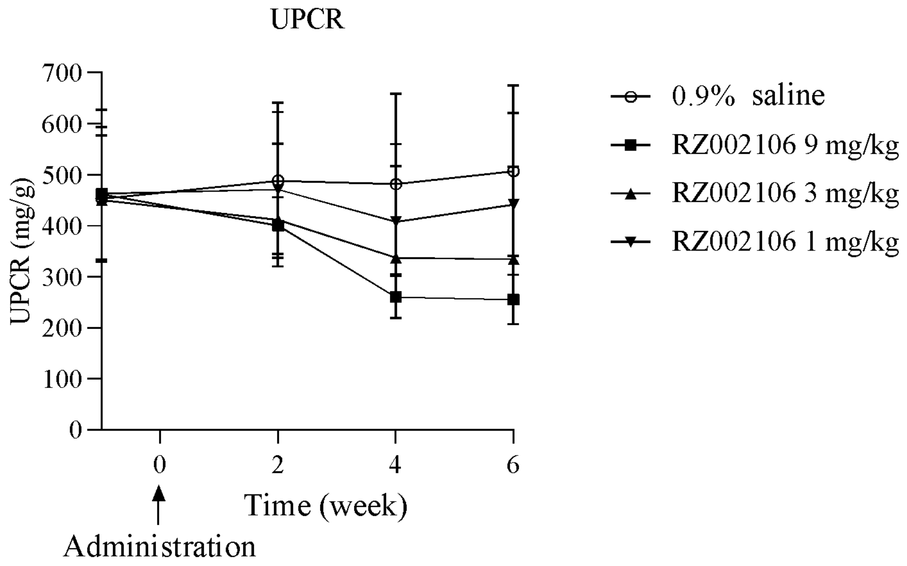
UPCR
UPCR (mg/g)
700
600
500
400
300
200
100
0
0
2
4
6
Time (week)
Administration
0.9% saline
RZ002106 9 mg/kg
RZ002106 3 mg/kg
RZ002106 1 mg/kg

FIG. 11

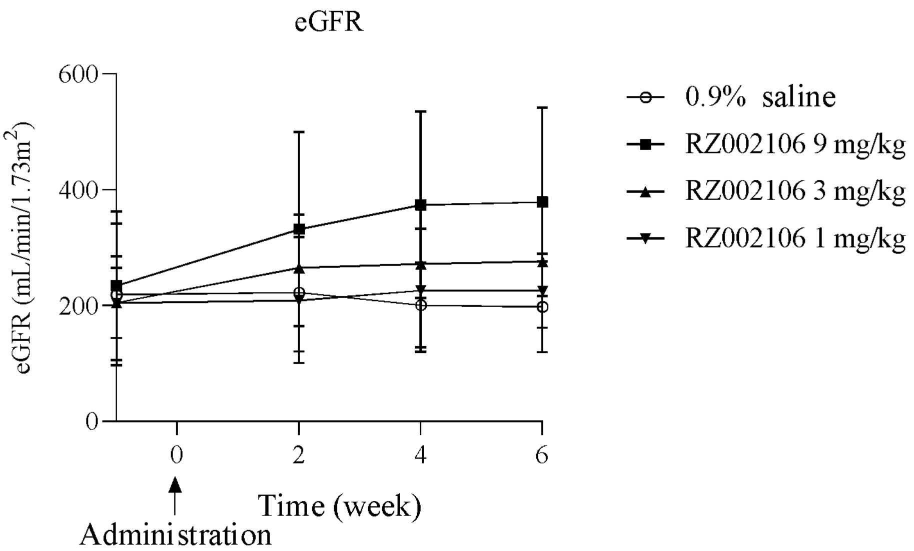
eGFR
eGFR (mL/min/1.73m2)
600
400
200
0
0
2
4
6
Time (week)
Administration
0.9% saline
RZ002106 9 mg/kg
RZ002106 3 mg/kg
RZ002106 1 mg/kg

FIG. 12

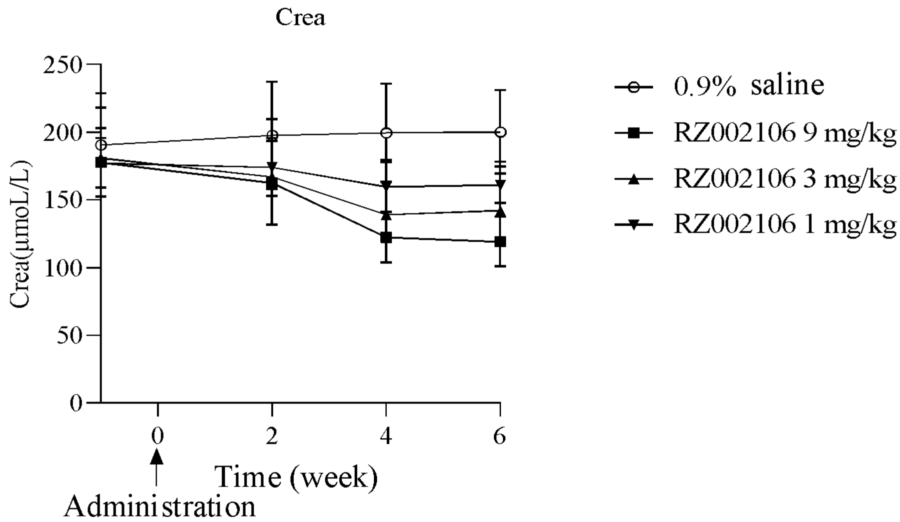
Crea
250
200
150
100
50
0
Crea(µmoL/L)
0
2
4
6
Time (week)
Administration
0.9% saline
RZ002106 9 mg/kg
RZ002106 3 mg/kg
RZ002106 1 mg/kg

**FIG. 13**

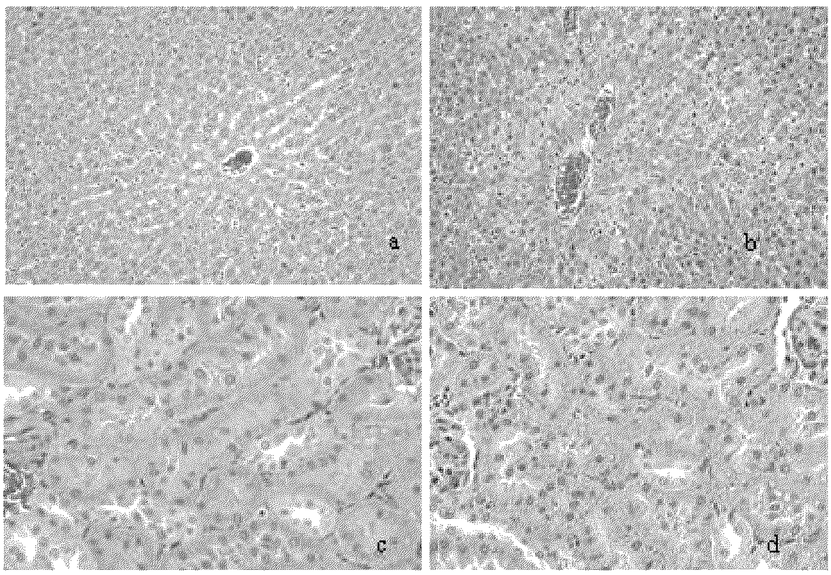
a
b
c
d

**FIG. 14**

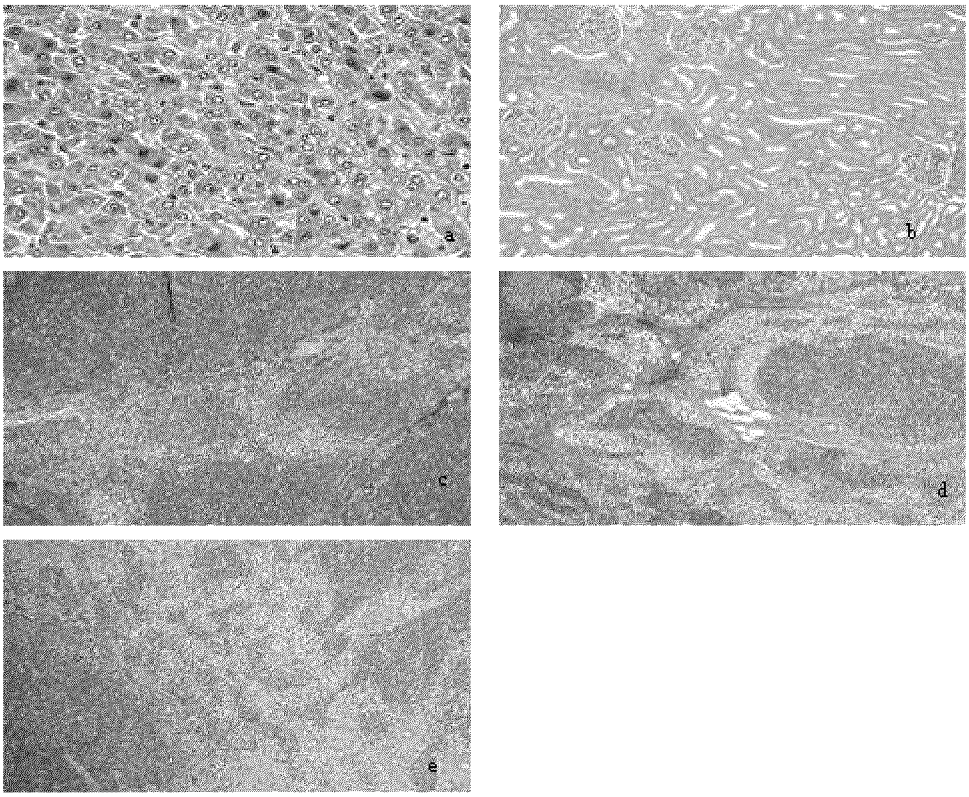


FIG. 15

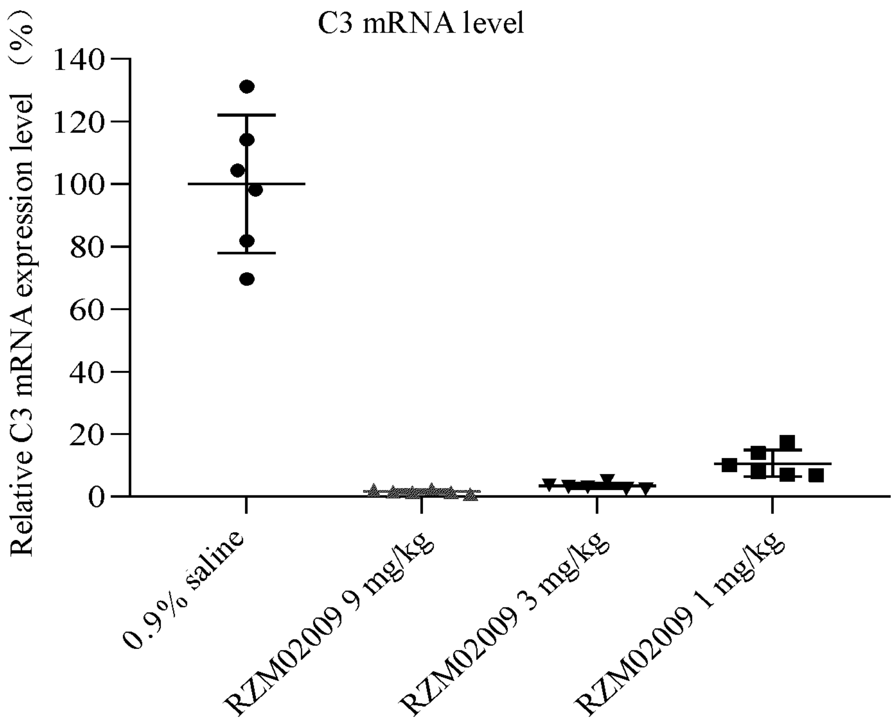
C3 mRNA level
Relative C3 mRNA expression level （%）
140
120
100
80
60
40
20
0
0.9% saline
RZM02009 9 mg/kg
RZM02009 3 mg/kg
RZM02009 1 mg/kg

FIG. 16

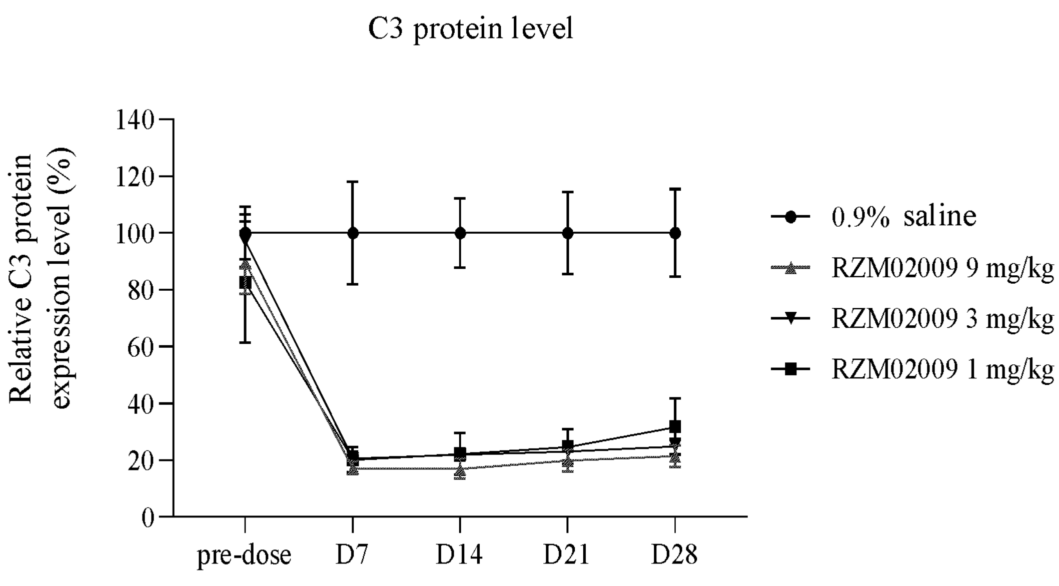
C3 protein level
Relative C3 protein expression level (%)
140
120
100
80
60
40
20
0
pre-dose
D7
D14
D21
D28
0.9% saline
RZM02009 9 mg/kg
RZM02009 3 mg/kg
RZM02009 1 mg/kg

FIG. 17

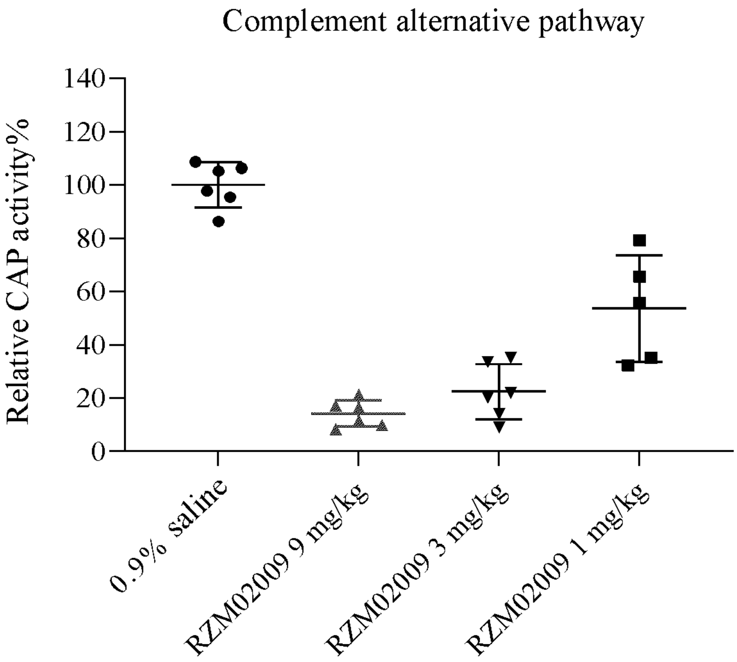
Complement alternative pathway
Relative CAP activity%
140
120
100
80
60
40
20
0
0.9% saline
RZM02009 9 mg/kg
RZM02009 3 mg/kg
RZM02009 1 mg/kg

**FIG. 18**

**INTERNATIONAL SEARCH REPORT**

International application No.
**PCT/CN2024/086555**

**A. CLASSIFICATION OF SUBJECT MATTER**

C12N15/113(2010.01)i；A61K31/7088(2006.01)i；A61P37/00(2006.01)i

According to International Patent Classification (IPC) or to both national classification and IPC

**B. FIELDS SEARCHED**

Minimum documentation searched (classification system followed by classification symbols)

IPC: C12N A61K A61P

Documentation searched other than minimum documentation to the extent that such documents are included in the fields searched

Electronic data base consulted during the international search (name of data base and, where practicable, search terms used)

CNABS, CNTXT, DWPI, WOTXT, EPTXT, USTXT, CNKI, 万方, WANFANG, 读秀, DUXIU, PUBMED, ISI_Web of Science, Science Direct, STNext: 苏州炫景生物科技有限公司, 黄渊余, 结构检索, 序列1-10, sequences 1-10, C3, 双链寡核苷酸, structure search, SiRNA

**C. DOCUMENTS CONSIDERED TO BE RELEVANT**

| Category* | Citation of document, with indication, where appropriate, of the relevant passages | Relevant to claim No. |
|---|---|---|
| Y | CN 113286803 A (ALIGOS THERAPEUTICS, INC.) 20 August 2021 (2021-08-20) claims 1-104, and figure 4A | 1-14 |
| Y | WO 2019089922 A1 (ALNYLAM PHARMACEUTICALS, INC.) 09 May 2019 (2019-05-09) claims 1-58 | 1-14 |
| Y | CN 113905744 A (ALIGOS THERAPEUTICS, INC.) 07 January 2022 (2022-01-07) claims 1-68, table 1, and description, paragraphs 0164-0166 | 1-14 |
| Y | US 2022160748 A1 (ALIGOS THERAPEUTICS, INC.) 26 May 2022 (2022-05-26) table 2, and claims 1-81 | 1-14 |
| Y | WO 2021081026 A1 (ALNYLAM PHARMACEUTICALS, INC.) 29 April 2021 (2021-04-29) claims 1-58, and table 2 | 1-14 |
| Y | WO 2022251484 A1 (APELLIS PHARMACEUTICALS, INC.) 01 December 2022 (2022-12-01) description, paragraph 0177, and claims 1-67 | 1-14 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **11 July 2024** | **17 July 2024** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**

International application No.
**PCT/CN2024/086555**

**C. DOCUMENTS CONSIDERED TO BE RELEVANT**

| Category* | Citation of document, with indication, where appropriate, of the relevant passages | Relevant to claim No. |
|---|---|---|
| Y | WO 2023039076 A1 (ALIGOS THERAPEUTICS, INC. et al.) 16 March 2023 (2023-03-16) claims 1-108 | 1-14 |

Form PCT/ISA/210 (second sheet) (July 2022)

**INTERNATIONAL SEARCH REPORT**

International application No.
**PCT/CN2024/086555**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed.
   b. ☐ furnished subsequent to the international filing date for the purposes of international search (Rule 13*ter*.1(a)),
      ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**

International application No.
**PCT/CN2024/086555**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **13-14**
because they relate to subject matter not required to be searched by this Authority, namely:

Claim 13 sets forth a method for inhibiting C3 gene expression, and claim 14 sets forth a method for relieving, treating and/or preventing C3-mediated diseases or conditions, which methods comprise a treatment regimen for a human or animal body, and therefore said claims do not comply with PCT Rule 39.1(iv). The opinion regarding claims 13-14 is provided on the basis of the following amendment: the use of the compound of any one of claims 1-9 or the composition of claim 10 in the preparation of a drug for relieving, preventing and/or treating C3 gene-mediated diseases or conditions.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**INTERNATIONAL SEARCH REPORT**
**Information on patent family members**

International application No.
**PCT/CN2024/086555**

| Patent document cited in search report | Publication date (day/month/year) | Patent family member(s) | Publication date (day/month/year) |
|---|---|---|---|
| CN 113286803 A | 20 August 2021 | CO 2021005762 A2 | 30 July 2021 |
| | | PE 20211783 A1 | 08 September 2021 |
| | | MX 2021005357 A | 30 June 2021 |
| | | MA 53674 A | 28 July 2021 |
| | | TW 202031267 A | 01 September 2020 |
| | | JP 2022512975 A | 07 February 2022 |
| | | PH 12021551007 A1 | 04 October 2021 |
| | | BR 112021008539 A2 | 03 August 2021 |
| | | CA 3117163 A1 | 14 May 2020 |
| | | IL 282640 A | 30 June 2021 |
| | | SG 11202104636 XA | 29 June 2021 |
| | | US 2020147124 A1 | 14 May 2020 |
| | | EP 3853240 A1 | 28 July 2021 |
| | | EP 3853240 A4 | 30 November 2022 |
| | | AU 2019376079 A1 | 27 May 2021 |
| | | CL 2021001202 A1 | 17 December 2021 |
| | | WO 2020097342 A1 | 14 May 2020 |
| | | KR 20210090217 A | 19 July 2021 |
| WO 2019089922 A1 | 09 May 2019 | US 2021261959 A1 | 26 August 2021 |
| | | US 11866701 B2 | 09 January 2024 |
| | | AU 2018360697 A1 | 14 May 2020 |
| | | IL 274120 A | 30 June 2020 |
| | | SG 11202002940 QA | 29 April 2020 |
| | | CA 3078971 A1 | 09 May 2019 |
| | | EP 3704252 A1 | 09 September 2020 |
| CN 113905744 A | 07 January 2022 | WO 2020243490 A2 | 03 December 2020 |
| | | WO 2020243490 A3 | 21 January 2021 |
| | | CA 3141874 A1 | 03 December 2020 |
| | | CO 2021018034 A2 | 19 April 2022 |
| | | AU 2020282828 A1 | 27 January 2022 |
| | | TW 202113079 A | 01 April 2021 |
| | | EP 3976056 A2 | 06 April 2022 |
| | | US 2020385735 A1 | 10 December 2020 |
| | | US 11466274 B2 | 11 October 2022 |
| | | IL 288259 A | 01 January 2022 |
| | | BR 112021023488 A2 | 18 January 2022 |
| | | KR 20220024192 A | 03 March 2022 |
| | | PE 20220574 A1 | 20 April 2022 |
| | | SG 11202112741 XA | 30 December 2021 |
| | | MX 2021014206 A | 06 January 2022 |
| | | JP 2022535735 A | 10 August 2022 |
| US 2022160748 A1 | 26 May 2022 | TW 202237189 A | 01 October 2022 |
| | | CA 3200094 A1 | 27 May 2022 |
| | | AU 2021383758 A1 | 15 June 2023 |
| | | WO 2022109129 A1 | 27 May 2022 |
| | | EP 4247393 A1 | 27 September 2023 |
| WO 2021081026 A1 | 29 April 2021 | TW 202134435 A | 16 September 2021 |
| | | EP 4048793 A1 | 31 August 2022 |
| | | IL 292360 A | 01 June 2022 |
| | | MX 2022004726 A | 13 May 2022 |

Form PCT/ISA/210 (patent family annex) (July 2022)

**INTERNATIONAL SEARCH REPORT**
**Information on patent family members**

International application No.
**PCT/CN2024/086555**

| Patent document cited in search report | Publication date (day/month/year) | Patent family member(s) | Publication date (day/month/year) |
|---|---|---|---|
| | | BR 112022007540 A2 | 12 July 2022 |
| | | KR 20220084399 A | 21 June 2022 |
| | | JP 2022553348 A | 22 December 2022 |
| | | AR 120267 A1 | 09 February 2022 |
| | | CA 3158320 A1 | 29 April 2021 |
| | | US 2022364088 A1 | 17 November 2022 |
| | | AU 2020369515 A1 | 21 April 2022 |
| WO 2022251484 A1 | 01 December 2022 | EP 4346844 A1 | 10 April 2024 |
| | | JP 2024521792 A | 04 June 2024 |
| | | WO 2022251484 A9 | 12 October 2023 |
| WO 2023039076 A1 | 16 March 2023 | CA 3230382 A1 | 16 March 2023 |
| | | KR 20240053635 A | 24 April 2024 |
| | | AU 2022343115 A1 | 14 March 2024 |

Form PCT/ISA/210 (patent family annex) (July 2022)